# EUROPEAN PATENT APPLICATION

(11) **EP 2 216 330 A1**
(43) Date of publication of application: **11.08.2010**
(21) Application number: 08842584.8
(22) Date of filing: 22.10.2008
(51) Int. Cl.: C07D 271/06, A61K 31/4245, A61K 31/454, A61P 3/04, A61P 3/10, A61P 5/50, A61P 43/00, C07D 413/12

(54) **OXADIAZOLIDINEDIONE COMPOUND**

(30) Priority: 24.10.2007 JP 2007275840
(71) Applicant: Astellas Pharma Inc., Tokyo 103-8411 (JP)
(72) Inventor: NEGORO, Kenji, Tokyo 103-8411 (JP); IWASAKI, Fumiyoshi, Tokyo 103-8411 (JP); OHNUKI, Kei, Tokyo 103-8411 (JP); KUROSAKI, Toshio, Tokyo 103-8411 (JP); TSUCHIYA, Kazuyuki, Tokyo 103-8411 (JP); KURAMOTO, Kazuyuki, Tokyo 103-8411 (JP); YOSHIDA, Shigeru, Tokyo 103-8411 (JP); SOGA, Takatoshi, Tokyo 103-8411 (JP)
(74) Representative: Gille Hrabal Struck Neidlein Prop Roos
(86) International application number: PCT/JP2008/069164
(87) International publication number: WO 2009/054423

(57) **Abstract**

A pharmaceutical agent having GPR40 receptor agonistic action, particularly a compound which is useful as an insulin secretagogue or an agent for preventing and/or treating diabetes mellitus. The present inventors have examined a compound having GPR40 receptor agonistic action, confirmed that an oxadiazolidinedione compound which has a substituent such as a benzyl group, etc. linked with a substituent such as a phenyl group, etc. through a linker at the 2-position of an oxadiazolidinedione ring, or a pharmaceutically acceptable salt thereof has an excellent GPR40 agonistic activity, and thus completed the invention. The oxadiazolidinedione compound has excellent insulin secretagogue action and anti-hyperglycemic action, and therefore can be used as an insulin secretagogue or an agent for preventing and/or treating diabetes mellitus.

## Description

### Technical Field

The present invention relates to an oxadiazolidinedione compound useful as a pharmaceutical agent, particularly as an insulin secretagogue or an agent for preventing and/or treating diabetes mellitus.

### Background Art

Diabetes mellitus is a disease whose cardinal sign is chronic hyperglycemia, and it occurs as a result of absolute or relative deficiency of insulin action. Diabetes mellitus is roughly classified into two types according to its diagnostic in clinical practice, which are insulin-dependent diabetes mellitus (type 1 diabetes) and non-insulin-dependent diabetes mellitus (type 2 diabetes). In the non-insulin-dependent diabetes mellitus, decrease in insulin secretion from β-cells of the pancreas is one of cardinal pathogenesis, and in particular, postprandial hyperglycemia which is caused by deficient secretion of insulin is recognized in the early stages.

Lately, it has been confirmed by a large-scale clinical trial that the correction of postprandial hyperglycemia is important in preventing onset and progress of diabetic complications. In addition, it is reported that arteriosclerosis only occurs in the stage of postprandial hyperglycemia and that duration of mild postprandial hyperglycemia increases the mortality from cardiovascular diseases or the like. This shows that postprandial hyperglycemia is an independent risk factor of cardiac death even if it is mild. According to the findings as described above, the need of pharmacotherapy for postprandial hyperglycemia has been realized.

At present, sulfonylurea (SU) drugs are the mainstream as an insulin secretagogue, but it is known that the drug is likely to cause hypoglycemia and its chronic administration leads to secondary failure due to pancreatic exhaustion. Furthermore, the SU drug has a beneficial effect on glycemic control between meals, but it is difficult to suppress postprandial hyperglycemia.

It is reported that GPR40 is a G protein-coupled receptor which is highly expressed in β cells of the pancreas and identified as a fatty acid receptor, and associated with the insulin secretagogue action of fatty acid (Non-Patent Document 1).

Accordingly, GPR40 receptor agonist is expected to correct postprandial hyperglycemia on the basis of insulin secretagogue action, and thus it is useful as an agent for preventing and/or treating insulin-dependent diabetes mellitus (type 1 diabetes), non-insulin-dependent diabetes mellitus (type 2 diabetes) and borderline thereof (impaired glucose tolerance/ fasting blood glucose).

In Patent Document 1, it is reported that a compound of the formula (A) including a wide range of compounds has GPR40 receptor regulatory action, and is useful as an insulin secretagogue or an agent for preventing and/or treating diabetes mellitus. However, there is no specific disclosure about compounds having an oxadiazolidinedione structure. 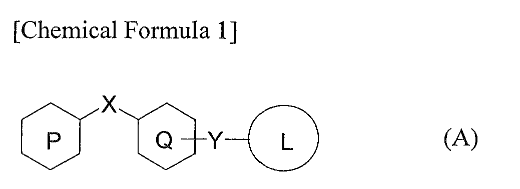 (In the formula, ring P represents an aromatic ring which may have substituents, ring Q represents an aromatic ring which may have substituents other than the following: 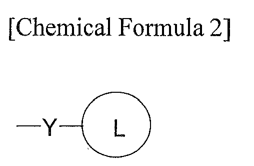 ; X and Y each represents a spacer; and 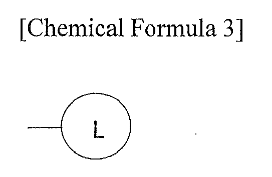 represents a group capable of releasing a cation)

In Patent Document 2, it is reported that a compound of the formula (B) has GPR40 receptor regulatory action, and is useful as an insulin secretagogue or an agent for preventing and/or treating diabetes mellitus. 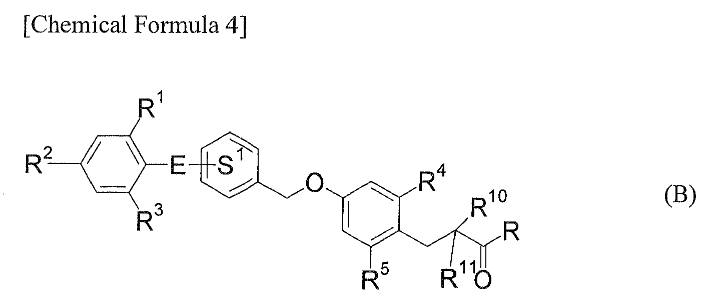 (For symbols in the formula, please refer to the publication)

In Patent Document 3, it is reported that a compound of the formula (C) has GPR40 receptor regulatory action, and is useful as an insulin secretagogue or an agent for preventing and/or treating diabetes mellitus. 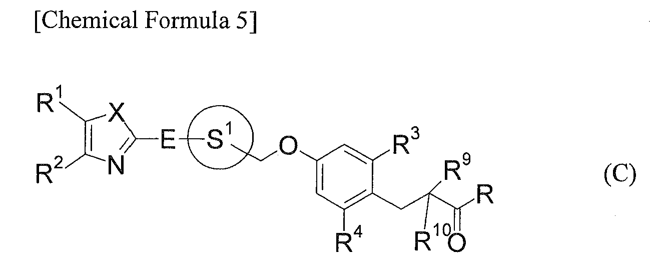 (For symbols in the formula, please refer to the publication)

In Patent Document 4, it is reported that an oxadiazolidinedione compound of the formula (D) has plasminogen-activator inhibitor (PAI)-1 inhibitory action, and is useful for treating thrombus, atrial fibrillation, myocardial ischemia, diabetes mellitus or the like. 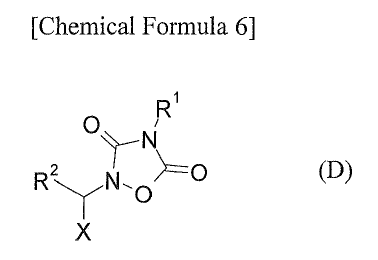 (In the formula, X represents 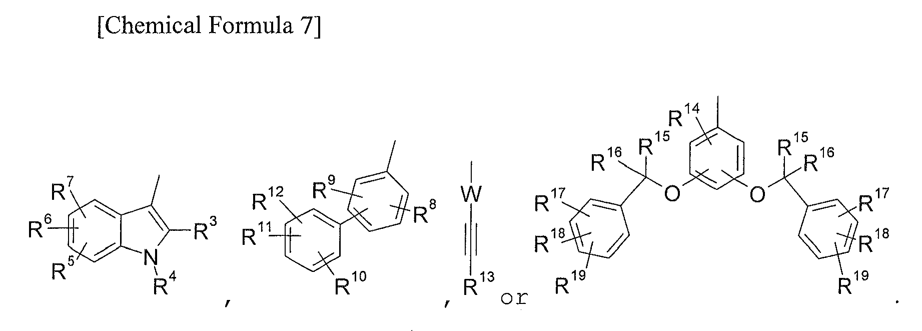 For other symbols, please refer to the publication) In Patent Document 5, it is reported that a compound of the formula (E) having two oxadiazolidinedione structures has insulin sensitivity potentiating action, and is useful for treating diabetes mellitus. 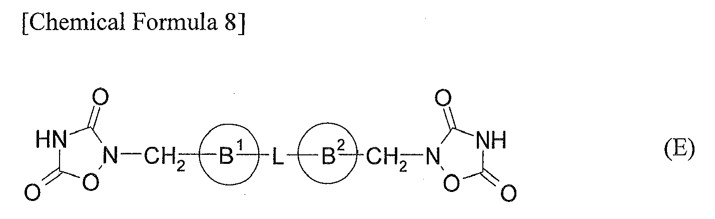 (For symbols in the formula, please refer to the publication)

In Patent Document 6, it is reported that an oxadiazolidinedione compound of the formula (F) has hypoglycemic action and hypolipidemic action in blood, and is useful for treating diabetes mellitus. 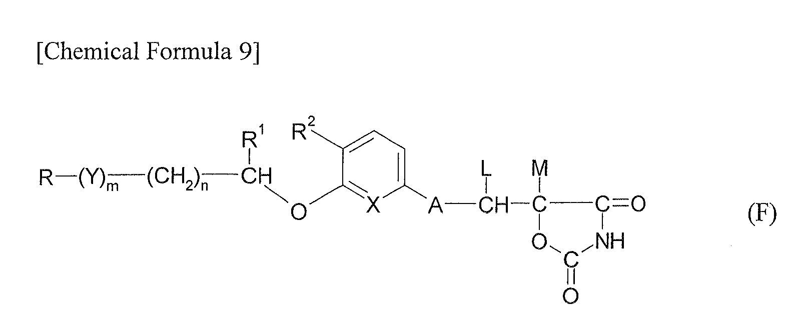 (For symbols in the formula, please refer to the publication)

In Patent Document 7, it is reported that an oxadiazolidinedione compound of the formula (G) has hypoglycemic action, and is useful for treating diabetes mellitus. 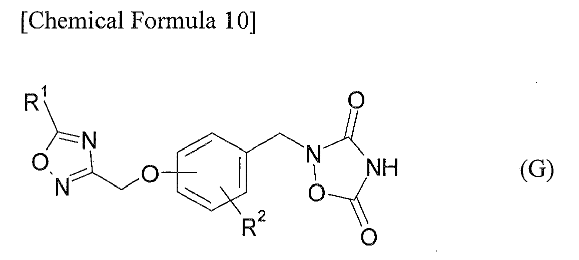 (For symbols in the formula, please refer to the publication)

In Patent Document 8, it is reported that a compound of the formula (H) has hypoglycemic action, and is useful for treating diabetes mellitus. 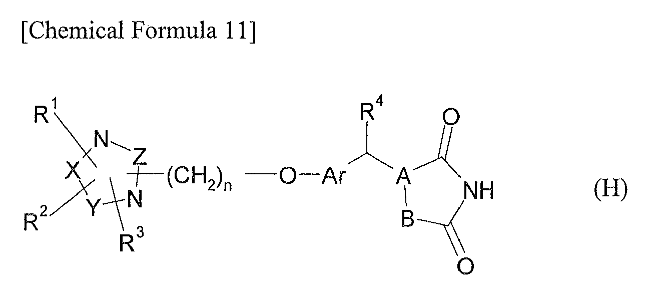 (For symbols in the formula, please refer to the publication)

In Patent Document 9, it is reported that an oxadiazolidinedione compound of the formula (J) has hypoglycemic action, and is useful for treating diabetes mellitus. 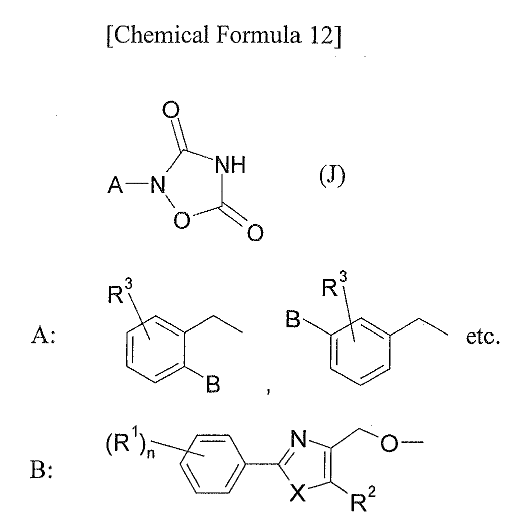 (In the formula, X refers to an oxygen atom or a sulfur atom. For other symbols, please refer to the publication)

In Patent Document 10, it is reported that a compound of the formula (K) is useful for hyperlipidemia, hyperglycemia, obesity or the like. 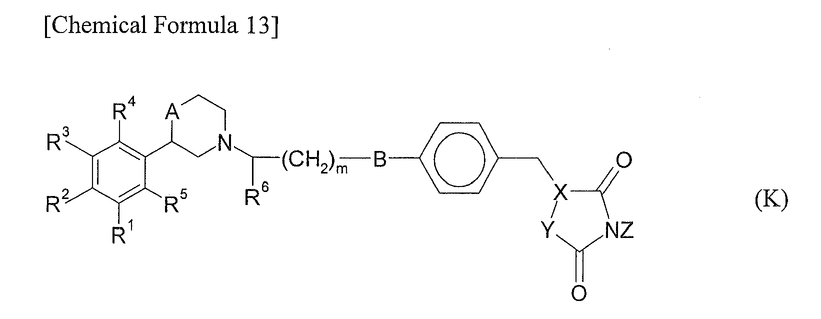 (In the formula, A means an oxygen atom or a sulfur atom. For other symbols, please refer to the publication)

In Non-Patent Document 2, it is reported that an oxadiazolidinedione compound of the formula (L) has hypoglycemic action, and is useful for treating diabetes mellitus. 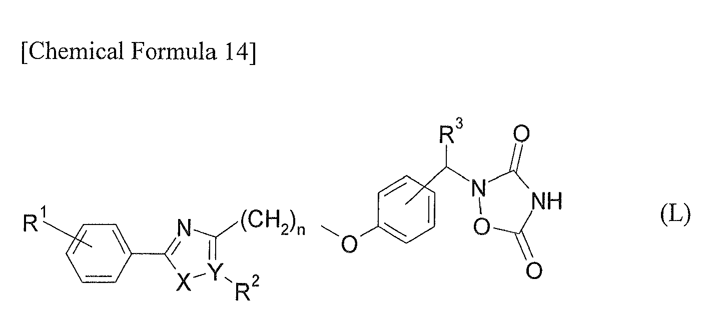 (In the formula, X means O, S or N; Y means C or N; and n means 1 or 2. For other symbols, please refer to the publication)

In Patent Document 11, it is reported that a compound of the formula (M) has GPR40 receptor regulatory action, and is useful as an insulin secretagogue or an agent for preventing and/or treating diabetes mellitus. 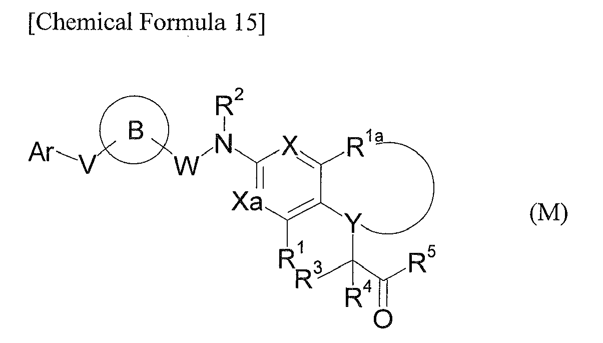 (For symbols in the formula, please refer to the publication)

In Patent Document 12 which was published after the priority date of the present application, it is reported that a compound of the formula (N) has GPR40 receptor regulatory action, and is useful as an insulin secretagogue or an agent for preventing and/or treating diabetes mellitus. 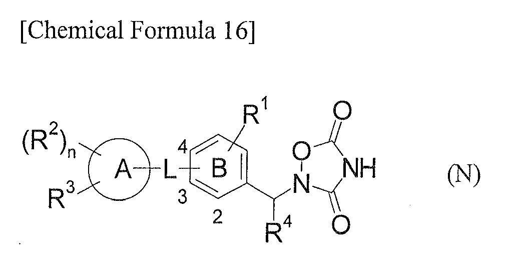 (For symbols in the formula, please refer to the publication)
[Non-Patent Document 1] "Nature", (Britain), 2003, Vol. 422, p. 173-176 [Non-Patent Document 2] "European Journal of Medicinal Chemistry", (France), 2001, Vol. 36, p. 31-42
[Patent Document 1] Pamphlet of International Publication No. WO 2004/041266
[Patent Document 2] Pamphlet of International Publication No. WO 2005/063729
[Patent Document 3] Pamphlet of International Publication No. WO 2005/063725
[Patent Document 4] Pamphlet of International Publication No. WO 2005/030203
[Patent Document 5] Pamphlet of International Publication No. WO 94/25448
[Patent Document 6] Japanese Patent Application, JP-A No. 2000-212174
[Patent Document 7] Pamphlet of International Publication No. WO 95/30664
[Patent Document 8] Pamphlet of International Publication No. WO 97/41097
[Patent Document 9] US Patent No. 5480896
[Patent Document 10] Japanese Patent Application, JP-A No. H7-2848
[Patent Document 11] Pamphlet of International Publication No. WO 2005/087710
[Patent Document 12] Pamphlet of International Publication No. WO 2007/123225

### DISCLOSURE OF THE INVENTION

### PROBLEM THAT THE INVENTION IS TO SOLVE

It is to provide a compound useful as a pharmaceutical agent having GPR40 receptor agonistic action, particularly an insulin secretagogue or an agent for preventing and/or treating diabetes mellitus.

### MEANS FOR SOLVING THE PROBLEM

The present inventors have examined a compound having GPR40 receptor agonistic action, and as a result, they have found that an oxadiazolidinedione compound having a substituent such as a benzyl group etc. linked with a substituent such as a phenyl group etc. through a linker at the 2-position of an oxadiazolidinedione ring exhibits excellent GPR40 receptor agonistic action. Furthermore, they found that these oxadiazolidinedione compounds have excellent insulin secretagogue action and strongly suppress the rise in blood glucose after a glucose tolerance test. Thus they have completed the invention.

That is, the present invention relates to a compound of the formula (I) or a pharmaceutically acceptable salt thereof, as well as a pharmaceutical composition containing the compound of the formula (I) or a pharmaceutically acceptable salt thereof and a pharmaceutically acceptable excipient. 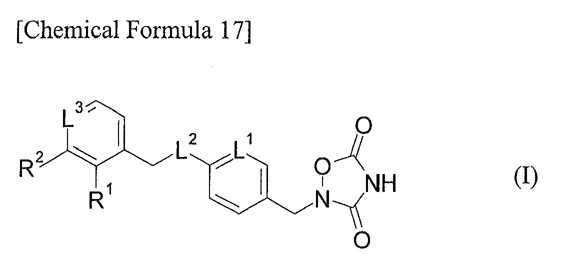 (Symbols in the formula have the following meanings:
L¹ and L³ are the same with or different from each other and each represents CH or N;
L² represents O or NH;
R¹ represents -H or C₁₋₆ alkyl;
R² represents a group of the formula (II) or (III): 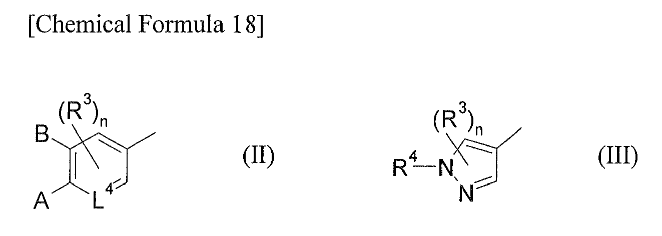
L⁴ represents CH or N;
A and B are the same with or different from each other and each represents -O-(C₁₋₆ alkyl substituted with one or more group(s) selected from G¹ group), amino which may be substituted with one or more group(s) selected from G² group, -H or -R³ (provided that at least one of A and B represents a group other than -H and -R³);
R³ is the same with or different from each other and represents C₁₋₆ alkyl which may be substituted with one or more group (s) selected from the group consisting of-OH and halogen, halogen or -O-(C₁₋₆ alkyl);
R⁴ represents C₁₋₆ alkyl which is substituted with one or more group(s) selected from G¹ group;
n represents 1 or 2;
G¹ group represents the group consisting of -NHCO₂R^{Z}, -NH₂, -NHCOR^{Z},-NHCO-(cycloalkyl), -NHCO-(aryl), -NHSO₂R^{Z}, 1,3-dioxolan-4-yl which may be substituted with 1 to 5 C₁₋₆ alkyl, -OH, -OCOR^{Z}, -OR^{Z}, -CO₂R^{Z}, -CO₂H, -CONHR^{Z} and -CON(R^{Z})₂;
G² group represents the group consisting of -CO₂R^{Z} and -R^{Z}; and
R^{Z} is the same with or different from each other and represents C₁₋₆ alkyl which may be substituted with one or more group(s) selected from the group consisting of-OH and -OCO-(C₁₋₆ alkyl). The same shall apply hereinafter.)

The present invention relates to a GPR40 receptor agonist or an insulin secretagogue which comprises a compound of the formula (I) or a pharmaceutically acceptable salt thereof.

The present invention relates to a pharmaceutical composition for preventing and/or treating diabetes mellitus which comprises a compound of the formula (I) or a pharmaceutically acceptable salt thereof, namely, an agent for preventing and/or treating diabetes mellitus which comprises a compound of the formula (I) or a pharmaceutically acceptable salt thereof.

In addition, the present invention relates to use of the compound of the formula (I) or a pharmaceutically acceptable salt thereof for producing an insulin secretagogue or an agent for preventing and/or treating diabetes mellitus, and a method for promotion of insulin secretion or a method for preventing and/or treating diabetes mellitus which includes administering an effective dose of the compound of the formula (I) or a salt thereof to patients.

That is:
(1) a pharmaceutical composition which comprises the compound of the formula (I) or a pharmaceutically acceptable salt thereof, and a pharmaceutical acceptable excipient;
(2) a GPR40 agonist which comprises the compound of the formula (I) or a pharmaceutically acceptable salt thereof;
(3) an insulin secretagogue which comprises the compound of the formula (I) or a pharmaceutical acceptable salt thereof;
(4) a pharmaceutical composition for preventing and/or treating diabetes mellitus which comprises the compound of the formula (I) or a pharmaceutically acceptable salt thereof;
(5) use of the compound of the formula (I) or a pharmaceutically acceptable salt thereof for producing an insulin secretagogue or an agent for preventing and/or treating diabetes mellitus; and
(6) a method for promotion of insulin secretion or a method for preventing and/or treating diabetes mellitus which includes administering an effective dose of the compound of the formula (I) or a salt thereof to patients.

### EFFECT OF THE INVENTION

The compound of the formula (I) or a pharmaceutically acceptable salt thereof has GPR40 receptor agonistic action, and can be used as insulin secretagogues and an agent for preventing and/or treating diseases associated with GPR40 such as diabetes mellitus (insulin-dependent diabetes mellitus (type 1 diabetes), non-insulin-dependent diabetes mellitus (type 2 diabetes) and borderline thereof (impaired glucose tolerance/fasting blood glucose), obesity and the like.

### BEST MODE FOR CARRYING OUT THE INVENTION

The present invention provides the following.
[1] A compound of the formula (I): 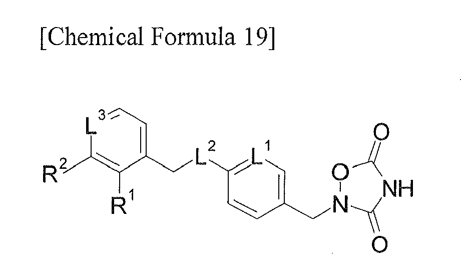 (symbols in the formulae have the following meanings:
   L¹ and L³ are the same with or different from each other and each represents CH or N;
   L² represents O or NH;
   R¹ represents -H or C₁₋₆ alkyl;
   R² represents a group of the formula (II) or (III): 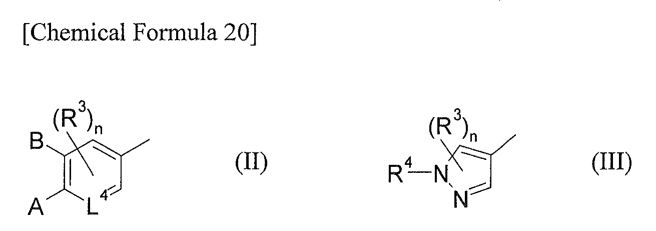
   L⁴ represents CH or N;
   A and B are the same with or different from each other and represent -O-(C₁₋₆ alkyl substituted with one or more group(s) selected from G¹ group), amino which may be substituted with one or more group(s) selected from G² group, -H or -R³ (provided that at least one of A and B represents a group other than -H and -R³);
   R³ is the same with or different from each other and represents C₁₋₆ alkyl which may be substituted with one or more group (s) selected from the group consisting of-OH and halogen, halogen or -O-(C₁₋₆ alkyl);
   R⁴ represents C₁₋₆ alkyl which is substituted with one or more group(s) selected from G¹ group;
   n represents 1 or 2;
   G¹ group represents the group consisting of -NHCO₂R^{Z}, -NH₂, -NHCOR^{Z},-NHCO-(cycloalkyl), -NHCO-(aryl), -NHSO₂R^{Z}, 1,3-dioxolan-4-yl which may be substituted with 1 to 5 c₁₋₆ alkyl, -OH, -OCOR^{Z}, -OR^{Z}, -CO₂R^{Z}, -CO₂H, -CONHR^{Z} and -CON(R^{Z})₂;
   G² group represents the group consisting of -CO₂R^{Z} and -R^{Z}; and
   R^{Z} is the same with or different from each other and represents C₁₋₆ alkyl which may be substituted with one or more group(s) selected from the group consisting of -OH and -OCO-(C₁₋₆ alkyl)) or a pharmaceutically acceptable salt thereof.
[2] The compound as described in [1], in which L³ is CH; R¹ is -H or methyl; R² is a group of the formula (II); either one of A and B is -O- (C₁₋₆ alkyl substituted with one or more group(s) selected from G¹ group), and the other of A or B is -H or -R³; and R³ is the same with or different from each other, and methyl which may be substituted with one or more halogen, halogen or -O-methyl; or a pharmaceutically acceptable salt thereof.
[3] The compound as described in [2], in which either one of A and B is -O-(C₁₋₆ alkyl which is substituted with one or more group(s) selected from the group consisting of -NHCOR^{Z}, -NHCO-(cycloalkyl), -OH and -OR^{Z}); R^{Z} is C₁₋₆ alkyl which may be substituted with one or more -OH, and the other of A or B is -H, methyl or halogen; or a pharmaceutically acceptable salt thereof.
[4] The compound as described in [3], in which R³ is methyl, or a pharmaceutically acceptable salt thereof.
[5] The compound as described in [4], in which R¹ is methyl, or a pharmaceutically acceptable salt thereof.
[6] The compound as described in [5], in which either one of A and B is -H; and n is 2; or a pharmaceutically acceptable salt thereof.
[7] The compound as described in [5], in which either one of A and B is methyl or halogen; and n is 1; or a pharmaceutically acceptable salt thereof.
[8] The compound as described in [6] or [7], in which A is -O-(C₁₋₆ alkyl which is substituted with one or more group(s) selected from the group consisting of - NHCOR^{Z}, -NHCO-(cycloalkyl), -OH and -OR^{Z}), or a pharmaceutically acceptable salt thereof.
[9] The compound as described in [8], in which A is -O-(C₁₋₆ alkyl substituted with one or more -OH), or a pharmaceutically acceptable salt thereof.
[10] The compound as described in [9], in which L¹ is CH, or a pharmaceutically acceptable salt thereof.
[11] The compound as described in [10], in which L² is O, or a pharmaceutically acceptable salt thereof.
[12] The compound as described in [10], wherein L² is NH, or a pharmaceutically acceptable salt thereof.
[13] The compound as described in [1] which is
   2-(4-{ [(4'-{ [(2S)-2,3-dihydroxypropyl]oxy}-2,2',6'-trimethylbiphenyl-3-yl)methyl]amino}benzyl)-1,2,4-oxadiazolidine-3,5-dione,
   2-(4-{[(4'-{[(2R)-2,3-dihydroxypropyl]oxy}-2,2',6'-trimethylbiphenyl-3-yl)methyl]amino}benzyl)-1,2,4-oxadiazolidine-3,5-dione,
   2-[4-({[5'-fluoro-4'-(2-hydroxyethoxy)-2,2'-dimethylbiphenyl-3-yl]methyl}amino)benzyl]-1,2,4-oxadiazolidine-3,5-dione,
   2-[4-({3-[6-(2-hydroxyethoxy)-2,5-dimethylpyridin-3-yl]-2-methylbenzyl}amino)benzyl]-1,2,4-oxadiazolidine-3,5-dione, 2-(4-{[5'-fluoro-4'-(2-hydroxyethoxy)-2,2'-dimethylbiphenyl-3-yl]methoxy}benzyl)-1,2,4-oxadiazolidine-3,5-dione,
   2-(4-{[(4'-{[(2S)-2,3-dihydroxypropyl]oxy}-2,2',5'-trimethylbiphenyl-3-yl)methyl]amino}benzyl)-1,2,4-oxadiazolidine-3,5-dione,
   2-(4-{[(4'-{[(2R)-2,3-dihydroxypropyl]oxy}-2,2',5'-trimethylbiphenyl-3-yl)methyl]amino}benzyl)-1,2,4-oxadiazolidine-3,5-dione,
   2-(4-{[(4'-{[(3R)-3,4-dihydroxybutyl]oxy}-2,2',6'-trimethylbiphenyl-3-yl)methyl]amino}benzyl)-1,2,4-oxadiazolidine-3,5-dione,
   2-(4-{[(4'-{[(3S)-3,4-dihydroxybutyl]oxy}-2,2',6'-trimethylbiphenyl-3-yl)methyl]amino}benzyl)-1,2,4-oxadiazolidine-3,5-dione,
   2-[4-({2,2',6'-trimethyl-4'-[3-(propionylamino)propoxy]biphenyl-3-yl}methoxy)benzyl]-1,2,4-oxadiazolidine-3,5-dione,
   2-{4-[(4'-{3-[(cyclopropylcarbonyl)amino]propoxy}-2,2',6'-trimethylbiphenyl-3-yl)methoxy]benzyl}-1,2,4-oxadiazolidine-3,5-dione,
   2-{4-[(4'-{[(2S)-2,3-dihydroxypropyl]oxy}-2,2',6'-trimethylbiphenyl-3-yl)methoxy]benzyl}-1,2,4-oxadiazolidine-3,5-dione,
   2-{4-[(4'-{[(2R)-2,3-dihydroxypropyl]oxy}-2,2',6'-trimethylbiphenyl-3-yl)methoxy]benzyl}-1,2,4-oxadiazolidine-3,5-dione,
   2-(4-{[3'-(3-hydroxy-3-methylbutoxy)-2,2'-dimethylbiphenyl-3-yl]methoxy}benzyl)-1,2,4-oxadiazolidine-3,5-dione,
   2-(4-{[4'-(3-hydroxy-3-methylbutoxy)-2,2',5'-trimethylbiphenyl-3-yl]methoxy}benzyl)-1,2,4-oxadiazolidine-3,5-dione,
   2-(4-{[4'-(3-hydroxy-3-methylbutoxy)-2,2',3'-trimethylbiphenyl-3-yl]methoxy}benzyl)-1,2,4-oxadiazolidine-3,5-dione,
   2-({6-[(4'-{[(3R)-3-hydroxybutyl]oxy}-2,2',6'-trimethylbiphenyl-3-yl)methoxy]pyridin-3-yl}methyl)-1,2,4-oxadiazolidine-3,5-dione,
   2-({6-[(4'-{[(3S)-3-hydroxybutyl]oxy}-2,2',6'-trimethylbiphenyl-3-yl)methoxy]pyridin-3-yl}methyl)-1,2,4-oxadiazolidine-3,5-dione,
   2-{4-[(4'-{[(2R)-2-hydroxy-3-methoxypropyl]oxy}-2,2',6'-trimethylbiphenyl-3-yl)methoxy]benzyl}-1,2,4-oxadiazolidine-3,5-dione,
   2-[4-({3-[6-(3-hydroxy-3-methylbutoxy)-2,4-dimethylpyridin-3-yl]-2-methylbenzyl}oxy)benzyl]-1,2,4-oxadiazolidine-3,5-dione,
   2-[(6-{[4'-(2-hydroxyethoxy)-2,2',6'-trimethylbiphenyl-3-yl]methoxy}pyridin-3-yl)methyl]-1,2,4-oxadiazolidine-3,5-dione,
   2-(4-{[4'-(2-hydroxyethoxy)-2,2',5'-trimethylbiphenyl-3-yl]methoxy}benzyl)-1,2,4-oxadiazolidine-3,5-dione,
   2-[4-({[4'-(2-hydroxyethoxy)-2,2',6'-trimethylbiphenyl-3-yl]methyl}amino)benzyl]-1,2,4-oxadiazolidine-3,5-dione,
   2-{4-[(4'-{[(2S)-3-hydroxy-2-methoxypropyl]oxy}-2,2',6'-trimethylbiphenyl-3-yl)methoxy]benzyl}-1,2,4-oxadiazolidine-3,5-dione,
   2-{[6-({[4'-(2-hydroxyethoxy)-2,2',6'-trimethylbiphenyl-3-yl]methyl} amino)pyridin-3-yl]methyl}-1,2,4-oxadiazolidine-3,5-dione,
   2-(4-{[3-(6-{[(3R)-3-hydroxybutyl]oxy}-2,4-dimethylpyridin-3-yl)-2-methylbenzyl]amino}benzyl)-1,2,4-oxadiazolidine-3,5-dione or
   2-(4-{[3-(6-{[(3R)-3-hydroxybutyl]oxy}-2,4-dimethylpyridin-3-yl)-2-methylbenzyl]oxy}benzyl)-1,2,4-oxadiazolidine-3,5-dione,
   or a pharmaceutically acceptable salt thereof.
[14] The compound as described in [1] which is
   2-(4-{[(4'-{[(2S)-2,3-dihydroxypropyl]oxy}-2,2',6'-trimethylbiphenyl-3-yl)methyl]amino}benzyl)-1,2,4-oxadiazolidine-3,5-dione,
   2-(4-{[(4'-{[(2R)-2,3-dihydroxypropyl]oxy}-2,2',6'-trimethylbiphenyl-3-yl)methyl]amino}benzyl)-1,2,4-oxadiazolidine-3,5-dione,
   2-[4-({[5'-fluoro-4'-(2-hydroxyethoxy)-2,2'-dimethylbiphenyl-3-yl]methyl}amino)benzyl]-1,2,4-oxadiazolidine-3,5-dione,
   2-[4-({3-[6-(2-hydroxyethoxy)-2,5-dimethylpyridin-3-yl]-2-methylbenzyl}amino)benzyl]-1,2,4-oxadiazolidine-3,5-dione,
   2-(4-{[5'-fluoro-4'-(2-hydroxyethoxy)-2,2'-dimethylbiphenyl-3-yl]methoxy} benzyl)-1,2,4-oxadiazolidine-3,5-dione,
   2-(4-{[(4'-{[(2S)-2,3-dihydroxypropyl]oxy}-2,2',5'-trimethylbiphenyl-3-yl)methyl]amino}benzyl)-l,2,4-oxadiazolidine-3,5-dione,
   2-(4-{[(4'-{[(2R)-2,3-dihydroxypropyl]oxy}-2,2',5'-trimethylbiphenyl-3-yl)methyl]amino}benzyl)-1,2,4-oxadiazolidine-3,5-dione,
   2-(4-{[(4'-{[(3R)-3,4-dihydroxybutyl]oxy}-2,2',6'-trimethylbiphenyl-3-yl)methyl]amino}benzyl)-1,2,4-oxadiazolidine-3,5-dione or
   2-(4-{[(4'-{[(3S)-3,4-dihydroxybutyl]oxy}-2,2',6'-trimethylbiphenyl-3-yl)methyl]amino}benzyl)-1,2,4-oxadiazolidine-3,5-dione,
   or a pharmaceutically acceptable salt thereof.
[15] A pharmaceutical composition which comprises the compound as described in [1] or a pharmaceutically acceptable salt thereof, and a pharmaceutical acceptable excipient.
[16] A GPR40 agonist which comprises the compound as described in [1] or a pharmaceutically acceptable salt thereof.
[17] An insulin secretagogue which comprises the compound as described in [1] or a pharmaceutical acceptable salt thereof.
[18] A pharmaceutical composition for prevention and/or treating diabetes mellitus which comprises the compound as described in [1] or a pharmaceutically acceptable salt thereof.
[19] Use of the compound as described in [1] or a pharmaceutically acceptable salt thereof for the manufacture of an insulin secretagogue or an agent for preventing and/or treating diabetes mellitus.
[20] A method of promoting insulin secretion or a method of preventing and/or treating diabetes mellitus, which comprises administering an effective amount of the compound as described in [1] or a salt thereof to a patient.

Hereinafter, the present invention will be illustrated in detail.

In the specification, "C₁₋₆ alkyl" is linear or branched alkyl having 1 to 6 carbon atom(s), for example, methyl, ethyl, n-propyl, isopropyl, n-butyl, isobutyl, sec-butyl, tert-butyl, n-pentyl, n-hexyl or the like.

"Halogen" means F, Cl, Br and I.

"Cycloalkyl" is a "C₃₋₁₀ saturated hydrocarbon cyclic group, for example, cyclopropyl, cyclobutyl, cyclopentyl, cyclohexyl, cycloheptyl, cyclooctyl, adamantly group or the like. Another aspect is a cycloproyl, cyclobutyl, cyclopentyl or cyclohexyl group.

"Aryl" is a "C₆₋₁₄ aromatic hydrocarbon cyclic group, for example, a phenyl, naphthyl, tetrahydronaphthyl group or the like. Another aspect is a phenyl group.

In the specification, "may be substituted" means being unsubstituted or having 1 to 5 substituent(s), and another aspect means being unsubstituted or having 1 to 2 substituent(s). "Substituted" means having 1 to 5 substituent(s), and another aspect means having 1 to 2 substituent(s). In the case of having a plurality of substituents, they may be the same with or different from each other.

Here is one aspect of the present invention.
(1) (1-1) A compound in which R¹ is -H or methyl. (1-2) As another aspect, a compound in which R¹ is -H. (1-3) As a further aspect, a compound in which R¹ is methyl.
(2) (2-1) A compound in which R² is a group of the formula (II) or the formula (III). (2-2) As another aspect, a compound in which R² is a group of the formula (II).
(3) (3-1) A compound in which R² is a group of the formula (II), either one of A and B is -O-(C₁₋₆ alkyl which is substituted with one or more group(s) selected from G¹ group), the other of A or B is -H or -R³. (3-2) As another aspect, a compound in which R² is a group of the formula (II), either one of A and B is -O-(C₁₋₆ alkyl which is substituted with one or more group(s) selected from the group consisting of -NHCOR^{Z}, -NHCO-(cycloalkyl), -OH and -OR^{Z}), R^{Z} is C₁₋₆ alkyl which may be substituted with one or more -OH, and the other of A or B is -H, methyl or halogen. (3-3) As a further aspect, a compound in which R² is a group of the formula (II), either one of A and B is - O-(C₁₋₆ alkyl which is substituted with one or more group(s) selected from the group consisting of -NHCOR^{Z}, -NHCO-(cycloalkyl), -OH and -OR^{Z}), R^{Z} is C₁₋₆ alkyl which may be substituted with one or more -OH, the other of A or B is -H, and n is 2. (3-4) As a further aspect, a compound in which R² is a group of the formula (II), either one of A and B is -O-(C₁₋₆ alkyl which is substituted with one or more group(s) selected from the group consisting of -NHCOR^{Z}, -NHCO-(cycloalkyl), -OH and -OR^{Z}), R^{Z} is C₁₋₆ alkyl which may be substituted with one or more -OH, the other of A or B is methyl or halogen, and n is 1. (3-5) As a further aspect, a compound in which A is -O-(C₁₋₆ alkyl which is substituted with one or more group(s) selected from the group consisting of-NHCOR^{Z}, -NHCO-(cycloalkyl), -OH and -OR^{Z}), R^{Z} is C₁₋₆ alkyl which may be substituted with one or more -OH, and B is -H, methyl or halogen. (3-6) As a further aspect, a compound in which R² is a group of the formula (II), A is -O-(C₁₋₆ alkyl which is substituted with one or more group(s) selected from the group consisting of - NHCOR^{Z}, -NHCO-(cycloalkyl), -OH and -OR^{Z}), R^{Z} is C₁₋₆ alkyl which may be substituted with one or more -OH, B is -H, and n is 2. (3-7) As a further aspect, a compound in which R² is a group of the formula (II), A is -O-(C₁₋₆ alkyl which is substituted with one or more group(s) selected from the group consisting of -NHCOR^{Z}, -NHCO-(cycloalkyl), -OH and -OR^{Z}), R^{Z} is C₁₋₆ alkyl which may be substituted with one or more -OH, B is methyl or halogen, and n is 1. (3-8) As a further aspect, a compound in which R² is a group of the formula (II), A is -O-(C₁₋₆ alkyl substituted with one or more -OH), and B is -H, methyl or halogen. (3-9) As a further aspect, a compound in which R² is a group of the formula (II), A is -O-(C₁₋₆ alkyl substituted with one or more -OH), B is -H, and n is 2. (3-10) As a further aspect, a compound in which R² is a group of the formula (II), A is -O-(C₁₋₆ alkyl substituted with one or more -OH), B is methyl or halogen, and n is 1.
(4) (4-1) A compound in which R² is a group of the formula (II), R³ is the same with or different from each other and is methyl which may be substituted with one or more halogen, halogen or -O-methyl. (4-2) As another aspect, a compound in which R² is a group of the formula (II) and R³ is methyl.
(5) (5-1) A compound in which L¹ is CH or N. (5-2) As another aspect, a compound in which L¹ is CH. (5-3) As a further aspect, a compound in which L¹ is N.
(6) (6-1) A compound in which L² is O or NH. (6-2) As another aspect, a compound in which L² is O. (6-3) As a further aspect, a compound in which L² is NH.
(7) (7-1) A compound in which L³ is CH or N. (7-2) As another aspect, a compound in which L³ is CH. (7-3) As a further aspect, a compound in which L³ is N.
(8) (8-1) A compound in which R² is a group of the formula (II) and L⁴ is CH or N. (8-2) As another aspect, a compound in which R² is a group of the formula (II) and L⁴ is CH. (8-3) As a further aspect, a compound in which R² is a group of the formula (II) and L⁴ is N.
(9) A compound in consistent combination of two or more groups which are described in the above (1) to (8).

Compounds included in the present invention may include the following:
2-(4-{[(4'-{[(2S)-2,3-dihydroxypropyl]oxy}-2,2',6'-trimethylbiphenyl-3-yl)methyl]amino}benzyl)-1,2,4-oxadiazolidine-3,5-dione,
   2-(4-{[(4'-{[(2R)-2,3-dihydroxypropyl]oxy}-2,2',6'-trimethylbiphenyl-3-yl)methyl]amino}benzyl)-1,2,4-oxadiazolidine-3,5-dione,
   2-[4-({[5'-fluoro-4'-(2-hydroxyethoxy)-2,2'-dimethylbiphenyl-3-yl]methyl}amino)benzyl]-1,2,4-oxadiazolidine-3,5-dione,
   2-[4-({3-[6-(2-hydroxyethoxy)-2,5-dimethylpyridin-3-yl]-2-methylbenzyl}amino)benzyl]-1,2,4-oxadiazolidine-3,5-dione,
   2-(4-{[5'-fluoro-4'-(2-hydroxyethoxy)-2,2'-dimethylbiphenyl-3-yl]methoxy}benzyl)-1,2,4-oxadiazolidine-3,5-dione,
   2-(4-{[(4'-{[(2S)-2,3-dihydroxypropyl]oxy}-2,2',5'-trimethylbiphenyl-3-yl)methyl]amino}benzyl)-1,2,4-oxadiazolidine-3,5-dione,
   2-(4-{[(4'-{[(2R)-2,3-dihydroxypropyl]oxy}-2,2',5'-trimethylbiphenyl-3-yl)methyl]amino}benzyl)-1,2,4-oxadiazolidine-3,5-dione,
   2-(4-{[(4'-{[(3R)-3,4-dihydroxybutyl]oxy}-2,2',6'-trimethylbiphenyl-3-yl)methyl]amino}benzyl)-1,2,4-oxadiazolidine-3,5-dione, and
   2-(4-{[(4'-{[(3S)-3,4-dihydroxybutyl]oxy}-2,2',6'-trimethylbiphenyl-3-yl)methyl]amino}benzyl)-1,2,4-oxadiazolidine-3,5-dione.

As another aspect of compounds included in the present invention, the following compound may include:
2-[4-({2,2',6'-trimethyl-4'-[3-(propionylamino)propoxy]biphenyl-3-yl}methoxy)benzyl]-1,2,4-oxadiazolidine-3,5-dione,
   2-{4-[(4'-{3-[(cyclopropylcarbonyl)amino]propoxy}-2,2',6'-trimethylbiphenyl-3-yl)methoxy]benzyl}-1,2,4-oxadiazolidine-3,5-dione,
   2-{4-[(4'-{[(2S)-2,3-dihydroxypropyl]oxy}-2,2',6'-trimethylbiphenyl-3-yl)methoxy]benzyl}-1,2,4-oxadiazolidine-3,5-dione,
   2-{4-[(4'-{[(2R)-2,3-dihydroxypropyl]oxy}-2,2',6'-trimethylbiphenyl-3-yl)methoxy]benzyl}-1,2,4-oxadiazolidine-3,5-dione,
   2-(4-{[3'-(3-hydroxy-3-methylbutoxy)-2,2'-dimethylbiphenyl-3-yl]methoxy}benzyl)-1,2,4-oxadiazolidine-3,5-dione,
   2-(4-{[4'-(3-hydroxy-3-methylbutoxy)-2,2',5'-trimethylbiphenyl-3-yl]methoxy} benzyl)-1,2,4-oxadiazolidine-3,5-dione,
   2-(4-{[4'-(3-hydroxy-3-methylbutoxy)-2,2',3'-trimethylbiphenyl-3-yl]methoxy}benzyl)-1,2,4-oxadiazolidine-3,5-dione,
   2-({6-[(4'-{[(3R)-3-hydroxybutyl]oxy}-2,2',6'-trimethylbiphenyl-3-yl)methoxy]pyridin-3-yl}methyl)-1,2,4-oxadiazolidine-3,5-dione,
   2-({6-[(4'-{[(3S)-3-hydroxybutyl]oxy}-2,2',6'-trimethylbiphenyl-3-yl)methoxy]pyridin-3-yl}methyl)-1,2,4-oxadiazolidine-3,5-dione,
   2-{4-[(4'-{[(2R)-2-hydroxy-3-methoxypropyl]oxy}-2,2',6'-trimethylbiphenyl-3-yl)methoxy]benzyl}-1,2,4-oxadiazolidine-3,5-dione,
   2-[4-({3-[6-(3-hydroxy-3-methylbutoxy)-2,4-dimethylpyridin-3-yl]-2-methylbenzyl}oxy)benzyl]-1,2,4-oxadiazolidine-3,5-dione,
   2-[(6-{[4'-(2-hydroxyethoxy)-2,2',6'-trimethylbiphenyl-3-yl]methoxy}pyridin-3-yl)methyl]-1,2,4-oxadiazolidine-3,5-dione,
   2-(4-{[4'-(2-hydroxyethoxy)-2,2',5'-trimethylbiphenyl-3-yl]methoxy}benzyl)-1,2,4-oxadiazolidine-3,5-dione,
   2-[4-({[4'-(2-hydroxyethoxy)-2,2',6'-trimethylbiphenyl-3-yl]methyl} amino)benzyl]-1,2,4-oxadiazolidine-3,5-dione,
   2-{4-[(4'-{[(2S)-3-hydroxy-2-methoxypropyl]oxy}-2,2',6'-trimethylbiphenyl-3-yl)methoxy]benzyl}-1,2,4-oxadiazolidine-3,5-dione,
   2-{[6-({[4'-(2-hydroxyethoxy)-2,2',6'-trimethylbiphenyl-3-yl]methyl}amino)pyridin-3-yl]methyl}-1,2,4-oxadiazolidine-3,5-dione,
   2-(4-{[3-(6-{[(3R)-3-hydroxybutyl]oxy}-2,4-dimethylpyridin-3-yl)-2-methylbenzyl]amino}benzyl)-1,2,4-oxadiazolidine-3,5-dione, and
   2-(4-{[3-(6-{[(3R)-3-hydroxybutyl]oxy}-2,4-dimethylpyridin-3-yl)-2-methylbenzyl]oxy}benzyl)-1,2,4-oxadiazolidine-3,5-dione.

In the compound of the formula (I) and a pharmaceutically acceptable salt thereof (hereinafter, may be referred to as "compound of the formula (I)"), other tautomers or geometric isomers may exist depending on the kind of substituents. In the specification, although only one aspect of the isomers may be described, the present invention includes these isomers, and also includes separated isomers or mixtures thereof.

In addition, the compound of the formula (I) may have asymmetric carbon atoms or axial asymmetry, and optical isomers such (R)-form and (S)-form may exist on the basis of this. The present invention includes all of the mixtures and isolated forms of these optical isomers.

The present invention also includes a pharmaceutically acceptable prodrug of the compound of the formula (I). The pharmaceutically acceptable prodrug is a compound having a group which can convert into an amino group, a hydroxyl group, a carboxyl group or the like of the compound of the formula (I) by solvolysis or under physiological conditions. A group forming a prodrug may include a group described in, for example, "PROGRESS IN MEDICINE", 5, 2157-2161 (1985) and "Iyakuhin no Kaihatsu", (Hirokawa shoten, 1990) Vol. 7 Molecular Design 163-198.

Further, the compound of the formula (I) may form a salt with an acid addition salt or a base depending on the kind of substituents, and such salt is included in the present invention as long as the salt is a pharmaceutically acceptable salt. Specific examples thereof may include acid addition salts with an inorganic acid such as hydrochloric acid, hydrobromic acid, hydroiodic acid, sulfuric acid, nitric acid and phosphoric acid, etc., or an organic acid such as formic acid, acetic acid, propionic acid, oxalic acid, malonic acid, succinic acid, fumaric acid, maleic acid, lactic acid, malic acid, mandelic acid, tartaric acid, dibenzoyltartaric acid, ditoluoyltartaric acid, citric acid, methanesulfonic acid, ethanesulfonic acid, benzenesulfonic acid, p-toluenesulfonic acid, aspartic acid and glutamic acid, etc.; salts with an inorganic base such as sodium, potassium, magnesium, calcium and aluminum, etc., or an organic base such as methylamine, ethylamine, ethanolamine, lysine and ornithine etc.; salts with various amino acids such as acetylleucine etc. and an amino acid derivative; ammonium salts; and the like.

The present invention also includes substances of various hydrates, solvates and crystalline polymorphs of the compound of the formula (I) and a pharmaceutically acceptable salt. Moreover, the invention also includes various radiolabeled or nonradiolabeled compounds.

### (Production Method)

The compound of the formula (I) and a pharmaceutically acceptable salt thereof can be produced by applying various known synthetic methods by making use of the feature based on its basic skeleton or the kind of substituents. At the time, it may be effective in the producing technique that the relevant functional group is replaced by a proper protecting group (group capable of easily converting into the relevant functional group) at the stage of a raw material to an intermediate, depending on the kind of substituents.

Such protecting group may include, for example, protecting groups described in "Protective Groups in Organic Synthesis (the 3rd edition, 1999)" written by Greene and Wuts and the like, and may be optionally selected and used according to these reaction conditions. In the method like this, a desired compound can be obtained by removing the protecting group after introducing the protecting group to react.

The prodrug of the formula (I) can be produced by introducing the specific group at the stage of a raw material to an intermediate, or further reacting with the use of the obtained compound of the formula (I), the same as the above-mentioned protecting group. The reaction can be carried out by applying the method known to those skilled in the art such as normal esterification, amidation, dehydration or the like.

Hereinafter, typical production methods of the compound of the formula (I) will be illustrated. Each production method can be carried out by referring to reference documents attached to the explanation. In addition, the production method of the invention is not limited to the examples as follows.

Production method 1: cyclization reaction

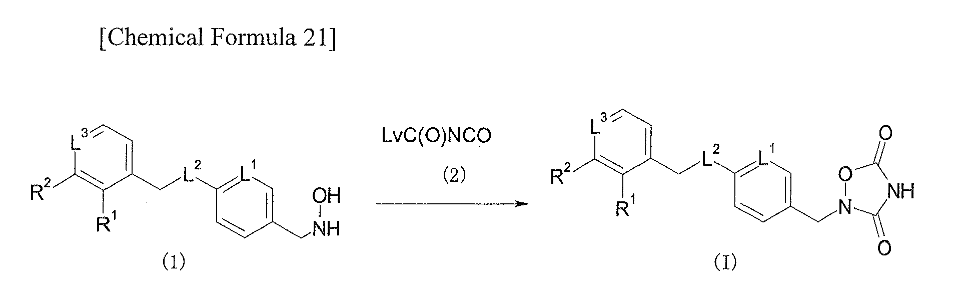

### (In the formula, Lv means a leaving group. The same shall apply hereinafter.)

The production method is a method for producing a compound (I) of the invention by a ring construction reaction using a compound (1) and a compound (2). For the leaving group of Lv, halogen such as chloro, bromo, etc.; and an alkoxy group such as methoxy, ethoxy, etc. are preferred. The reaction can be carried out by using the compound (1) and the compound (2), in which the compound have equal amount or one compound has excessive amount, in a solvent of ethers such as diethyl ether, tetrahydrofuran (THF), dioxane, dimethoxyethane (DME), etc.; halogenated hydrocarbons such as dichloromethane, 1,2-dichloroethane, chloroform, etc.; aromatic hydrocarbons such as benzene, toluene, xylene, etc.; or the like, under cooling, room temperature or heating.

In the case where the compound (1) has a hydroxyl group other than a hydroxyamino group, the hydroxyl group may be carbamoylated. The carbamoyl group can be removed by the method in which those skilled in the art usually use for decarbamoylation. More specifically, the method can be carried out, for example, using a base such as sodium methoxide, sodium ethoxide, sodium hydroxide, etc. in a solvent of an alcohol such as methanol, ethanol, etc., water, or the like under cooling, room temperature or heating.

### Production method 2: coupling reaction

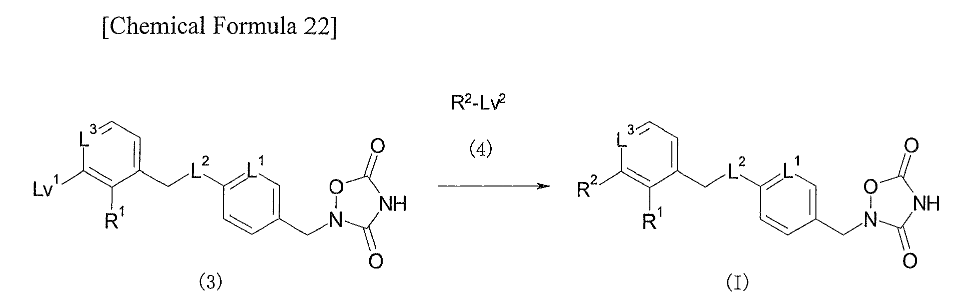

(In the formula, either one of Lv¹ and Lv² represents halogen or a trifluoromethanesulfonyloxy group, and the other represents -B(OH)₂, -B(OR⁰)₂ and-Sn(C₁₋₆ alkyl)₃; R⁰ means C₁₋₆ alkyl and two R⁰s may form C₂₋₆ alkylene. The same shall apply hereinafter.)

The production method is a method for producing a compound (I) of the invention by a coupling reaction between a compound (3) and a compound (4).

The reaction can be carried out by using the compound (3) and the compound (4), in which the compound have equal amount or one compound has excessive amount, using a palladium complex such as tetrakistriphenylphosphine palladium, palladium acetate or the like as a catalyst, in a solvent such as ethers, alcohols, halogenated hydrocarbons, aromatic hydrocarbons, water, etc. or mixed solvent thereof, under cooling, room temperature or heating. It may be favorable in smooth progress of the reaction that the reaction is carried out in the presence of a base such as sodium carbonate, cesium carbonate, sodium tert-butoxide or the like, or a lithium salt such as lithium chloride, lithium bromide or the like.

### Production method 3: reductive amination

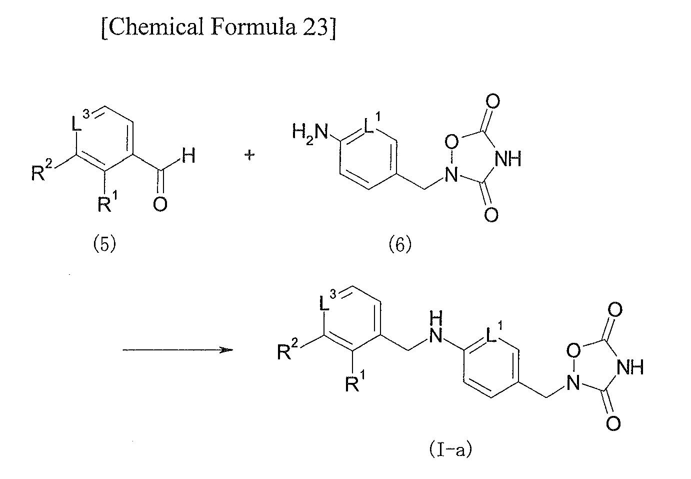

The production method is a method for producing a compound (I-a) of the invention by reductively aminating a compound (5) using a compound (6).

The reaction is carried out by stirring in the presence of a reducing agent in a solvent inert to the reaction under heating to reflux from -45°C, preferably at 0°C to room temperature for normally 0.1 hours to 5 days, using the compound (5) and the compound (6), in which the compound have equal amount or one compound has excessive amount. The solvent may include, for example, alcohols, ethers and mixtures thereof. The reducing agent may include sodium cyanoborohydride, sodium triacetoxyborohydride, sodium borohydride and the like. The reaction may be preferred to carry out in the presence of a dehydrating agent such as molecular sieves etc., or an acid such as acetic acids, hydrochloric acids, titanium (IV) isopropoxide complexes, etc. Depending on the reaction, in the case where an imine compound which is formed in the reaction system as an intermediate can be isolated stably, a reduction reaction may be separately carried out after obtaining the imine compound.

### [Document]

"Comprehensive Organic Functional Group Transformations II", Vol. 2, Elsevier Pergamon, 2005, by A. R. Katritzky and R. J. K. Taylor

"Jikken Kagaku Koza (5th Ed.)", edited by Nippon Kagakukai, Vol. 14 (2005) (Maruzen)

### Production method 4: other production methods

Further, some compounds of the formula (I) also can be produced by optionally combining adoptable processes such as known amidation, oxidation, hydrolysis and the like for those skilled in the art from compounds of the invention as obtained above. Specifically, the following reaction can be applied.

### 4-1: amidation

An amide compound can be obtained by a reaction of a carboxylic compound with an amine compound.

In this reaction, using the carboxylic compound and the amine compound, in which the compounds have equal amount or one compound has excessive amount, these mixture is stirred in the presence of a reducing agent in a solvent inert to the reaction under cooling to heating, preferably at -20 to 60°C for normally 0.1 hours to 5 days. Examples of the solvent to be used here may include, not limited in particular, aromatic hydrocarbons, halogenated hydrocarbons, ethers, *N,N*-dimethylformamide (DMF), dimethylsulfoxide (DMSO), dimethylacetamide (DMA), ethyl acetate, acetonitrile or water, and mixtures thereof. Examples of the condensing agent may include 1-(3-dimethylaminopropyl)-3-ethylcarbodiimide (EDCI) or a salt thereof, dicyclohexylcarbodiimide, 1,1'-carbonyldiimidazole, diphenylphosphoryl azide and phosphorous oxychloride, but it is not limited to these. It may be favorable to use additives (e.g., 1-hydroxybenzotriazole) in smooth progress of the reaction. It may be favorable in smooth progress of the reaction that the reaction is carried out in the presence of an organic base such as triethylamine, *N,N*-diisopropylethylamine and *N-*methylmorpholine, etc., or an inorganic base such as potassium carbonate, sodium carbonate, potassium hydroxide, etc.

The method to react with an amine compound after converting a carboxylic compound into a reactive derivative can be used. Examples of the reactive derivative of the carboxylic acid may include an acid halide obtained by a reaction with a halogenating agent such as phosphorous oxychloride, thionyl chloride or the like; mixed acid anhydride obtained by a reaction with isobutyl chloroformate or the like; active ester obtained by condensation with 1-hydroxybenzotriazole or the like; and the like. The reaction of these reactive derivatives with the amine compound can be carried out in a solvent inert to the reaction of halogenated hydrocarbons, aromatic hydrocarbons, ethers or the like under cooling to heating, preferably at -20 to 60°C.

### 4-2: oxidation

A sulfoxide compound or a sulfone compound can be produced by oxidizing the S atom of a sulfide compound with various oxidizing agents. The reaction can be carried out, for example, by using m-chloroperbenzoic acid, peracetic acid, a hydrogen peroxide solution, Dess-Martin reagent (1,1,1-triacetoxy-1,1-dihydro-1,2-benziodoxol-3(*1H*)one) or the like as an oxidizing agent with an equal amount to excessive amount in a solvent such as halogenated hydrocarbons, acetic acid, water etc., under cooling, room temperature or heating.

### 4-3: hydrolysis

A compound having a carboxyl group can be produced by hydrolysis of a compound having an ester group. For example, the reaction can be carried out in a solvent inert to the reaction such as aromatic hydrocarbons, ethers, halogenated hydrocarbons, alcohols, DMF, DMA, *N-*methyl-2-pyrrolidinone (NMP), DMSO, pyridine, water, etc., in the presence of an acid such as mineral acid such as sulfuric acid, hydrochloric acid, hydrobromic acid, etc.; an organic acid such as formic acid, acetic acid, etc., or the like; or in the presence of a base such as lithium hydroxide, sodium hydroxide, potassium hydroxide, potassium carbonate, sodium carbonate, cesium carbonate, ammonia, etc., under cooling or heating.

### (Production Method of raw material)

A raw material to be used for producing the invention can be manufactured by applying, for example, the following method, a method described in production methods as follows, known methods or obvious methods for those skilled in the art, or modified methods thereof.

### Synthesis of raw materials

### Synthesis of raw material 1: O-allcylation

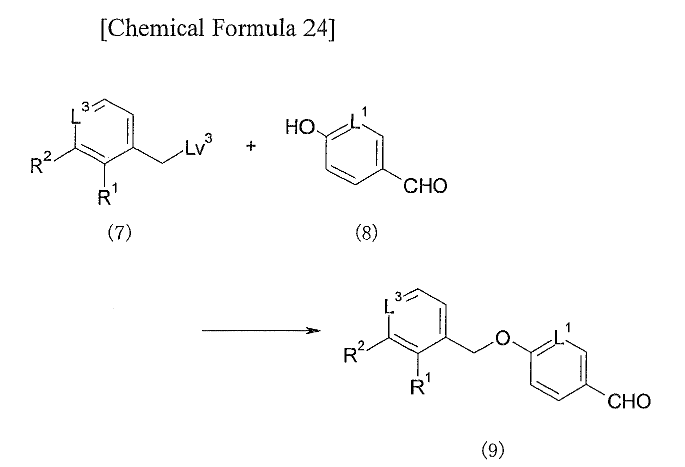

(In the formula, Lv³ represents -OH, or a leaving group such as halogen, methanesulfonyloxy, *p*-toluenesulfonyloxy or the like. The same shall apply hereinafter.)

The production method is a method for obtaining a compound (9) by O-alkylating a compound (8) with a compound (7).

In the case of using the compound (7) whose Lv³ is -OH, the method can be carried out by Mitsunobu reaction in which those skilled in the art normally use. More specifically, the method can be carried out using an activating agent which is regulated by a phosphorous compound such as tributylphosphine, triphenylphosphine, etc. and an azodicarbonyl compound such as diethyl azodicarboxylate, 1,1'-(diazocarbonyl)dipiperidine, etc., or a reagent such as cyanomethylene tributylphosphorane, or the like, in a solvent such as halogenated hydrocarbons, ethers, aromatic hydrocarbons, etc., under cooling, room temperature or heating.

In the case of the compound (7) in which Lv³ is a leaving group such as halogen, methanesulfonyloxy, *p*-toluenesulfonyloxy or the like, the method can be carried out by using the compound (7) and the compound (8), in which the compound have equal amount or one compound has excessive amount, for example, in the presence of a base such as potassium carbonate, cesium carbonate, sodium methoxide, sodium hydride, etc. in a solvent such as halogenated hydrocarbons, ethers, aromatic hydrocarbons, etc., DMF, and the like, under cooling, room temperature or heating.

### Synthesis of raw material 2

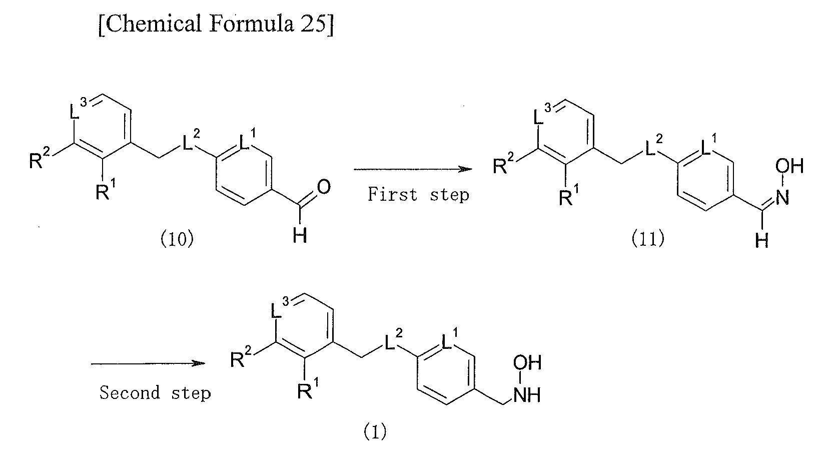

### Process 1: oxime formation

The process is a process for obtaining a compound (11) by oxime formation of a compound (10).

Methods of oxime formation in which those skilled in the art normally use can be applied for the oxime formation. More specifically, the method can be carried out using the compound (10) and hydroxylamine or salts thereof, in which they have equal amount or either one of them has excessive amount, in a solvent such as alcohols, acetic acid, pyridine, water, etc. under cooling, room temperature or heating. Depending on the kind of the compound, it may be advantageous to add sodium acetate, p-toluenesulfonic acid or the like for smooth progress of the reaction.

### Process 2: reduction

The process is a process for obtaining a compound (1) by reduction of a compound (11).

Methods of reduction of oxime in which those skilled in the art normally use can be applied for the reduction reaction of oxime. More specifically, the reaction can be carried out using the compound (11) and a reducing agent such as a borane-pyridine complex, sodium cyanoborohydride, etc., in which they have equal amount or either one of them has excessive amount, in a solvent such as ethers, alcohols, aromatic hydrocarbons, etc. acetic acid, and the like, under cooling, room temperature or heating.

The compound of the formula (I) is isolated as a substance of a free compound, a pharmaceutically acceptable salt thereof, a hydrate, a solvate or a crystalline polymorph, thereby purifying. The pharmaceutically acceptable salt of the compound of the formula (I) also can be produced by a conventional salt forming reaction.

Isolation and purification are carried out by applying normal chemical operations such as extraction, fractional crystallization, fractional chromatography of many types or the like.

Various isomers can be produced by selecting a proper raw material, or separated by making use of the difference of physiochemical properties between isomers. For example, an enantiomer can be led to a pure isomer by a general optical resolution (e.g., fractional crystallization leading to diastereomeric salt with an optically-active base or acid, or chromatography using a chiral column, etc., or the like). In addition, the isomer also can be produced from a proper optically-active raw material.

The pharmacological activity of the compound of the formula (I) was confirmed by the following tests. Unless otherwise noted, test examples as described below can be carried out in accordance with known methods. When a commercially available reagent, kit, etc. is used, the test examples can be carried out in accordance with the instruction of the commercial product.

### Test method 1: measurement of GPR40 agonistic activity

### i) Cloning of human GPR40

In accordance with procedures as described below, the full-length sequence of GPR40 was obtained by a polymerase chain reaction (PCR) to set human genomic DNA (Clontech) as a template.

Oligonucleotide which consists of a base sequence represented by SEQ ID NO. 1 was used as a forward primer, and oligonucleotide which consists of a base sequence represented by SEQ ID NO. 2 was used as a reverse primer. To each 5' terminus of the forward primer and the reverse primer described above, a base sequence including the XbaI recognition site is attached. In the PCR, using Taq DNA polymerase (Ex Taq DNA polymerase; TAKARA BIO INC.), a cycle consisting of 94°C (15 sec)/55°C (30 sec)/72°C (1 min) was repeated 30 times in the presence of 5% DMSO. As a result, the DNA fragment of about 0.9 kbp was amplified. This DNA fragment was digested with XbaI, followed by inserting into the XbaI site of plasmid pEF-BOS-dhfr (Nucleic Acids Research, 18, 5322, 1990) to obtain plasmid pEF-BOS-dhfr-GPR40.

The base sequence of GPR40 gene in plasmid pEF-BOS-dhfr-GPR40 was decided by a dideoxy terminator method using a DNA sequencer (ABI377 DNA Sequencer; Applied Biosystems). The base sequence of GPR40 gene was the same with the base sequence represented by SEQ ID NO. 3. The base sequence represented by SEQ ID NO. 3 had an open reading frame (ORF) of a 903 base, an amino acid sequence predicted from this ORF (300 amino acid) was the same with the amino acid sequence represented by SEQ ID NO. 4

### ii) Preparation of GPR40 stable expression cell

A CHO dhfr cell (CHO cell deleting a dihydrofolate reductase (dhfr) gene) was used as a cell in which GPR 40 protein was expressed. In addition, the plasmid pEF-BOS-dhfr-GPR40 obtained in the above i) was used as an expressing plasmid to express GPR40 protein. To a 6 well plate (Asahi Techno Glass Corp.), the CHO dhfr cells were seeded to become 80 to 90% confluent in an αMEM medium having 10% fetal calf serum (FCS), and cultivated overnight. Then, 2µg of plasmid pEF-BOS-dhfr-GPR40 per well was transferred generetically thereto using a transfection reagent (Lipofectamine 2000; Invitrogen Corporation). After cultivating for 24 hours subsequent to the gene transfer, the cell were diluted and reseeded. On this occasion, the αMEM medium having 10% FCS was changed for an αMEM medium having 10% FCS and no nucleic acid. After 20 days cultivation, formed cell colonies were collected respectively and cultivated, thereby obtaining CHO cells stably expressing GPR40. Among these, cells high-reactive to an oleic acid and a linoleic acid which were intrinsic ligands were selected.

### iii) Measurement of GPR40 agonistic activity

The GPR40 agonistic activity was measured with fluctuation of the intracellular calcium concentration as an index by FLIPR (registered trademark, Molecular Devices Corporation). Hereinafter, test methods will be described.

A CHO cell line expressing human GPR40 was seeded with 6×10³ per well to a 384-well black plate (Becton, Dickinson and Company), and cultivated in a CO₂ incubator overnight. For a luminescent pigment, a Calcium-3 assay kit (Molecular Devices Corporation) was used, and dissolved in 10 ml of a HBSS-HEPES buffer (PH7.4, 1×HBSS, 20 mM HEPES, Invitrogen Corporation) for a bottle. 35.68mg of probenecid (Sigma) was dissolved in 250 µl of 1M NaOH, and then 250 µl of a HBSS-HEPES buffer was added to blend. 16 ml of a HBSS-HEPES buffer, 640 µl of the luminescent pigment and 32 µl of probenecid were added to blend for a solution of the luminescent pigment per plate. The medium of the plate was removed, 40 µl of the solution of the luminescent pigment per well was dispensed, followed by incubating at room temperature for 2 hours. A compound to be inspected was dissolved in DMSO, and diluted with a HBSS-HEPES buffer. A reaction was initiated by 10 µl of dispensation, the fluctuation of the intracellular calcium concentration was measured by FLIPR. An EC₅₀ value of the compound to be inspected was calculated from the dose-response curve of change in fluorescence intensity one minute after measurement.

Consequently, the compound of the invention showed GPR40 agonistic activity. The EC₅₀ values of some compounds according to the invention are shown in Table 1. Ex represents example compound numbers.

**[Table 1]**

| Ex | EC₅₀ (µM) | EX | EC₅₀ (µM) | Ex | EC₅₀ µM |
|---|---|---|---|---|---|
| 7 | 0.41 | 61 | 0.094 | 89 | 0.11 |
| 21 | 0.60 | 63 | 0.38 | 94 | 0.64 |
| 26 | 0.41 | 68 | 0.022 | 96 | 0.19 |
| 27 | 0.23 | 69 | 0.069 | 97 | 0.19 |
| 31 | 0.069 | 70 | 0.070 | 101 | 0.23 |
| 32 | 0.068 | 74 | 0.32 | 102 | 0.23 |
| 44 | 0.19 | 80 | 0.25 | 107 | 0.093 |
| 45 | 0.28 | 81 | 0.16 | 119 | 0.81 |
| 56 | 0.82 | 84 | 0.28 | | |
| 60 | 0.16 | 85 | 0.42 | | |

### Test method 2: insulin secretagogue action using MIN6 cell

Insulin secretagogue action of the compound to be inspected was examined using MIN6 cells which were mouse pancreatic P cell lines. Hereinafter, the test method will be described.

MIN6 cells were seeded to set 5×10⁴/well (200 µl) to a 96-well plate. For the medium, DMEM (25 mM glucose) containing 10% FBS, 55 µM of 2-mercaptoethanol, 100 U/ml of penicillin and 100 µg/ml of streptomycin was used. 2 days later, the medium was removed by an aspirator, the plate was washed once with 200 µl of KRB-HEPES (116 mM NaCl, 4.7 mM KCl, 1.2 mM KH₂PO₄, 1.2 mM MgSO₄, 0.25 mM CaCl₂, 25 mM NaHCO₃, 0.005% FFA Free BSA, 24 mM HEPES (pH 7.4)) containing 2.8 mM glucose which was warmed to 37°C, and then reincubated using 200 µl of the same buffer solution at 37°C for 1 hour. The above buffer solution was removed by the aspirator, and the plate was washed with a buffer solution (200 µl) again. Thereafter, the compound to be inspected having fixed concentration was added to KRB-HEPES having 2.8 mM or 22.4 mM glucose, followed by adding 100 µl to each well to incubate at 37°C for 2 hours. The above sample was taken, diluted 100 times, and the concentration of insulin was quantitated by using an insulin RIA kit (Amersham RI).

As a result, some compound of the invention exhibited 120% or more insulin secretagogue action in the test, and it was confirmed that the compound of the invention had the excellent action.

### Test method 3: single oral glucose tolerance test on normal mice

The anti-hyperglycemic action after glycemic load of the compound to be inspected was examined using normal mice. Hereinafter, the test method will be described.

Male ICR mice which was preparedly reared for one week (6-week-old) was fasted overnight, and used as an animal to be inspected. A compound to be inspected was 0.5% methylcellulose suspension, and the suspension was orally administered 30 minutes before the glucose (2 g/kg) load. With regard to some compounds to be inspected, prior blood drawing was performed (0 min value), and blood glucose levels were measured at 5, 15, 30, 60 and 120 minutes later glucose load when the compound to be inspected was orally administered. A control group was given 0.5% methylcellulose. The evaluation was carried out in the following manner.

(Evaluation 1) The reduction rate of blood glucose levels after glucose loading (%) with respect to the control group was calculated at a point of glucose load which is 30 minutes after the oral administration of 10 mg/kg of the compound to be inspected. As a result, some compounds of the invention exhibited anti-hyperglycemic action, and it was confirmed that the compound of the invention had the excellent action. The results were shown in Table 2. Ex represents example compound numbers.

**[Table 2]**

| Ex | Reduction rate of blood glucose level (%) | Ex | Reduction rate of blood glucose level (%) | Ex | Reduction rate of blood glucose level (%) |
|---|---|---|---|---|---|
| 7 | 30 | 61 | 43 | 89 | 25 |
| 21 | 26 | 63 | 22 | 94 | 39 |
| 26 | 27 | 68 | 23 | 96 | 23 |
| 27 | 42 | 69 | 34 | 97 | 29 |
| 31 | 50 | 70 | 31 | 101 | 39 |
| 32 | 47 | 74 | 23 | 102 | 48 |
| 44 | 34 | 80 | 26 | 119 | 26 |
| 45 | 35 | 81 | 33 | | |
| 56 | 29 | 84 | 35 | | |
| 60 | 45 | 85 | 24 | | |

(Evaluation 2) The reduction rate of blood glucose levels after glucose loading (%) with respect to the control group was calculated at a point of glucose load which is 30 minutes after the oral administration of 10, 3, 1, 0.3 or 0.1 mg/kg of the compound to be inspected. The minimum dose in which the reduction rate of blood glucose levels after glucose loading showed 20% or more, or the minimum dose which showed significant decrease to the control group (Dunnet multiple comparison test) was to be a minimum effective dose (MED). The results are shown in Table 3. Ex represents example compound numbers. In addition, MED describing "below" in Table 3 means that a test was not carried out due to less than dose described.

**[Table 3]**

| Ex | MED (mg/kg) | Ex | MED (mg/kg) | Ex | MED (mg/kg) |
|---|---|---|---|---|---|
| 7 | 10 | 61 | 3 | 89 | 3 |
| 21 | 3 | 63 | Below 1 | 94 | 3 |
| 26 | 10 | 68 | 3 | 96 | 10 |
| 27 | 1 | 69 | 1 | 97 | 10 |
| 31 | 1 | 70 | 10 | 101 | 3 |
| 32 | 1 | 74 | 3 | 102 | 10 |
| 44 | Below 0.3 | 80 | 10 | 119 | 3 |
| 45 | 1 | 81 | Below 3 | | |
| 56 | 3 | 84 | Below 3 | | |
| 60 | Below 3 | 85 | 3 | | |

### (Evaluation 3)

The area under the blood concentration-time curve (AUC) of blood glucose levels for 0 to 120 minute(s) was calculated from the blood glucose levels before the blood drawing, and the blood glucose levels at 5, 15, 30, 60, and 120 minutes after the glucose load when 30, 10, 3, 1 or 0.3 mg/kg of the compound to be inspected was orally administered. Then, a dose which showed a significant (Dunnet multiple comparison test) decrease to the control group and a dose in which reduction rate of blood glucose levels after glucose loading was 20% (ED₂₀ value) were calculated. As a result, the comparative compound (compound of Example 36 described in International Publication No. WO 2005/087710 Pamphlet) showed significant decrease in the blood glucose level at a dose of 30 mg/kg, and the ED₂₀ value thereof was 17.8 mg/kg. Meanwhile, one or more compounds of the invention showed significant decrease in the blood glucose level in spite of a dose of 3 mg/kg or less, and the ED₂₀ value thereof was 3 mg/kg or less.

As the result of each test described above, the compound of the invention has excellent GPR40 receptor agonistic action, therefore, it is obvious that the compound of the invention is useful for insulin secretagogues and an agent for preventing and/or treating diseases associated with GPR40 such as for diabetes mellitus (insulin-dependent diabetes mellitus (type 1 diabetes), non-insulin-dependent diabetes mellitus (type 2 diabetes), borderline thereof (impaired glucose tolerance/ fasting blood glucose)) and the like.

A pharmaceutical composition containing one or more kind(s) of the compound of the formula (I) or a pharmaceutically acceptable salt thereof as an active ingredient can be prepared by a usually used method using a usually used excipient for medicine, a carrier for medicine or the like.

Administration can be any forms such as oral administration with tablets, pills, capsules, granules, powder medicine, solution or the like; intra-articular, intravenous, intramuscular or the like injectables; or parenteral administration with suppositories, eye-drops, eye ointments, transdermal solutions, ointments, transdermal patches, transmucosal solutions, transmucosal patches, inhalants or the like.

As a solid composition for oral administration, pills, powder medicines, granules or the like was used. In such solid composition, one or more kind(s) of active ingredients are mixed with at least one kind of an inert excipient, for example, lactose, mannitol, glucose, hydroxypropylcellulose, microcrystalline cellulose, starch, polyvinylpyrrolidone and/or magnesium metasilicate alminate, etc.. The composition may have inert additives, for example, lubricants such as magnesium stearate, disintegrants such as sodium carboxymethyl starch, etc., stabilizers and solubilizing agents by the law of the art. Tablets or pills may be coated with a film which is sugarcoated, soluble in the stomach or enteric, if needed.

A liquid composition for oral administration contains a pharmaceutically acceptable opalizer, solution, suspension agent, syrup, elixir or the like, and includes a generally used inert diluents, for example, purified water or ethanol. The liquid composition may contain auxiliary substances such as solubilizing agent, humectants and suspension agent, sweetener, flavor, fragrance and antiseptic agent.

The parenteral injectables contain an aseptic aqueous or nonaqueous solution, a suspension agent or an opalizer. As the aqueous solution, for example, distilled water for injection or physiological saline is included. As the nonaqueous solution, for example, there are vegetable oils such as propylene glycol, polyethylene glycol and olive oil; alcohols such as ethanol; polysorbate 80 (official name); and the like. Such composition further may include a tonicity agent, an antiseptic agent, a humectant, an emulsifier, a dispersant, a stabilizer or a solubilizing agent. These are sterilized by filtration through a bacteria filter, blend of antiseptic agents or irradiation. In addition, these produce antiseptic solid composition, and the composition can be used by suspending in antiseptic water or an antiseptic solvent for injection before use.

As the external preparation includes ointments, plasters, creams, jellies, adhesive skin patches, air sprays, lotions, eye-drops, eye-ointments and the like. Generally used ointment bases, lotion bases, aqueous or nonaqeous solutions, suspension agents, emulsion or the like are included. More specifically, the ointment or the lotion base includes polyethylene glycol, propylene glycol, white petrolatum, bleached wax, polyoxyethylene hydrogenated castor oil, glyceryl monostearate, stearyl alcohol, cetyl alcohol, lauromacrogol, sorbitan sesquioleate and the like.

The transmucosal agent such as an inhalant, a transnasal agent, and the like is used in the form of solid, liquid or semi-solid, and can be produced in accordance with conventional known methods. For example, a known excipient, further, a pH adjuster, an antiseptic agent, a surfactant, a lubricant, a stabilizer, a thickener, or the like may be optionally added. A device for proper inhalation or insufflations can be used for the administration. For example, using known devices such as a metered-dose inhaler, etc. or a nebulizer, the compound is administered alone, as a powder of a prescribed mixture, or a solution or suspending solution which is combined with a pharmaceutically carrier. A dry powder inhaler or the like may be for single or multiple administration, and dry powder or a capsule having powder is available. Alternatively, a form of pressured aerosol spray using a preferred gas such as chlorofluoroalkane, hydrofluoroalkane, carbon dioxide, etc. as a proper propellant, or the like can be used.

In the case of normal oral administration, the daily dosage is proper to be approximately 0.0001 to 50 mg/kg per weight, preferably approximately 0.001 to 10 mg/kg, more preferably 0.01 to 1 mg/kg, and administered at one time or in 2 to 4 divided doses. In the case of intravenous administration, the daily dosage is proper to be approximately 0.0001 to 3 mg/kg per weight, preferably approximately 0.0001 to 0.3 mg/kg, and administered at one time or in multiple divided doses. The dosage is arbitrarily determined depending on an individual case in consideration of symptoms, age, sex and the like.

The compound of the formula (I) can be used in combination with various therapeutic agents or agents for preventing a disease expected that the above compound of the formula (I) shows the efficacy. In the combination use, the administration can be concurrent, separate and continuous, or at desired intervals. The concurrently-administered preparation can be combination preparation or formulated separately.

The medicine of possible combination can include insulin, GLP-1 receptor agonist, a SU agent, a DPP4 inhibitor, an α glycosidase inhibitor, a SGLT inhibitor, a biguanide agent and an insulin sensitizer. Concretely, the medicine can include byetta, glibenclamide, glimepiride, sitagliptin, vildagliptin, acarbose, voglibose, metformin, pioglitazone and the like.

### EXAMPLES

Hereinbelow, the production method of the formula (I) will be illustrated in more detail with reference to Examples. The invention is not limited to compounds described in the following Examples. In addition, the production methods of the raw material compound are shown in Production Examples. Further, the production method of the formula (I) is not limited to the production method of concrete Examples showing as follows, and the compound of the formula (I) may be produced by the combination of these production methods, or the methods obvious for those skilled in the art.

In Examples, Production Examples and the following Tables, abbreviation as described below may be used.
PEx: Production Example number, Ex: Example number, Str: structural formula (when HCl or H₂SO₄ exists in the structural formula, it means that the compound is each hydrochloride or hydrosulfate.), Syn: production method (in the case of only figures, it represents the example number of the compound produced similarly, and in the case where p exists in front of figures, it represents the production example number of the compound produced similarly.), Data: physiochemical date (NMR1: δ(ppm) of ¹H NMR in DMSO-d₆, NMR2: δ(ppm) of ¹H NMR in CDCl₃, NMR3: δ(ppm) of ¹H NMR in CD₃OD, FAB+: FAB-MS [M+H]⁺, FAB-: FAB-MS [M-H]⁻, ESI+: ESI-MS [M+H]⁺, ESI-: ESI-MS [M-H]⁻, APCI+: APCI-MS [M+H]⁺, APCI-: APCI-MS [M-H]⁻, EI: EI-MS [M]+, CI: CI-MS [M+H]⁺), Me: methyl, Et: ethyl, Boc: *tert*-butoxycarbonyl, TBS: *tert*butyldimethylsilyl, and Ac: acetyl.

### Production Example 1

To a mixture of tert-butyl [5-(hydroxymethyl)pyridine-2-yl]carbamate (2.13 g), triethylamine (5.3 ml), and DMSO (15 ml), a sulfur trioxide-pyridine complex (3.02 g) in a DMSO solution (15 ml) was added dropwise, followed by stirring at room temperature for 4.5 hours. To the reaction mixture, water was added, followed by extraction with ethyl acetate. The organic layer was washed with a saturated aqueous solution of sodium chloride, and dried over anhydrous magnesium sulfate. After removing the desiccant, the solvent was evaporated under reduced pressure. The obtained residue was purified by silica gel column chromatography (hexane-ethyl acetate) to obtain 2.00 g of tert-butyl (5-formylpyridin-2-yl)carbamate.

### Production Example 2

To a mixture of tert-butyl (5-formylpyridin-2-yl)carbamate (1.99 g), THF (20 ml), and methanol (20 ml), a solution of hydroxylamine hydrochloride (747 mg) and sodium acetate (955 mg) in water (4 ml) was added dropwise, followed by stirring at room temperature for 1 hour. To the reaction mixture, a saturated aqueous solution of sodium bicarbonate was added, followed by extraction with chloroform. The organic layer was dried over anhydrous magnesium sulfate, and then the desiccant was removed. The solvent was evaporated under reduced pressure. To the obtained residue, acetic acid (50 ml) and sodium cyanoborohydride (4.90 g) were added, followed by stirring at room temperature for 27 hours. The reaction mixture was diluted with chloroform, alkalified by adding an 1 M aqueous solution of sodium hydroxide, and then extracted with chloroform-methanol (4:1). The organic layer was dried over anhydrous magnesium sulfate, and then the desiccant was removed. The solvent was evaporated under reduced pressure. The obtained residue was purified by silica gel column chromatography (chloroform-methanol) to obtain a solid (0.79 g). Thereto, THF (10 ml) and chlorocarbonyl isocyanate (0.293 ml) were added, followed by stirring at room temperature for 1.5 hours. The precipitated solid was collected by filtration, and dried by heating under reduced pressure, to obtain 805 mg of tert-butyl {5-[(3,5-dioxo-1,2,4-oxadiazolidin-2-yl)methyl]pyridine-2-yl}carbamate hydrochloride.

### Production Example 3

To a solution of tert-butyl {5-[(3,5-dioxo-1,2,4-oxadiazolidin-2-yl)methyl]pyridine-2-yl}carbamate hydrochloride (773 mg) in methanol (5 ml), a 4 M solution of hydrogen chloride in dioxane (15 ml) was added, followed by stirring at room temperature overnight. To the obtained residue, ethyl acetate was added, the precipitated solid was collected by filtration, and dried by heating under reduced pressure, to obtain 712 mg of 2-[(6-aminopyridine-3-yl)methyl]-1,2,4-oxadiazolidin-3,5-dione hydrochloride.

### Production Example 4

To a mixture of 3-bromo-2-methylphenol (2.60 g), imidazole (1.23 g) and DMF (30 ml), tert-butyl(dimethyl)silyl chloride (2.70 g) was added at room temperature, followed by stirring at room temperature for two days. To the reaction mixture, hexane (30 ml) and water (100 ml) were added, followed by extraction with diethyl ether. The organic layer was washed with a saturated aqueous solution of sodium chloride, and dried over anhydrous magnesium sulfate. After removing the desiccant, the solvent was evaporated under reduced pressure. The obtained residue was purified by silica gel column chromatography (hexane) to obtain 3.86 g of (3-bromo-2-methylphenoxy)(tert-butyl)dimethylsilane.

### Production Example 5

To a solution of (3-bromo-2-methylphenoxy)(tert-butyl)dimethylsilane (3.85 g) in THF (40 ml), a 1.57 M solution of n-butyllithium in hexane (9.0ml) was added dropwise at -75°C. The reaction mixture was stirred at -75°C for 1 hour. To the reaction mixture, a THF (7 ml) solution of triisopropyl borate (3.6 ml) was added dropwise at -75°C. After stirring the reaction mixture at -75°C for 1 hour, the temperature was raised to room temperature during a course of three hours. After methanol (7 ml) was added to the reaction mixture, the reaction mixture was poured into 1 M hydrochloric acid (30 ml), followed by extraction with ethyl acetate. The organic layer was washed with saturated aqueous solution of sodium chloride, and dried over anhydrous magnesium sulfate. After removing the desiccant, the solvent was evaporated under reduced pressure. The obtained residue was purified by silica gel column chromatography (hexane-ethyl acetate) to obtain 3.05 g of (3-{[tert-butyl(dimethyl)silyl]oxy}-2-methylphenyl)boronic acid.

### Production Example 6

Under a nitrogen atmosphere, a mixture of methyl 3-bromo-2-methylbenzoate (53.00 g), 4,4,4',4',5,5,5',5'-octamethyl-2,2'-bis-1,3,2-dioxaborolane (88.10 g), bistriphenylphosphine palladium(II) dichloride (8.12 g), triphenylphosphine (6.07 g), potassium acetate (68.10 g) and dioxane (530 ml) was stirred at 100°C for 29 hours, and then cooled to room temperature. The obtained reaction mixture was filtrated over Celite and washed with ethyl acetate. The obtained filtrate was concentrated under reduced pressure, and the obtained residue was purified by silica gel column chromatography (hexane-ethyl acetate) to obtain 54.00 g of methyl 2-methyl-3-(4,4,5,5-tetramethyl-1,3,2-dioxaborolane-2-yl)benzoate.

### Production Example 7

Under a nitrogen atmosphere, to a solution of tert-butyl(3,5-dimethoxyphenoxy)dimethylsilane (10.00 g) in THF (80 ml), a 1.6 M solution of n-butyllithium in hexane (26 ml) was added dropwise at -78°C. After raising the temperature of the reaction mixture to room temperature, followed by stirring at the same temperature for 5 hours, the reaction mixture was cooled once more to -78°C. Next, a solution of 2-isopropoxy-4,4,5,5-tetramethyl-1,3,2-dioxaborolane (9.12 ml) in THF (20 ml) was added dropwise to the reaction mixture, the temperature of the reaction mixture was raised to room temperature, and followed by stirring at the same temperature for 3 hours. The reaction mixture was cooled in an ice-methanol bath, and diethyl ether (100 ml) and water (250 ml) were added, followed by extraction with ethyl acetate. The organic layer was washed with a saturated aqueous solution of sodium chloride, and dried over anhydrous magnesium sulfate. After removing the desiccant by filtration, the filtrate was concentrated under reduced pressure. To the obtained residue, methanol (6 ml) was added then cooled. The solid was collected by filtration, washed with a small amount of cooled methanol, and dried by heating under reduced pressure, to obtain 6.67 g of tert-butyl[3,5-dimethoxy-4-(4,4,5,5-tetramethyl-1,3,2-dioxaborolane-2-yl)phenoxy]dimethylsilane.

### Production Example 8

To a solution of 3-(hydroxymethyl)-6-methylpyridine-2(1H)-one (5.77 g) in acetic acid (50 ml), 10% palladium on carbon (50% hydrated, 4.42 g) was added and stirred under 4 kg/cm² hydrogen atmosphere at room temperature for 1 hour. The catalyst was removed by Celite filtration and washed with ethanol. The filtrate was concentrated under reduced pressure to obtain 6.26 g of 3,6-dimethylpyridin-2(1H)-one.

### Production Example 9

To a solution of 3,6-dimethylpyridine-2(1H)-one (7.23 g) in acetic acid (60 ml), a solution of bromine (2.6 ml) in acetic acid (25 ml) was added dropwise at around 10°C. The temperature of the reaction mixture was raised to room temperature, followed by stirring for 1 hour. The reaction mixture was concentrated under reduced pressure, and a saturated aqueous solution of sodium bicarbonate (100 ml) was slowly added to the obtained residue. Next, water (100 ml) was added and acetic acid was used to adjust the pH to 6. The precipitated solid was collected by filtration, washed with water, and dried by heating at 60°C under reduced pressure, to obtain 6.91 g of 5-bromo-3,6-dimethylpyridin-2(1H)-one.

### Production Example 10

A mixture of bromo-4-fluoro-2-(trifluoromethyl)benzene (3.00 g), 2-hydroxyethyl acetate (1.74 ml), sodium hydride (approximately 40% mineral oil included, 642 mg) and DMF (30 ml), was stirred at room temperature for 1 hour. To the reaction mixture, water was added, and it was evaporated under reduced pressure, and then water was added to the residue, followed by extraction with ethyl acetate. The organic layer was washed with a saturated aqueous solution of sodium chloride and dried over anhydrous magnesium sulfate. After removing the desiccant, the solvent was evaporated under reduced pressure. To the obtained residue, methanol (15 ml), THF (15 ml), and an 1 M aqueous solution of sodium hydroxide (15 ml) was added, followed by stirring at room temperature for 25 minutes. To the reaction mixture, 1 M hydrochloric acid (15 ml) was added, followed by extraction with ethyl acetate. The organic layer was washed with a saturated aqueous solution of sodium chloride, and dried over anhydrous magnesium sulfate. After removing the desiccant, the solvent was evaporated under reduced pressure, and the obtained residue was purified by silica gel column chromatography (hexane-ethyl acetate) to obtain 1.59 g of 2-[4-bromo-3-(trifluoromethyl)phenoxy]ethanol.

### Production Example 11

Under a nitrogen atmosphere, to a mixture of sodium carbonate (5.67 g), water (28 ml), 4-bromo-3-methylphenol (5.00 g), (3-formylphenyl)boronic acid (4.40 g), ethanol (20 ml) and toluene (40 ml), tetrakistriphenylphosphinepalladium (1.54 g) was added, stirred at 80°C for 13 hours, and cooled to room temperature. To the reaction mixture, activated carbon (0.5 g) was added, stirred for 5 minutes, filtrated over Celite, and washed with ethyl acetate and water. The obtained filtrate was separated, and the aqueous layer was extracted with ethyl acetate. The organic layer was combined and washed with water and a saturated aqueous solution of sodium chloride, and then anhydrous magnesium sulfate and activated carbon (0.5 g) was added. The desiccant and activated carbon were removed by filtration, the filtrate was evaporated under reduced pressure. The obtained residue was purified by silica gel column chromatography (hexane-ethyl acetate) to obtain 4.42 g of 4'-hydroxy-2'-methylbiphenyl-3-carbaldehyde.

### Production Example 12

Under a nitrogen atmosphere, a mixture of tert-butyl[3,5-dimethyl-4-(4,4,5,5-tetramethyl-1,3,2-dioxaborolane-2-yl)phenoxy]dimethylsilane (9.49 g), methyl 3-bromo-2-methylbenzoate (5 g), palladium acetate(II) (245 mg), dicyclohexyl(2',6'-dimethoxybiphenyl-2-yl)phosphine (896 mg), tripotassium phosphate (9.27 g), toluene (100 ml) and water (10 ml) was stirred at 60°C for 17 hours. The solvent was evaporated under reduced pressure, and a saturated aqueous solution of ammonium chloride was added to the residue, and the mixture was extracted with ethyl acetate. The organic layer was washed with a saturated aqueous solution of sodium chloride, and dried over anhydrous magnesium sulfate. After removing the desiccant, the solvent was evaporated under reduced pressure. The obtained residue was purified by silica gel column chromatography (hexane-ethyl acetate) to obtain 8.40 g of methyl 4'-{[tert-butyl(dimethyl)silyl]oxy}-2,2',6'-trimethylbiphenyl-3-carboxylate.

### Production Example 13

To a solution of methyl 3-(6-amino-2,4-dimethylpyridine-3-yl)-2-methylbenzoate (4.00 g) in tert-butanol (40 ml), di-tert-butyl dicarbonate (4.84 g) was added and stirred at 95°C for 13 hours. After evaporating the solvent under reduced pressure, the obtained residue was purified by silica gel column chromatography (hexane-ethyl acetate) to obtain 3.24 g of methyl 3-{6-[tert-butoxycarbonyl]amino}-2,4-dimethylpyridine-3-yl}-2-methylbenzoate.

### Production Example 14

To a solution of methyl 4'-methoxy-2,2',5'-trimethylbiphenyl-3-carboxylate (2.70 g) in dichloromethane (15 ml), aluminum chloride (3.80 g) was added under ice cooling, then the temperature was raised to room temperature. To the reaction mixture, dodecane-1-thiol (4.6 ml) was added and stirred at room temperature for 2 hours. The reaction mixture was poured into ice, and the mixture was stirred at room temperature for 1 hour, followed by phase separation. The organic layer was dried over anhydrous magnesium sulfate, and after removing the desiccant, the solvent was evaporated under reduced pressure. The obtained residue was purified by silica gel column chromatography (hexane-ethyl acetate) to obtain 2.30 g of methyl 4'-hydroxy-2,2',5'-trimethylbiphenyl-3-carboxylate.

### Production Example 15

Under a nitrogen atmosphere, lithium aluminum hydride (700 mg) was added to THF (40 ml) under ice cooling, and then a solution of methyl 4'-{[tert-butyl(dimethyl)silyl]oxy}-2,2'-dimethylbiphenyl-3-carboxylate (4.67 g) in THF (20 ml) was slowly added dropwise. After stirring the reaction mixture under ice cooling for 2 hours, ethyl acetate (0.4 ml) and a saturated aqueous solution of ammonium chloride (10 ml) were slowly added dropwise, followed by stirring at the same temperature for 0.5 hour. To the reaction mixture, a mixed solution (100 ml) of ethyl acetate-methanol-triethylamine (87:10:3) was added, followed by stirring for 0.5 hour. The mixture was filtrated over Celite to remove insoluble materials. The obtained filtrate was concentrated under reduced pressure and a saturated aqueous solution of sodium bicarbonate (100 ml) was added to the obtained residue, and the mixture was extracted with ethyl acetate. The organic layer was washed with a saturated aqueous solution of sodium chloride and dried over anhydrous magnesium sulfate. After removing the desiccant by filtration, the filtrate was concentrated under reduced pressure. The obtained residue was purified by silica gel column chromatography (hexane-ethyl acetate) to obtain 3.74 g of (4'-{[tert-butyl(dimethyl)silyl]oxy}-2,2'-dimethylphenyl-3-yl)methanol.

### Production Example 16

To a solution of (4'-{[tert-butyl(dimethyl)silyl]oxy}-2,2'-dimethylphenyl-3-yl)methanol (3.58 g) in chloroform (70 ml), manganese dioxide (4.55 g) was added, the reaction mixture was stirred at 50°C for 3 hours. The temperature of the reaction mixture was raised to 60 °C and stirred at the same temperature for 11 hours. Insoluble materials were removed by Celite filtration and washed with chloroform. The filtrate was concentrated under reduced pressure to obtain 3.39 g of 4'-{[tert-butyl(dimethyl)silyl]oxy}-2,2'-dimethylbiphenyl-3-carbaldehyde.

### Production Example 17

To a solution of 3-[6-(2-hydroxyethoxy)-2,4-dimethylpyridine-3-yl]-2-methylbenzaldehyde (1.67 g) in pyridine (7 ml), acetic anhydride (1.0 ml) was added dropwise, followed by stirring at room temperature for 2 hours. To the reaction mixture, ethanol (2 ml) was added and stirred for 10 minutes, and then concentrated at reduced pressure. To the obtained residue, ethyl acetate and water were added, followed by phase separation, and then the aqueous layer was once again extracted by ethyl acetate. The combined organic layers were washed with a 10% aqueous solution of citric acid and a saturated aqueous solution of sodium chloride and dried over anhydrous magnesium sulfate. The desiccant was removed by filtration and the filtrate was concentrated under reduced pressure. The obtained residue was purified by silica gel column chromatography (hexane-ethyl acetate) to obtain 1.48 g of 2-{[5-(3-formyl-2-methylphenyl)-4,6-dimethylpyridine-2-yl]oxy}ethyl acetate.

### Production Example 18

A mixture of 2,2'-{[3'-(hydroxymethyl)-2',6-dimethylbiphenyl-3,4-diyl]bis(oxy)diethanol (0.95 g), manganese dioxide (1.25 g) and THF (20 ml) was stirred at 50°C for 12 hours. To the reaction mixture, manganese dioxide (1.25 g) was added, followed by stirring at 60°C for 12 hours. Again to the reaction mixture, manganese dioxide (2.50 g) was added, followed by stirring at 60°C for 4 days. The insoluble was removed by Celite filtration, and then the solvent was evaporated under reduced pressure. The residue was purified by silica gel column chromatography (chloroform-methanol) to obtain 42 mg of 4',5'-bis(2-hydroxyethoxy)-2,2'-dimethylbiphenyl-3-carbaldehyde. To a mixture of the obtained 4',5'-bis(2-hydroxyethoxy)-2,2'-dimethylbiphenyl-3-carbaldehyde (74 mg) and pyridine (1.5 ml), acetic anhydride (0.085 ml) was added at room temperature, followed by stirring at room temperature for 2 hours. To the reaction mixture, ethanol (0.5 ml) was added, followed by stirring for 10 minutes. Thereto, water was added, followed by extraction with ethyl acetate. The organic layer was sequentially washed with 1 M hydrochloric acid, water and a saturated aqueous solution of sodium chloride, and then dried over anhydrous magnesium sulfate. The desiccant was removed, and then the solvent was evaporated under reduced pressure. The residue was purified by silica gel column chromatography (hexane-ethyl acetate) to obtain 78 mg of (3'-formyl-2',6-dimethylbiphenyl-3,4-diyl)bis(oxyethane-2,1-diyl)diacetate.

### Production Example 19

2,2'6'-trimethyl-4'-[(2-methylprop-2-ene-1-yl)oxy]biphenyl-3-carbaldehyde (1.00 g) was dissolved in THF (60 ml) and water (100 ml), and under ice cooling, 4-methylmorpholine 4-oxide and a 2.5 wt.% solution of osmium tetroxide in tert-butanol (3.1 ml) were added sequentially. After stirring at the same temperature for 30 minutes, the temperature was raised to room temperature and stirring was performed for 12 hours. A 10% aqueous solution of sodium thiosulfate was added and stirred for 1 hour, and then the solvent was evaporated under reduced pressure. Water was added to the obtained residue, followed by extraction with chloroform. The organic layer was washed with a saturated aqueous solution of sodium bicarbonate and a saturated aqueous solution of sodium chloride, and dried over anhydrous magnesium sulfate. After removing the desiccant, the solvent was evaporated under reduced pressure. The obtained residue was purified by silica gel column chromatography (hexane-ethyl acetate) to obtain 1.22 g of 4'-(2,3-dihydroxy-2-methylpropoxy)-2,2'6'-trimethylbiphenyl-3-carbaldehyde.

### Production Example 20

To a mixture of 4'-(3-hydroxy-3-methylbutoxy)-2'-methylbiphenyl-3-carbaldehyde (2.60 g), pyridine (1.5 ml), N,N-dimethylpyridine-4-amine (1.07 g) and chloroform (25 ml), acetic anhydride (1.65 ml) was added dropwise under ice cooling. The temperature of the reaction mixture was raised to room temperature and stirred at the same temperature for 11 hours. A saturated aqueous solution of ammonium chloride (100 ml) was added to the reaction mixture, followed by extraction with chloroform. After washing with a saturated aqueous solution of sodium chloride, the organic layer was dried over anhydrous magnesium sulfate. After removing the desiccant by filtration, the filtrate was concentrated under reduced pressure. The obtained residue was purified by silica gel column chromatography (hexane-ethyl acetate) to obtain 1.57 g of 3-[(3'-formyl-2-methylbiphenyl-4-yl)oxy]-1,1-dimethylpropyl acetate.

### Production Example 21

To a solution of methyl 3-{6-[(tert-butoxycarbonyl)amino]-2,4-dimethylpyridine-3-yl}-2-benzoate (3.20 g) in THF (48 ml), an 1.0M solution of diisobutylaluminum hydride in toluene (22 ml) was added dropwise under ice cooling, followed by stirring at the same temperature for 40 minutes and then at room temperature for 2 hours. Again under ice cooling, an 1.0 M solution of diisobutylaluminum hydride in toluene (11 ml) was added dropwise thereto, followed by stirring at room temperature for 12 hours. Under ice cooling, a saturated aqueous sodium potassium tartrate solution was added to the reaction mixture, followed by stirring for 10 minutes. Thereafter, the solvent was evaporated under reduced pressure. To the obtained residue, water was added, followed by extraction with chloroform. The organic layer was washed with a saturated aqueous solution of sodium bicarbonate and a saturated aqueous solution of sodium chloride, and then dried over anhydrous magnesium sulfate. The desiccant was removed, and the solvent was evaporated under reduced pressure, to obtain 2.87 g of tert-butyl {5-[3-(hydroxymethyl)-2-methylphenyl]-4,6-dimethylpyridin-2-yl}carbamate.

### Production Example 22

Under a nitrogen atmosphere, lithium aluminum hydride (1.00 g) was added under ice cooling to THF (50 ml), and a solution of methyl 3-[6-(2-acetoxyethoxy)-2,5-dimethylpyridine-3-yl]-2-methylbenzoate (4.64 g) in THF (40 ml) was slowly added dropwise. The reaction mixture was stirred at the same temperature for 2 hours and water (3.0 ml) was slowly added dropwise. Afterwards, THF (100 ml) was added and stirred for 15 minutes. The reaction mixture was dried over anhydrous magnesium sulfate, filtrated over Celite, and then washed with THF. The obtained filtrate was concentrated under reduced pressure to obtain 3.84 g of a crude substance of 2-({5-[3-(hydroxymethyl)-2-methylphenyl]-3,6-dimethylpyridine-2-yl}oxy)ethanol.

To a solution of the obtained crude substance of 2-({5-[3-(hydroxymethyl)-2-methylphenyl]-3,6-dimethylpyridine-2-yl}oxy)ethanol (3.84 g) in chloroform (75 ml), manganese dioxide (5.65 g) was added and the temperature of the reaction mixture was raised to 60°C, stirred at the same temperature for 17 hours, and cooled down to room temperature. The insoluble was removed by Celite filtration and washed with chloroform. The filtration was concentrated under reduced pressure, and the obtained residue was purified by silica gel column chromatography (hexane-ethyl acetate) to obtain 3.38 g of 3-[6-(2-hydroxyethoxy)-2,5-dimethylpyridine-3-yl]-2-methylbenzaldehyde.

### Production Example 23

To a solution of methyl 2-(4-{[tert-butyl(dimethyl)silyl]oxy}-2-methylphenyl)isonicotinic acid (4.1 g) in toluene (41 ml) cooled to -76°C, an 1.0 M solution of diisobutylaluminum hydride in toluene (27.5 ml) was added dropwise at - 70°C or lower, and stirred at -75°C for 30 minutes. To the reaction mixture, methanol (10 ml) and a saturated aqueous solution of sodium potassium tartrate (30 ml) was added and the temperature was raised to room temperature, followed by stirring at room temperature for 1 hour. The insoluble was removed by Celite filtration, and the solvent was evaporated under reduced pressure. The residue was purified by silica gel column chromatography (hexane-ethyl acetate) to obtain 3.09 g of 2-(4-{[tert-butyl(dimethyl)silyl]oxy}-2-methylphenyl)isonicotinaldehyde.

### Production Example 24

To a solution of 2-(4-{[tert-butyl(dimethyl)silyl]oxy}-2-methylphenyl)isonicotinaldehyde (3.09 g) in ethanol (31 ml), sodium borohydride (428 mg) was added under ice cooling and stirred at the same temperature for 30 minutes. To the reaction mixture, 1 M hydrochloric acid was added, followed by extraction with ethyl acetate. The organic layer was washed with a saturated aqueous solution of sodium chloride and dried over anhydrous magnesium sulfate. After removing the desiccant and evaporating the solvent under reduced pressure, the obtained residue was purified by silica gel column chromatography (hexane-ethyl acetate) to obtain 2.57 g of [2-(4-{[tert-butyl(dimethyl)silyl]oxy}-2-methylphenyl)pyridine-4-yl]methanol.

### Production Example 25

To a solution of 4'-{[tert-butyl(dimethyl)silyl]oxy}-2,2'-dimethylbiphenyl-3-carbaldehyde (3.39) in THF (35 ml), an 1.0 M solution of tetrabutylammonium fluoride in THF (11.0 ml) was added dropwise and the mixture was stirred at the same temperature for 2 hours. The reaction mixture was diluted with ethyl acetate (100 ml) and added saturated aqueous solution of ammonium chloride (50 ml), and the mixture was extracted with ethyl acetate. The organic layer was washed with a saturated aqueous solution of sodium chloride and dried over anhydrous magnesium sulfate. The desiccant was removed by filtration and the filtrate was concentrated under reduced pressure. The obtained residue was purified by silica gel column chromatography (hexane-ethyl acetate) to obtain 2.23 g of 4'-hydroxy-2,2'-dimethylbiphenyl-3-carbaldehyde.

### Production Example 26

To a mixture of (3-bromo-2-methylphenyl)methanol (13.40 g), triethylamine (11.51 ml) and ethyl acetate (134 ml) under ice cooling, mesyl chloride (5.67 ml) was added dropwise, followed by stirring at 0 °C for 1 hour. The insoluble was removed by filtration, and to the oily material obtained through evaporation of the solvent under reduced pressure, 4-hydroxy benzaldehyde (9.77 g), cesium carbonate (26.10 g) and DMF (134 ml) were added and stirred at 50°C for 1 hour. The solvent was evaporated under reduced pressure, and water was added to the residue, followed by extraction with ethyl acetate, and then washed three times with a 1 M aqueous solution of sodium hydroxide, washed with saturated a saturated aqueous solution of sodium chloride, and dried over anhydrous magnesium sulfate. To the solid obtained by evaporation under reduced pressure, hexane was added, and then the solid was collected by filtration, and dried by heating under reduced pressure to obtain 8.14 g of 4-[(3-bromo-2-methylbenzyl)oxy]benzaldehyde. From the filtrate, the solvent was once again evaporated under reduced pressure, and dried under reduced pressure to obtain 4.37 g of 4-[(3-bromo-2-methylbenzyl)oxy]benzaldehyde.

### Production Example 27

To a solution of (3-bromo-2-methylphenyl)methanol (6.09 g) in DMF (51 ml) under ice cooling, sodium hydride (approximately 40% mineral oil included, 1.21 g) was added and stirred at the same temperature for 25 minutes, and then 6-chloronicotinonitrile (3.50 g) was added and stirred at room temperature for 1 hour. The solvent was evaporated under reduced pressure, and water was added to the residue, followed by extraction with ethyl acetate. The organic layer was washed with a saturated aqueous solution of sodium chloride and dried over anhydrous magnesium sulfate. After the desiccant was removed, the solvent was evaporated under reduced pressure, and the residue was purified by silica gel column chromatography (hexane-ethyl acetate) to obtain 7.66 g of 6-[(3-bromo-2-methylbenzyl)oxy]nicotinonitrile.

### Production Example 28

To a solution of 6-[(3-bromo-2-methylbenzyl)oxy]nicotinonitrile (8.90 g) in dichloromethane (50 ml) cooled to -75 °C, an 1.0 M solution of diisobutylaluminum hydride in toluene (44 ml) was added dropwise at -70 °C or lower and stirred at -73 °C for 1.5 hours. To the reaction mixture, methanol (10 ml) and a saturated aqueous solution of sodium potassium tartarate (40 ml) was added, and the temperature was raised to room temperature, and then the insoluble was removed by Celite filtration, followed by washing of the filtrate with water and evaporation of the solvent under reduced pressure. The obtained residue was purified by silica gel column chromatography (hexane-ethyl acetate) to obtain 9.01 g of 6-[(3-bromo-2-methylbenzyl)oxy]nicotinaldehyde.

### Production Example 29

Under a nitrogen atmosphere, to a solution of (4'-{[tert-butyl(diemthyl)silyl]oxy}-2,2',6'-trimethylbiphenyl-3-yl)methanol (51.50 g) and 4'-hydroxybenzaldehyde (21.17 g) in THF (500 ml), 1,1'-(azodicarbonyl)dipiperidine (47.40 g) was added under ice cooling, and then tributylphosphine (47 ml) was added dropwise. The temperature of the reaction mixture was raised to room temperature, and stirred at the same temperature for 2 hours. The insoluble was removed by filtration, followed by washing with THF, and then the filtrate was concentrated under reduced pressure. The obtained residue was purified by silica gel column chromatography (hexane-ethyl acetate) to obtain 59.68 g of 4-[(4'-{[tert-butyl(dimethyl)silyl]oxy}-2-2',6'-trimethylbiphenyl-3-yl)methoxy]benzaldehyde.

### Production Example 30

A suspension of 4-[(4'-hydroxy-2,2',6'-trimethylbiphenyl-3-yl)methoxy]benzaldehyde (7.00 g), tert-butyl (3-bromopropyl)carbamate (5.80 g), cesium carbonate (7.90 g) in DMF (70 ml) was stirred at 60 °C for 13 hours. The solvent was evaporated under reduced pressure, and a saturated aqueous solution of ammonium chloride was added to the residue, and the mixture was extracted with ethyl acetate. The organic layer was washed with a saturated aqueous solution of sodium chloride, then dried over anhydrous magnesium sulfate. After removing the desiccant, the solvent was evaporated under reduced pressure to obtain 9.20 g of tert-butyl [3-({3'-[(4-formylphenoxy)methyl]-2,2',6-trimethylbiphenyl-4-yl}oxy)propyl]carbamate.

### Production Example 31

To a solution of 4-[(4'-{[(4R)-2,2-dimethyl-1,3-dioxolane-4-yl]methoxy}-2,2',6'-trimethylbiphenyl-3-yl)methoxy]benzaldehyde in THF (12.3 ml), 1 M hydrochloric acid (5 ml) was added and stirred at room temperature for 30 minutes. The solvent was evaporated under reduced pressure, and water was added to the residue, followed by extraction with chloroform. The organic layer was washed with a saturated aqueous solution of sodium chloride and dried over anhydrous magnesium sulfate. After removing the desiccant, the solvent was evaporated under reduced pressure, and the obtained residue was purified by silica gel column chromatography (chloroform-methanol) to obtain 770 mg of 4-[(4'-{[(2S)-2,3-dihydroxypropyl]oxy}-2,2',6'-trimethylbiphenyl-3-yl)methoxy]benzaldehyde.

### Production Example 32

To a solution of 4-[(4'-{[(2S)-2,3-dihydroxypropyl]oxy}-2,2',6'-trimethylbiphenyl-3-yl)methoxy]benzaldehyde (770 mg) and triethylamine (0.38 ml) in dichloromethane (4.0 ml), a solution of tert-butyl(dimethyl)silylchloride (414 mg) in dichloromethane (1.6 ml) was added under ice cooling, stirred at the same temperature for 1 hour, and then stirred at room temperature for 3 hours. After adding water and chloroform followed by phase separation, the organic layer was washed with a saturated aqueous solution of sodium chloride and dried over anhydrous magnesium sulfate. After removing the desiccant, the solvent was evaporated under reduced pressure, and the obtained residue was purified by silica gel column chromatography (hexane-ethyl acetate) to obtain 350 mg of 4-[(4'-{[(2R)-3-{[tert-butyl(dimethyl)silyl]oxy}-2-hydroxypropyl]oxy}-2,2',6'-trimethylbiphenyl-3-yl)methoxy]benzaldehyde.

### Production Example 33

To a solution of 4-[(4'-{[(2R)-3-{[tert-butyl(dimethyl)silyl]oxy}-2-hydroxypropyl]oxy}-2,2',6'-trimethylbiphenyl-3-yl)methoxy]benzaldehyde (350 mg) and methyl iodide (0.4 ml) in acetonitrile (3.5 ml), silver oxide(I) (227 mg) was added and stirred at 60 °C for 13 hours. After filter separation of the solid, the solvent was evaporated under reduced pressure, and the obtained residue was purified by silica gel column chromatography (hexane-ethyl acetate) to obtain 132 mg of 4-[(4'-{[(2R)-3-{[tert-butyl(dimethyl)silyl]oxy}-2-methoxypropyl]oxy}-2,2',6'-trimethylbiphenyl-3-yl)methoxy benzaldehyde.

### Production Example 34

Under a nitrogen atmosphere, to a solution of 2-{[5-(3-formyl-2-methylphenyl)-4,6-dimethylpyridine-2-yl]oxy}ethyl acetate in ethanol (17 ml), sodium borohydride (150 mg) was added under ice cooling and stirred at the same temperature for 0.5 hour. To the reaction mixture, a 10% aqueous solution of citric acid (20 ml) was added slowly, followed by extraction with ethyl acetate. The organic layer was washed with a saturated aqueous solution of sodium chloride and dried over anhydrous magnesium sulfate. The desiccant was removed by filtration and the filtrate was concentrated under reduced pressure to obtain 934 mg of crude 2-({5-[3-(hydroxymethyl)-2-methylphenyl]-4,6-dimethylpyridine-2-yl}oxy)ethyl acetate. To a solution of the obtained crude 2-({5-[3-(hydroxymethyl)-2-methylphenyl]-4,6-dimethylpyridine-2-yl}oxy)ethyl acetate (934 mg) and 4-hydroxybenzaldehyde (383 mg) in THF (7 ml), tributylphosphine (0.85 ml) and 1,1'-(azodicarbonyl)dipiperidine (860 mg) were added, and the reaction mixture was stirred at room temperature for 11 hours. The insoluble was removed by filtration, washed with THF and concentrated under reduced pressure. The obtained residue was purified by silica gel column chromatography (hexane-ethyl acetate) to obtain 599 mg of 2-[(5-{3-[(4-formylphenoxy)methyl]-2-methylphenyl}-4,6-dimethylpyridine-2-yl)oxy]ethyl acetate.

### Production Example 35

A solution of 2-({5-[3-(hydroxymethyl)phenyl]-6-methylpyridine-2-yl}oxy)ethyl acetate (750 mg), 4-hydroxybenzaldehyde (365 mg) and tributylphosphine (0.80 ml) in THF (7.5 ml) was ice cooled, and 1,1'-(azodicarbonyl)dipiperidine (816 mg) was added and stirred at room temperature for 3 hours. The insoluble was removed by filtration, and washed with ethyl acetate, followed by concentration under reduced pressure. The obtained residue was purified by silica gel column chromatography (hexane-ethyl acetate) to obtain an oily material. The obtained oily material was dissolved in ethanol (3 ml) and THF (6 ml), and an 1 M aqueous solution of sodium hydroxide (3 ml) was added and stirred at room temperature for 30 minutes. To the reaction mixture, water was added, and followed by extraction with ethyl acetate. The organic layer was dried over anhydrous magnesium sulfate and the desiccant was removed, and then the solvent was evaporated under reduced pressure to obtain 850 mg of 4-({3-[6-(2-hydroxyethoxy)-2-methylpyridine-3-yl]benzyl}oxy)benzaldehyde.

### Production Example 36

To a mixture of 2-{[5-fluoro-3'-(hydroxymethyl)-2,2'-dimethylbiphenyl-4-yl]oxy}ethyl acetate (1.00 g), 4-hydroxybenzaldehyde (0.45 g), tributylphosphine (0.98 ml) and THF (10 ml), 1,1'-(azodicarbonyl)dipiperidine (1.00 g) was added under ice cooling and stirred at room temperature for 2 days. After filter separation of the insoluble, the solvent was evaporated under reduced pressure. To the mixture of the solid (1.20 g) obtained from the residue which was purified by silica gel column chromatography (hexane-ethyl acetate), THF (10 ml) and methanol (10 ml), an 1 M aqueous solution of sodium hydroxide (10 ml) was added and stirred at room temperature for 1 hour. After evaporating the solvent under reduced pressure, the obtained residue was extracted with chloroform. The organic layer was washed with water and dried over anhydrous magnesium sulfate. After removing the desiccant and evaporating the solvent under reduced pressure, the obtained residue was dried under reduced pressure to obtain a solid (0.99 g). At room temperature, to the mixture of the obtained solid (0.99 g) and pyridine (3 ml) was added acetic anhydride (0.48 ml) and stirred at room temperature for 2 hours. To the reaction mixture, ethanol (5 ml) was added and stirred for 10 minutes, followed by evaporation of the solvent under reduced pressure. To the obtained residue, water was added, followed by extraction with ethyl acetate. The organic layer was washed with a saturated aqueous solution of sodium chloride and dried over anhydrous magnesium sulfate. After removing the desiccant, the solvent was evaporated under reduced pressure. The obtained residue was purified by silica gel column chromatography (hexane-ethyl acetate) to obtain 1.00 g of 2-({5-fluoro-3'-[(4-formylphenoxy)methyl]-2,2'-dimethylbiphenyl-4-yl}oxy)ethyl acetate.

### Production Example 37

Under a nitrogen atmosphere, to a suspension of 5-bromo-6-methylpyridine-2-one (2.00 g) in DMF (20 ml), sodium hydride (approximately 40% mineral oil included, 468 mg) was added under ice cooling and stirred at the same temperature for 1 hour. The temperature of the reaction mixture was raised to room temperature and stirred for 30 minutes. To the reaction mixture, 3-hydroxy-3-methylbutyl 4-methylbenzenesulfonate (3.02 g) was added at room temperature and stirred for 2 days. To the reaction mixture, a saturated aqueous solution of ammonium chloride and water were added, followed by extraction with chloroform. The organic layer was dried over anhydrous magnesium sulfate, and after the desiccant was removed, the solvent was evaporated under reduced pressure. The obtained residue was purified by silica gel column chromatography (hexane-ethyl acetate) to obtain 2.13 g of 4-[(5-bromo-6-methylpyridine-2-yl)oxy]-2-methylbutane-2-ol.

Compounds of Production Examples 38 to 235 were produced in the same manner as in Production Examples 1 to 37 using corresponding raw materials, respectively. The production, structure and physicochemical data of the compounds of Production Examples are shown in Tables 4 to 30.

### Example 1

To a solution of tert-butyl [3-({3'-[(4-formylphenoxy)methyl]-2,2',6-trimethylphenyl-4-}oxy)propyl]carbamate (9.10 g) in ethanol (91 ml), hydroxylamine hydrochloride (1.50 g) and a 2.8 M aqueous solution of sodium acetate (8.4 ml) were added sequentially and stirred at room temperature for 12 hours. After the solvent was evaporated under reduced pressure, water was added, followed by extraction with chloroform. The organic layer was dried over anhydrous magnesium sulfate, and after removing the desiccant, the solvent was evaporated under reduced pressure. The residue was dissolved in acetic acid (91 ml) and sodium cyanoborohydride (2.84 g) was added, followed by stirring at room temperature. Chloroform was added to the reaction mixture, alkalified by adding an 1 M aqueous solution of sodium hydroxide, and then the phase was separated. The aqueous layer was extracted with chloroform-2-propanol (10:1), and the organic layer was combined, washed with a saturated aqueous solution of sodium chloride and dried over anhydrous magnesium sulfate. After removing the desiccant, the solvent was evaporated under reduced pressure, and the obtained residue was purified by silica gel column chromatography (chloroform-methanol) to obtain a foamy product. The obtained foamy product was dissolved in THF (136 ml), and chlorocarbonylisocyanate (1.53 ml) was added thereto dropwise under ice cooling, followed by stirring at the same temperature for 30 minutes and stirring again at a raised temperature of room temperature for 1 hour. The solvent was evaporated under reduced pressure, and water was added to the obtained residue, followed by extraction with chloroform. The organic layer was washed with a saturated aqueous solution of sodium chloride and dried over anhydrous magnesium sulfate. After removing the desiccant, the solvent was evaporated under reduced pressure, and the obtained residue was purified by silica gel column chromatography (hexane-ethyl acetate) to obtain 7.20 g of tert-butyl (3-{[3'-({4-[(3,5-dioxo-1,2,4-oxadiazolidine-2-yl)methyl]phenoxy}methyl)-2,2',6-trimethylbiphenyl-4-yl]oxy}propyl)carbamate.

### Example 2

To a solution of tert-butyl (3-{[3'-({4-[(3,5-dioxo-1,2,4-oxadiazolidine-2-yl)methyl]phenoxy}methyl)-2,2',6-trimethylbiphenyl-4-yl]oxy}propyl)carbamate (7.20 g in acetic acid (3.6 ml), a 4 M solution of hydrogen chloride in dioxane (6.1 ml) was added dropwise under ice cooling and stirred for 1 hour, and then the temperature was raised to room temperature and stirred for 4 hours. The solvent was evaporated under reduced pressure, and the precipitated solid was collected by filtration and dried by heating under reduced pressure to obtain 5.83 g of 2-(4-{[4'-(3-aminopropoxy)-2,2',6'-trimethylbiphenyl-3-yl]methoxy}benzyl)-1,2,4-oxadiazolidine-3,5-dione hydrochloride.

### Example 3

To a suspension of 2-(4-{[4'-(3-aminopropoxy)-2,2',6'-trimethylbiphenyl-3-yl]methoxy}benzyl)-1,2,4-oxadiazolidine-3,5-dione hydrochloride (500 mg), acetic acid (0.082 ml), EDCI hydrochloride (273 mg) and 3H-[1,2,3]triazolo[4,5-b]pyridine-3-ol (194 mg) in DMF (7.5 ml), triethylamine (0.27 ml) was added dropwise under ice cooling, and then stirred at room temperature for 12 hours. N,N-dimethylpropane-1,3-diamine (0.18 ml) was added and stirred for 10 minutes, and then 1 M hydrochloric acid was added, followed by extraction with chloroform-2-propanol (10:1). The organic layer was washed with a saturated aqueous solution of sodium chloride and dried over anhydrous magnesium sulfate. After removing the desiccant, the solvent was evaporated under reduced pressure. The obtained residue was purified by silica gel column chromatography (chloroform-methanol) to obtain 424 mg of a foamy product. The obtained foamy product was dissolved in THF (5 ml) and an 1 M aqueous solution of sodium hydroxide (0.79 ml) was added and stirred for 1 hour, and then the solvent was evaporated under reduced pressure. The obtained residue was purified by ODS column chromatography (acetonitrile-water) to obtain a foamy product. To the obtained foamy product, diethyl ether was added for powderization, and then collected by filtration to obtain 299 mg of sodium 2-(4-{[4'-(3-acetamidepropoxy)-2,2',6'-trimethylbiphenyl-3-yl]methoxy}benzyl)-3,5-dioxo-1,2,4-oxazolidin-4-ide.

### Example 4

To a solution of 2-(4-{[4'-(3-aminopropoxy)-2,2',6'-trimethylbiphenyl-3-yl]methoxy}benzyl)-1,2,4-oxadiazolidine-3,5-dione hydrochloride (800 mg) in pyridine (8 ml), methanesulfonic anhydride (0.8 ml) was added dropwise. The solvent was evaporated under reduced pressure, and then 1 M hydrochloric acid and chloroform-2-propanol (10:1) were added, followed by phase separation. The obtained organic layer was washed with a saturated aqueous solution of sodium chloride and dried over anhydrous magnesium sulfate. After removing the desiccant, the solvent was evaporated under reduced pressure, and the obtained residue was purified by silica gel column chromatography (chloroform-methanol) to obtain 318 mg of a foamy product. The obtained foamy product was dissolved in THF (8 ml), added with an 1 M aqueous solution of sodium hydroxide (0.56 ml) and stirred for 1 hour, and then the solvent was evaporated under reduced pressure, the residue was added with diethyl ether and powderized, collected by filtration to obtain 198 mg of sodium 3,5-dioxo-2-{4-[2,2',6'-trimethyl-4'-{3-[methanesulfonyl)amino]propoxy}biphenyl-3-yl)methoxy]benzyl}-1,2,4-oxadiazolidin-4-ide.

### Example 5

To a solution of 2-[(3-{[3'-({4-[(3,5-dioxo-1,2,4-oxadiazolidine-2-yl)methyl]phenoxy}methyl)-2,2',6-trimethylbiphenyl-4-yl]oxy}propyl)amino]-2-oxoethyl acetate (375 mg) in THF (3.75 ml), an 1 M aqueous solution of sodium hydroxide (1.5 ml) was added, stirred for 2 hours. 1 M hydrochloric acid was added to adjust the pH to approximately 5, and the solvent was evaporated under reduced pressure. To the obtained residue, water was added, followed by extraction with chloroform-isopropanol (10:1). The obtained organic layer was washed with a saturated aqueous solution of sodium chloride and dried over anhydrous magnesium sulfate. After removing the desiccant, the solvent was evaporated under reduced pressure, and the obtained residue was purified by silica gel column chromatography (chloroform-methanol) to obtain 278 mg of a foamy product. The obtained foamy product was dissolved in THF (3.75 ml) and an 1 M aqueous solution of sodium hydroxide (0.61 ml) was added, followed by stirring for 1 hour. The solvent was evaporated under reduced pressure, and the obtained residue was purified by ODS column chromatography (acetonitrile-water) to obtain a foamy product. To the obtained foamy product, diethyl ether was added and powderized, and then collected by filtration to obtain 220 mg of sodium 2-[4-({4'-[3-(glycoloylamino)propoxy]-2,2',6'-trimethylbiphenyl-3-yl}methoxy)benzyl]-3,5-dioxo-1,2,4-oxazolidin-4-ide.

### Example 6

To a solution of 4-[(4'-{[(4R)-2,2-dimethyl-1,3-dioxolane-4-yl]methoxy}-2,2',6'-trimethylbiphenyl-3-yl)methoxy]benzaldehyde (997 mg) in ethanol (10 ml), hydroxylamine hydrochloride (180 mg) and a 2.8 M aqueous solution of sodium acetate (1 ml) were sequentially added and stirred at room temperature for 12 hours. The solvent was evaporated under reduced pressure, and water was added to the obtained residue, followed by extraction with chloroform. The organic layer was dried over anhydrous magnesium sulfate, and after removing the desiccant, the solvent was evaporated under reduced pressure to obtain a foamy product. To the obtained foamy product, acetic acid (5 ml) and sodium cyanoborohydride (408 mg) were added and stirred at room temperature. The reaction mixture was diluted with chloroform, and then an 1 M aqueous solution of sodium hydroxide was added for alkalification, followed by phase separation. The obtained organic layer was dried over anhydrous magnesium sulfate. After removing the desiccant, the solvent was evaporated under reduced pressure. The obtained residue was purified by silica gel column chromatography (chloroform-methanol) to obtain a foamy product. The obtained foamy product was dissolved in THF (15 ml), and under ice cooling, ethoxycarbonylisocyanate (0.23 ml) was added dropwise. After stirring at the same temperature for 1 hour, it was then stirred at room temperature for 1 hour. To the reaction mixture, an 1 M aqueous solution of sodium hydroxide (2.2 ml) was added and stirred at room temperature for 12 hours. To the reaction mixture, a 5% aqueous solution of citric acid was added to adjust the pH to 5, followed by extraction with chloroform. The organic layer was washed with a saturated aqueous solution of sodium chloride, and dried over anhydrous magnesium sulfate. After removing the desiccant, the solvent was evaporated under reduced pressure, and the obtained residue was purified by silica gel column chromatography (hexane-ethyl acetate) to obtain 700 mg of 2-{4-[(4'-{[(4R)-2,2-dimethyl 1-1,3-dioxolane-4-yl]methoxy}-2,2',6'-trimethylbiphenyl-3-yl)methoxy]benzyl}-1,2,4-oxadiazolidine-3,5-dione.

### Example 7

To a solution of 2-{4-[(4'-{[(4R)-2,2-dimethyl1-1,3-dioxolane-4-yl]methoxy}-2,2',6'-trimethylbiphenyl-3-yl)methoxy]benzyl}-1,2,4-oxadiazolidine-3,5-dione (700 mg) in THF (7 ml), 1 M hydrochloric acid (1.3 ml) was added and stirred at room temperature for 30 minutes, and then stirred at 50°C for 14 hours. The solvent was evaporated under reduced pressure, and water was added to the obtained residue, followed by extraction with chloroform. The organic layer was washed with a saturated aqueous solution of sodium chloride, and dried over anhydrous magnesium sulfate. After removing the desiccant, the solvent was evaporated under reduced pressure, and the obtained residue was purified by silica gel column chromatography (chloroform-methanol) to obtain 325 mg of a foamy product. The obtained foamy product was dissolved in THF (7 ml) and an 1 M aqueous solution of sodium hydroxide (0.64 ml) was added, followed by stirring for 1 hour. The solvent was evaporated under reduced pressure, and the obtained residue was purified by ODS column chromatography (acetonitrile-water) to obtain a foamy product. To the obtained foamy product, diethyl ether was added and powderized, and collected by filtration to obtain 258 mg of sodium 2-{4-[(4'-{[(2S)-2,3-dihydroxypropyl]oxy}-2,2',6'-trimethylbiphenyl-3-yl)methoxy]benzyl}-3,5-dioxo-1,2,4-oxadiazolidin-4-ide.

### Example 8

To a solution of ethyl {[3'-({4-[(3,5-dioxo-1,2,4-oxadiazolidine-2-yl)methyl]phenoxy}methyl)-2,2',6-trimethylbiphenyl-4-yl]oxy}acetate in ethanol (18 ml), an 1 M aqueous solution of sodium hydroxide (7 ml) was added and stirred at room temperature for 1.5 hours. The solvent was evaporated under reduced pressure, and 1 M hydrochloric acid was added for acidification, followed by extraction with chloroform. The organic layer was washed with a saturated aqueous solution of sodium chloride, and dried over anhydrous magnesium sulfate. After removing the desiccant, the solvent was evaporated under reduced pressure to obtain 1.69 g of {[3'-({4-[(3,5-dioxo-1,2,4-oxadiazolidine-2-yl)methyl]phenoxy}methyl)-2,2',6-trimethylbiphenyl-4-yl]oxy} acetic acid.

### Example 9

(1S)-2-({3'-[(4-formylphenoxy)methyl]-2,2'-dimethylbiphenyl-4-yl}oxy)-1-methylethyl acetate was dissolved in ethanol (5 ml) and THF (5 ml), and then added with an aqueous solution (1.5 ml) of hydroxylamine hydrochloride (104 mg) and sodium acetate (142 mg), followed by stirring at room temperature for 22 hours. Water was added to the reaction mixture, followed by extraction with ethyl acetate. The organic layer was washed with a saturated aqueous solution of sodium chloride, and dried over anhydrous magnesium sulfate. After removing the desiccant, the solvent was evaporated under reduced pressure, and a syrup-like substance was obtained. The obtained syrup-like substance was dissolved in methanol (4 ml) and THF (4 ml), and sodium cyanoborohydride (218 mg) was added and ice-cooled, afterwards a 4 M solution of hydrogen chloride in dioxane (1.7 ml) was added dropwise. The temperature of the reaction mixture was raised to room temperature, and stirred for 5.5 hours. The reaction mixture was ice-cooled, and added with an 1 M aqueous solution of sodium hydroxide (10 ml), followed by extraction with chloroform. The organic layer was dried over anhydrous magnesium sulfate, and after removing the desiccant, the solvent was evaporated under reduced pressure to obtain the syrup-like substance. A solution of the obtained syrup-like substance in THF (5 ml) was ice-cooled and ethoxycarbonylisocyanate (0.13 ml) was added, followed by stirring for 30 minutes. The temperature of the reaction mixture was raised to room temperature and stirred for 1 hour. To the reaction mixture, an 1 M solution of sodium hydroxide (2.5 ml) was added and stirred at room temperature for 12 hours. To the reaction mixture, 1 M hydrochloric acid was added, followed by extraction with chloroform. The organic layer was dried over anhydrous magnesium sulfate, and after removing the desiccant, the solvent was evaporated under reduced pressure. The obtained residue was purified by silica gel column chromatography (hexane-ethyl acetate) to obtain a syrup-like substance (250 mg). The obtained syrup-like substance (250 mg) was dissolved in THF (5 ml), and an 1 M aqueous solution of sodium hydroxide (0.53 ml) was added, followed by stirring at room temperature for 15 minutes. The solvent was evaporated under reduced pressure, and diethyl ether was added to the obtained residue, and then the resulting solid was collected by filtration and dried by heating under reduced pressure to obtain 225 mg of sodium 2-{4-[(4'-{[(2S)-2-hydroxypropyl]oxy}-2,-2'-dimethylbiphenyl-3-yl)methoxy]benzyl}-3,5-dioxo-1,2,4-oxadiazolidin-4-ide.

### Example 10

A mixture of 3-[(3'-formyl-2-methylbiphenyl-4-yl)oxy]-1,1-dimethylpropyl acetate (500 mg), 2-(4-aminobenzyl)-1,2,4-oxadiazoladine-3,5-dione (320 mg) and acetic acid (6 ml) was stirred at room temperature for 12.5 hours. To the reaction mixture, sodium triacetoxyborohydride (623 mg) was added and stirred at room temperature for 2 hours. To the reaction mixture, water was added, followed by extraction with chloroform. The organic layer was dried over anhydrous magnesium sulfate, and after removing the desiccant, the solvent was evaporated under reduced pressure. The obtained residue was purified by silica gel column chromatography (hexane-ethyl acetate) to obtain 700mg of a foamy product. The obtained foamy product (700 mg) was dissolved in ethanol (3.5 ml) and THF (3.5 ml), and an 1 M aqueous solution of sodium hydroxide (2.7 ml) was added, and then the reaction mixture was stirred at 50 °C for 5 hours. To the reaction mixture, an 1 M aqueous solution of sodium hydroxide (1.3 ml) was added and stirred at 50 °C for 1.5 hours. The reaction mixture was cooled to room temperature, and 1 M hydrochloric acid was added for mild acidification, followed by extraction with chloroform. The organic layer was dried over anhydrous magnesium sulfate, and after removing the desiccant, the solvent was evaporated under reduced pressure. The obtained residue was purified by silica gel column chromatography (hexane-ethyl acetate) to obtain 170 mg of gummy substance. The obtained gummy substance (170 mg) was dissolved in THF (5 ml) and an 1 M aqueous solution of sodium hydroxide (0.35 ml) was added, followed by stirring at room temperature for 30 minutes. The solvent was evaporated under reduced pressure, and diethyl ether was added to the obtained residue to collect the solid by filtration, and then dried by heating under reduced pressure to obtain 140 mg of sodium 2-[4-({[4'-(3-hydroxy-3-methylbutoxy)-2'-methylbiphenyl-3-yl]methyl} amino)benzyl]-3,5-dioxo-1,2,4-oxadiazolidin-4-ide.

### Example 11

A mixture of 2-[(3'-formyl-2,2'-dimethylbiphenyl-4-yl)oxy]ethyl acetate (600 mg), 2-(4-aminobenzyl)-1,2,4-oxadiazolidine-3,5-dione (478 mg) and acetic acid (7.5 ml) was stirred at room temperature for 20.5 hours. To the reaction mixture, sodium triacetoxyborohydride (814 mg) was added and stirred at room temperature for 40.5 hours. To the reaction mixture, water was added, followed by extraction with chloroform. The organic layer was dried over anhydrous magnesium sulfate, and after removing the desiccant, the solvent was evaporated under reduced pressure. The obtained residue was purified by silica gel column chromatography (hexane-ethyl acetate) to obtain a foamy product. The obtained foamy product was dissolved in methanol (6 ml), and sodium methoxide (415 mg) was added, followed by stirring at 50 °C for 7 hours. The reaction mixture was cooled down to room temperature, and a 10% aqueous solution of citric acid was added, followed by extraction with chloroform-2-propanol. The organic layer was dried over anhydrous magnesium sulfate, and after removing the desiccant, the solvent was evaporated under reduced pressure. The obtained residue was purified by silica gel column chromatography (hexane-ethyl acetate) to obtain 570 mg of a foamy product. The obtained foamy product (570 mg) was dissolved in THF (10 ml), and an 1 M aqueous solution of sodium hydroxide (1.2 ml) was added, followed by stirring at room temperature for 10 minutes. The solvent was evaporated under reduced pressure, and diethyl ether was added to the obtained residue to collect the solid by filtration, and then dried by heating under reduced pressure to obtain 550 mg of sodium 2-[4-({[4'-(2-hydroxyethoxy)-2,2'-dimethylbiphenyl-3-yl]methyl}amino)benzyl]-3,5-dioxo-1,2,4-oxadiazolidin-4-ide.

### Example 12

A mixture of 4'-{[(4R)-2,2-dimethyl-1,3-dioxolane-4-yl]methoxy}-2,2'-dimethylbiphenyl-3-carbaldehyde (491 mg), 2-(4-aminobenzyl)-1,2,4-oxadiazolidine-3,5-dione (359 mg) and acetic acid (6.5 ml) was stirred at room temperature for 20.5 hours. To the reaction mixture, sodium triacetoxyborohydride (611 mg) was added and the mixture was stirred at room temperature for 40.5 hours. Water was added to the reaction mixture, and the mixture was extracted with chloroform. The organic layer was dried over anhydrous magnesium sulfate, and after removing the desiccant, the solvent was evaporated under reduced pressure. The obtained residue was purified by silica gel column chromatography (hexane-ethyl acetate) to obtain a foamy product. The obtained foamy product was dissolved in THF (10 ml), and 1M hydrochloric acid (10 ml) was added, and then the reaction mixture was stirred at 50 °C for 7 hours. The reaction mixture was cooled to room temperature, and an 1 M aqueous solution of sodium hydroxide and saturated aqueous solution of sodium bicarbonate were added to make the mixture weakly acidic, the mixture was extracted with chloroform. The organic layer was dried over anhydrous magnesium sulfate, and after removing the desiccant, the solvent was evaporated under reduced pressure. The obtained residue was purified by silica gel column chromatography (hexane-ethyl acetate) to obtain 370 mg of a foamy product. The obtained foamy product (370 mg) was dissolved in THF (10 ml), and an 1 M aqueous solution of sodium hydroxide (0.75 ml) was added, followed by stirring at room temperature for 10 minutes. The solvent was evaporated under reduced pressure, and diethyl ether was added to the obtained residue, the mixture was filtrated to collect the solid, and then the solid was dried with heating under reduced pressure to obtain 250 mg of sodium 2-(4-{[(4'-{ [(2S)-2,3-dihydroxypropyl]oxy}-2,2'-dimethylbiphenyl-3-yl)methyl]amino}benzyl)-3,5-dioxo-1,2,4-oxadiazolidin-4-ide.

### Example 13

To a solution of 4-({3-[1-(3-hydroxy-3-methylbutyl)-3,5-dimethyl-1H-pyrazole-4-yl]-2-methylbenzyl}oxy)benzaldehyde (1.15 g) in ethanol (20 ml), hydroxylamine hydrochloride (600 mg) and a 2.8 M aqueous solution of sodium acetate (4 ml) were added and stirred at room temperature for 15 hours. The reaction mixture was concentrated under reduced pressure, and water (50 ml) was added to the residue, followed by extraction with chloroform. The organic layer was dried over anhydrous magnesium sulfate, and after removing the desiccant, the solvent was evaporated under reduced pressure to obtain 1.16 g of syrup-like substance. To a solution of the obtained syrup-like substance (1.16 g) in ethanol (10 ml) and THF (10 ml), sodium cyanoborohydride (712 mg) was added, and then a 4 M solution of hydrogen chloride in dioxane (5.7 ml) was added dropwise under ice cooling. The temperature of the reaction mixture was raised to room temperature, and stirred for 2 hours. To the reaction mixture, sodium cyanoborohydride (300 mg) was added, and stirred at room temperature for 0.5 hour. Under ice cooling, to the reaction mixture, a 5 M aqueous solution of sodium hydroxide (8 ml) and water (30 ml) were added, followed by extraction with chloroform. The organic layer was dried over anhydrous magnesium sulfate, and after removing the desiccant, the solvent was evaporated under reduced pressure to obtain 1.56 g of syrup-like substance. To a solution of the obtained syrup-like substance (1.56 g) in THF (14 ml), under ice cooling, ethoxycarbonylisocyanate (0.30 ml) was added dropwise and stirred for 15 minutes under ice cooling. Under ice cooling, to the reaction mixture, an 1 M aqueous solution of sodium hydroxide (4.0 ml) and ethanol (4 ml) were added, the temperature was raised to room temperature, and left at the same temperature for 18 hours. To the reaction mixture, 1 M hydrochloric acid (50 ml) was added, followed by extraction with chloroform. The organic layer was dried over anhydrous magnesium sulfate, and after removing the desiccant, the solvent was evaporated under reduced pressure. The obtained residue was purified by silica gel column chromatography (chloroform-methanol) to obtain a syrup-like substance (1.43 g), which was dissolved in methanol (4 ml), added with an 1 M aqueous solution of sodium hydroxide (2.83 ml) and purified by ODS column chromatography (acetonitrile-water) to obtain 433 mg of a foamy product. To a solution of the obtained foamy product (400 mg) in methanol (30 ml), sodium methoxide (890 mg) was added and the temperature of the reaction mixture was raised to 60°C and then stirred for 16 hours. To the reaction mixture, under ice cooling, 1 M hydrochloric acid (30 ml) and water (100 ml) were added, followed by extraction with chloroform. The organic layer was dried over anhydrous magnesium sulfate, and after removing the desiccant, the solvent was evaporated under reduced pressure. The obtained residue was dissolved in methanol (2 ml), and an 1 M aqueous solution of sodium hydroxide (0.65 ml) was added. The solvent was evaporated under reduced pressure, and the obtained residue was purified by ODS column chromatography (acetonitrile-water) to obtain a foamy product.
Diethyl ether (20 ml) was added to the obtained residue to solidify for powderization.
The solid was collected by filtration, washed with diethyl ether and then dried under reduced pressure at 60°C to obtain 224 mg of sodium 2-[4-({3-[1-(3-hydroxy-3-methylbutyl)-3,5-dimethyl-1H-pyrazole-4-yl]-2-methylbenzyl}oxy)benzyl]-3,5-dioxo-1,2,4-oxadiazolidin-4-ide.

### Example 14

A solution of 3-[6-(3-hydroxy-3-methylbutoxy)-2,4-dimethylpyridine-3-yl-benzaldehyde (300 mg) and 2-(4-aminobenzyl)-1,2,4-oxadiazolidine-3,5-dione (238 mg) in acetic acid (5 ml) was stirred at room temperature for 12 hours. To the reaction mixture, sodium triacetoxyborohydride (406 mg) was added and stirred at room temperature for 4 hours. To the reaction mixture, water was added, followed by extraction with chloroform. The organic layer was washed with a saturated aqueous solution of sodium chloride and dried over anhydrous magnesium sulfate. After removing the desiccant, the solvent was evaporated under reduced pressure. The obtained residue was purified by silica gel column chromatography (chloroform-methanol) to obtain an oily material (400 mg). To a solution of the oily material in methanol (5.8 ml), at room temperature, an 1 M aqueous solution of sodium hydroxide (0.75 ml) was added and the solvent was evaporated under reduced pressure. Diethyl ether was added to the residue for powderization, and the powder was collected by filtration and dried by heating under reduced pressure to obtain 373 mg of sodium 2-[4-({3-[6-(3-hydroxy-3-methylbutoxy)-2,4-dimethylpyridine-3-yl]benzyl}amino)benzyl]-3,5-dioxo-1,2,4-oxadiazolidin-4-ide.

### Example 15

To a solution of (1S)-3-[(5-{3-[(4-formylphenoxy)methyl]-2-methylphenyl}-6-methylpyridine-2-yl)oxy]-1-methylpropyl acetate in ethanol (4 ml) and THF (4 ml), an aqueous solution (1 ml) of hydroxylamine hydrochloride (71 mg) and sodium acetate (97 mg) was added and stirred at room temperature for 16.5 hours. To the reaction mixture, water was added, followed by extraction with ethyl acetate. The organic layer was washed with a saturated aqueous solution of sodium chloride, dried over anhydrous magnesium sulfate, and after removing the desiccant, the solvent was evaporated under reduced pressure, and a syrup-like substance was obtained. The obtained syrup-like substance was dissolved in methanol (3 ml) and THF (3 ml), and sodium cyanoborohydride (149 mg) was added and ice-cooled, and then a 4 M solution of hydrogen chloride in dioxane (1.2 ml) was added dropwise. The temperature of the reaction mixture was raised to room temperature and stirred for 3 hours. The reaction mixture was ice-cooled, and an 1 M aqueous solution of sodium hydroxide was added for mild acidification, and then a saturated aqueous solution of sodium bicarbonate was added for mild alkalification, followed by extraction with chloroform. The organic layer was washed with a saturated aqueous solution of sodium chloride, dried over anhydrous magnesium sulfate, and after removing the desiccant, the solvent was evaporated under reduced pressure, and a syrup-like substance was obtained. A solution of the obtained syrup-like substance in THF (10 ml) was cooled down in an ice-methanol bath, and ethoxycarbonylisocyanate (0.081 ml) was added, followed by stirring for 30 minutes. To the reaction mixture, an 1 M aqueous solution of sodium hydroxide (0.30 ml) was added and the temperature was raised to room temperature, followed by stirring for 12 hours. To the reaction mixture, 1 M hydrochloric acid was added for mild acidification, followed by extraction with chloroform. The organic layer was dried over anhydrous magnesium sulfate, and after removing the desiccant, the solvent was evaporated under reduced pressure. The obtained residue was purified by silica gel column chromatography (hexane-ethyl acetate) to obtain a syrup-like substance. To a solution of the obtained syrup-like substance in methanol (3 ml), sodium methoxide (40 mg) was added, and the reaction mixture was stirred at 60°C for 2 hours. To the reaction mixture, sodium methoxide (60 mg) was added, and the reaction mixture was stirred at 60°C for 2 hours. The reaction mixture was cooled to room temperature and an 10% aqueous solution of citric acid (10 ml) was added, followed by extraction with chloroform. The organic layer was dried over anhydrous magnesium sulfate, and after removing the desiccant, the solvent was evaporated under reduced pressure. The obtained residue was dissolved in methanol (4 ml), an 1 M aqueous solution of sodium hydroxide (0.30 ml) was added and stirred at room temperature for 30 minutes. The obtained mixture was purified by ODS column chromatography (acetonitrile-water) to obtain a foamy product. To the obtained foamy product was added diethyl ether to collect the resulting solid by filtration, and dried by heating under reduced pressure to obtain 50 mg of sodium 2-(4-{[3-(6-{[(3S)-3-hydroxybutyl]oxy}-2-methylpyridine-3-yl)-2-methylbenzyl]oxy}benzyl)-3,5-dioxo-1,2,4-oxadiazolidin-4-ide.

### Example 16

To a mixture of 4-[(2'-chloro-4'-{[(4R)-2,2-dimethyl-1,3-dioxolane-4-yl]methoxy}-2-methylbiphenyl-3-yl)methoxy]benzaldehyde (1.22 g), ethanol (5 ml), methanol (5 ml) and THF (5 ml), an aqueous solution (3 ml) of hydroxylamine hydrochloride (218 mg) and sodium acetate (279 mg) was added dropwise, and stirred at room temperature for 3 hours. The solvent was evaporated under reduced pressure, and water was added to the residue, followed by extraction with chloroform and drying the organic layer over anhydrous magnesium sulfate. After removing the desiccant, to the oily material obtained by evaporating the solvent under reduced pressure, sodium cyanoborohydride (775 mg) and acetic acid (10 ml) was added, followed by stirring at room temperature for 5 hours. The reaction mixture was diluted with chloroform, alkalified by adding an 1 M aqueous solution of sodium hydroxide, followed by extraction with chloroform. The organic layer was washed with water and dried over anhydrous magnesium sulfate. After removing the desiccant, the solvent was evaporated under reduced pressure, and the obtained residue was purified by silica gel column chromatography (chloroform-methanol) to obtain 650 mg of a foamy product, to which THF (10 ml) was added and ice-cooled, ethoxycarbonylisocyanate (0.2 ml) was added dropwise, and stirred briefly under ice cooling and stirred at room temperature for 30 minutes. To the reaction mixture, an 1 M aqueous solution of sodium hydroxide (2.6 ml) was added and stirred at room temperature for 25 hours. The solvent was evaporated under reduced pressure, and an 1 M aqueous solution of sodium hydroxide was added to the residue and washed with diethyl ether. 1 M hydrochloric acid was added to the aqueous layer for mild acidification (pH5), followed by extraction with chloroform. The organic layer was dried over anhydrous magnesium sulfate, and after removing the desiccant, the solvent was evaporated under reduced pressure, and the obtained residue was purified by silica gel column chromatography (hexane-ethyl acetate) to obtain 588 mg of a foamy product, to which 1 M hydrochloric acid (2.6 ml), THF (5 ml) and methanol (1 ml) were added and stirred at 50°C for 3 hours. Water was added to the reaction mixture, followed by extraction with chloroform. The organic layer was dried over anhydrous magnesium sulfate, and after removing the desiccant, the solvent was evaporated under reduced pressure. The obtained residue was purified by silica gel column chromatography (hexane-ethyl acetate) to obtain 218 mg of oily material, to which an 1 M aqueous solution of sodium hydroxide (0.425 ml), methanol (3 ml) and THF (3 ml) were added and stirred at room temperature for 5 minutes. The solvent was evaporated under reduced pressure, and the obtained residue was purified by ODS column chromatography (acetonitrile-water) to obtain 148 mg of sodium 2-{4-[(2'-chloro-4'-{[(2S)-2,3-dihydroxypropyl]oxy}-2-methylbiphenyl-3-yl)methoxy]benzyl}-3,5-dioxo-1,2,4-oxadiazolidine-4-ide.

### Example 17

A tert-butyl {5-[3-({4-[(3,5-dioxo-1,2,4-oxazolidine-2-yl)methyl]phenoxy}methyl)-2-methylphenyl]-4,6-dimethylpyridien-2-yl}carbamate hydrochloride (2.19 g) was dissolved in methanol (10 ml), and a 4 M solution of hydrogen chloride in dioxane (4.8 ml) was added, and stirred at room temperature for 13 hours. The solvent was evaporated under reduced pressure, and a saturated aqueous solution of sodium bicarbonate was added to adjust the pH to approximately 8. The precipitated solid was collected by filtration, dried under reduced pressure, and washed with diethyl ether to obtain 1.39 g of 2-(4-{[3-(6-amino-2,4-dimethylpyridine-3-yl)-2-methylbenzyl]oxy}benzyl)-1,2,4-oxadiazolidine-3,5-dione.

### Example 18

To a solution of 2-(4-{[3-(6-amino-2,4-dimethylpyridine-3-yl)-2-methylbenzyl]oxy}benzyl)-1,2,4-oxadiazolidine-3,5-dione (1.00 g) in acetic acid (30 ml), {[tert-butyl(dimethyl)silyl]oxy}acetaldehyde (0.53 ml)was added, and stirred at room temperature for 11 hours. To the reaction mixture, sodium triacetoxyborohydride (0.98 g) was added and stirred for 6 hours, and then water was added, followed by extraction with chloroform. The organic layer was dried over anhydrous magnesium sulfate, and after removing the desiccant, the solvent was evaporated under reduced pressure. The obtained residue was purified by silica gel column chromatography (chloroform-methanol) to obtain an oily material. The obtained oily material was dissolved in THF (10 ml), added with 5 M hydrochloric acid (2 ml) and stirred at room temperature for 2 hours. Next, a saturated aqueous solution of sodium bicarbonate was added to adjust the pH to approximately 7, followed by extraction with chloroform-2-propanol (10:1), and the obtained organic layer was washed with a saturated aqueous solution of sodium chloride and dried over anhydrous magnesium sulfate. After removing the desiccant, the solvent was evaporated under reduced pressure. The obtained residue was purified by silica gel column chromatography (chloroform-methanol) to obtain a foamy product. The obtained foamy product was dissolved in methanol (10 ml), cooled down in an ice-methanol bath, added with an 1 M aqueous solution of sodium hydroxide and stirred for 10 minutes, and then the solvent was evaporated under reduced pressure, and purified with ODS column chromatography (acetonitrile-water) to obtain 366 mg of a foamy product. To a solution of the obtained foamy product in toluene (5 ml), hexane-2,5-dione (0.27 ml) and acetic acid (0.027 ml) were added and heated to reflux for 14 hours using the Dean-Stark apparatus. To the reaction mixture, a saturated aqueous solution of sodium bicarbonate was added to adjust the pH to approximately 7, followed by extraction with chloroform. The organic layer was washed with a saturated aqueous solution of sodium chloride, dried over anhydrous magnesium sulfate, and after removing the desiccant, the solvent was evaporated under reduced pressure. The obtained residue was purified by silica gel column chromatography (chloroform-methanol) to obtain a foamy product (17 mg). To the obtained foamy product, THF (0.2 ml) and 1 M hydrochloric acid (0.036 ml) were added and stirred for 10 minutes, and the solvent was evaporated under reduced pressure to obtain 18 mg of 2-{4-[(3-{6-[(2-hydroxyethyl)amino]-2,4-dimethylpyridine-3-yl}-2-methylbenzyl)oxy]benzyl}-1,2,4-oxadiazolidine-3,5-dione hydrochloride as a foamy product.

### Example 19

To a mixture of 2-(4-{[(4'-{[(2R)-2,3-dihydroxypropyl]oxy}-2,2',6'-trimethylbiphenyl-3-yl)methyl]amino}benzyl)-1,2,4-oxadiazolidine-3,5-dione (1.72 g), acetonitrile (8.5 ml) and water (0.6 ml), a mixture of concentrated sulfuric acid (0.18 ml) and water (0.6 ml) was slowly added, and then the mixture was dissolved by heating at an outside temperature of 50°C. To this solution, acetonitrile (7.5 ml) was slowly added dropwise, and the solution was slowly cooled till it reached room temperature and precipitation of solid could be confirmed, and stirred under ice cooling for another 1 hour. The precipitated solid was collected by filtration, washed with acetonitrile (2 ml), and then dried at 40°C for 3 hours under reduced pressure to obtain 1.56 g of 2-(4-{[(4'-{[(2R)-2,3-dihydroxypropyl]oxy}-2,2',6'-trimethylbiphenyl-3-yl)methyl]amino}benzyl)-1,2,4-oxadiazolidine-3,5-dione sulfate as a slightly yellow crystal.

Compounds of Examples 20 to 119 were produced in the same manner as in Examples 1 to 19 using corresponding raw materials, respectively. Structures of the compounds of Examples are shown in Tables 31 to 45. Furthermore, Production methods and instrumental analysis data for those compounds of Examples are shown in Tables 46 to 57.

**[Table 4]**

| PEx | Syn | Structure | Data |
|---|---|---|---|
| 1 | P1 | 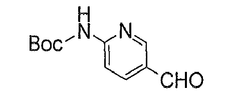 | FAB+: 223 |
| 2 | P2 | 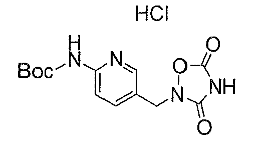 | ESI-: 307 |
| 3 | P3 | 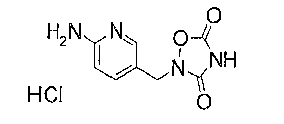 | ESI+: 209 |
| 4 | P4 | 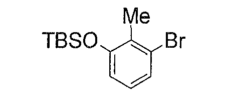 | EI: 300 |
| 5 | P5 | 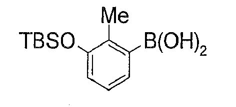 | FAB-: 265 |
| 6 | P6 | 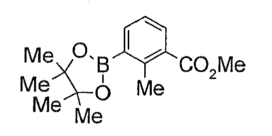 | ESI+: 277 |
| 7 | P7 | 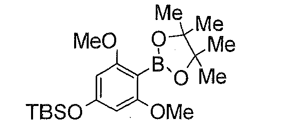 | ESI+: 395 |
| 8 | P8 | 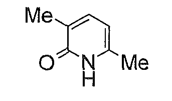 | ESI+: 124 |
| 9 | P9 | 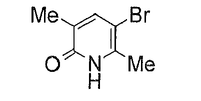 | ESI+: 202, 204 |
| 10 | P10 | 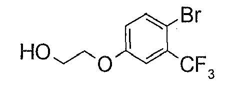 | ESI+: 285 |
| 11 | P11 | 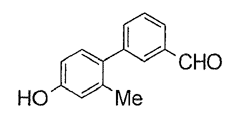 | ESI-: 211 |

**[Table 5]**

| PEx | Syn | Structure | Data |
|---|---|---|---|
| 12 | P12 | 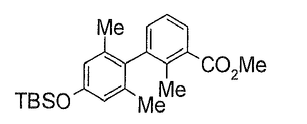 | ESI+: 385 |
| 13 | P13 | 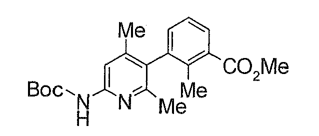 | FAB+: 371 |
| 14 | P14 | 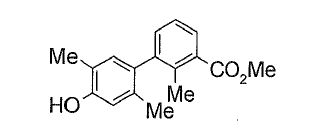 | EI: 270 |
| 15 | P15 | 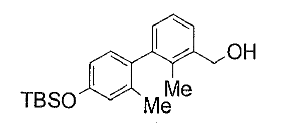 | EI: 342 |
| 16 | P16 | 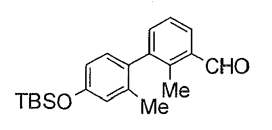 | ESI+: 341 |
| 17 | P17 | 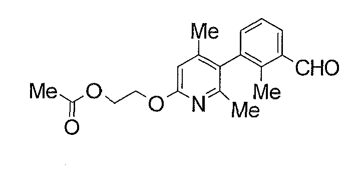 | NMR2: 1.89(3H,s),2.06(3H, s),2.12(3H,s),2.33(3H,s), 4.39-4.49(2H,m),4.51-4.62(2H,m),6.57(1H,s),7. 24-7.31(1H,m),7.44(1H,t,J= 7.6Hz),7.82-7.88(1H,m),10.37(1H,s) |
| 18 | P18 | 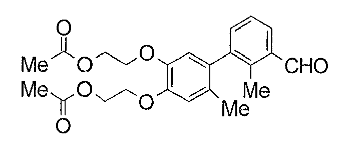 | NMR2: 1.96(3H,s),2.08(3H, s),2.12(3H,s),2.38(3H,s), 4.11-4.34(4H,m),4.35-4.59(4H,m),6.67(1H,s),6. 85(1H,s),7.29-7.49(2H,m),7.75-7.90(1H,m),10.36(1H,s) |

**[Table 6]**

| PEx | Syn | Structure | Data |
|---|---|---|---|
| 19 | P19 | 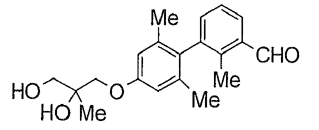 | EI: 328 |
| 20 | P20 | 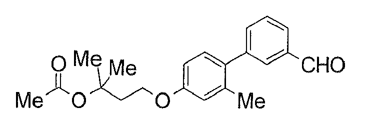 | ESI-MS [M-OAc]⁺: 281 |
| 21 | P21 | 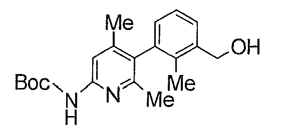 | ESI+: 343 |
| 22 | P22 | 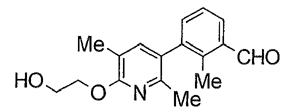 | EI: 285 |
| 23 | P23 | 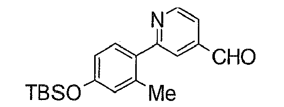 | ESI+: 328 |
| 24 | P24 | 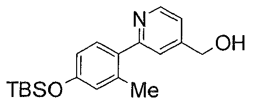 | ESI+: 330 |
| 25 | P25 | 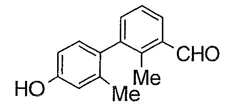 | ESI-: 225 |
| 26 | P26 | 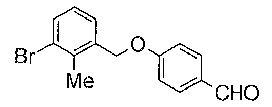 | ESI-: 303, 305 |
| 27 | P27 | 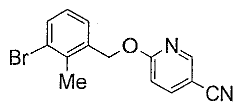 | EI: 302, 304 |
| 28 | P28 | 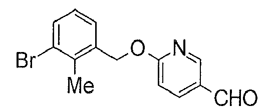 | ESI-: 304 |

**[Table 7]**

| PEx | Syn | Structure | Data |
|---|---|---|---|
| 29 | P29 | 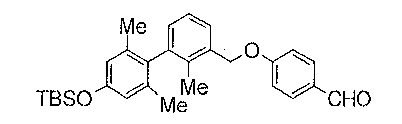 | ESI+: 461 |
| 30 | P30 | 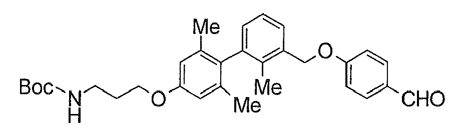 | ESI-: 502 |
| 31 | P31 | 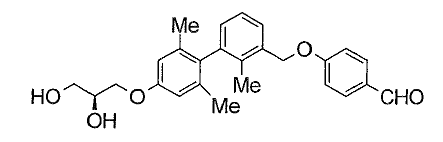 | ESI-: 419 |
| 32 | P32 | 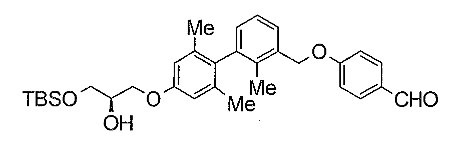 | ESI+: 535 |
| 33 | P33 | 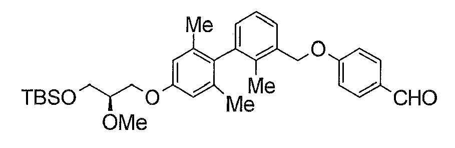 | ESI+: 549 |
| 34 | P34 | 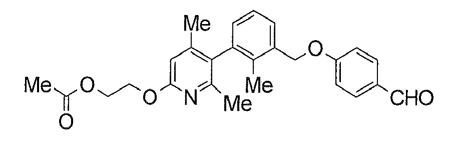 | ESI+: 434 |
| 35 | P35 | 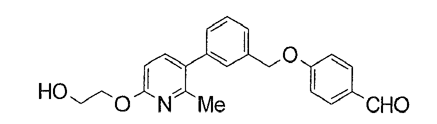 | ESI+: 364 |
| 36 | P36 | 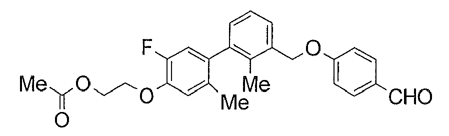 | ESI+: 437 |
| 37 | P37 | 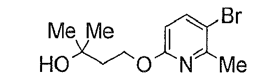 | EI: 273, 275 |
| 38 | P37 | 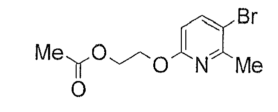 | EI: 273, 275 |

**[Table 8]**

| PEx | Syn | Structure | Data |
|---|---|---|---|
| 39 | P37 | 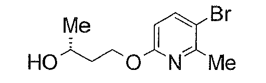 | EI: 259, 261 |
| 40 | P37 | 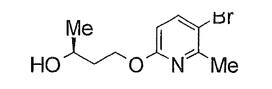 | EI: 259, 261 |
| 41 | P37 | 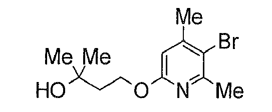 | EI: 287, 289 |
| 42 | P37 | 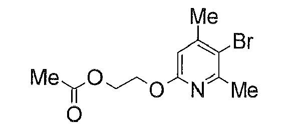 | EI: 287, 289 |
| 43 | P37 | 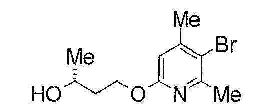 | ESI+: 273, 275 |
| 44 | P37 | 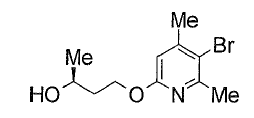 | ESI+: 273, 275 |
| 45 | P37 | 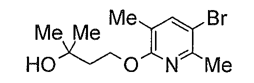 | ESI+: 288, 290 |
| 46 | P37 | 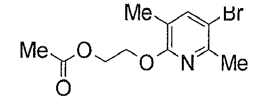 | EI: 287, 289 |
| 47 | P37 | 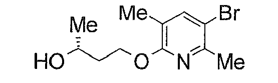 | EI: 273, 275 |
| 48 | P37 | 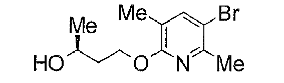 | EI: 273, 275 |
| 49 | P30 | 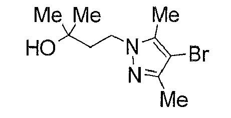 | ESI-MS [M-OH]⁺: 243, 245 |
| 50 | P30 | 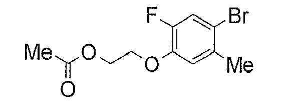 | CI: 291, 293 |
| 51 | P30 | 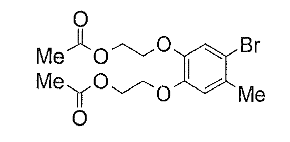 | EI: 374, 376 |

**[Table 9]**

| PEx | Syn | Structure | Data |
|---|---|---|---|
| 52 | P30 | 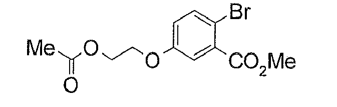 | EI: 316, 318 |
| 53 | P15 | 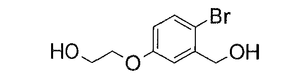 | EI: 246, 248 |
| 54 | P17 | 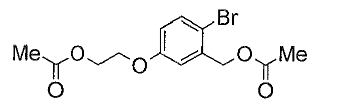 | EI: 330, 332 |
| 55 | P11 | 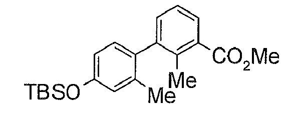 | ESI+: 371 |
| 56 | P11 | 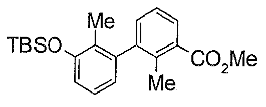 | EI: 370 |
| 57 | P11 | 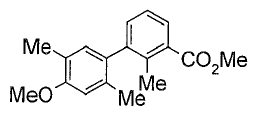 | EI: 284 |
| 58 | P11 | 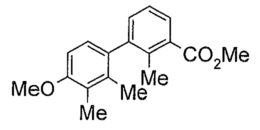 | EI: 284 |
| 59 | P12 | 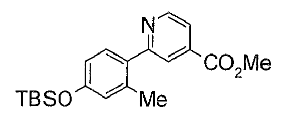 | ESI+: 358 |
| 60 | P12 | 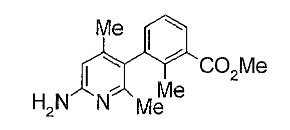 | ESI+: 271 |
| 61 | P11 | 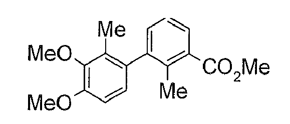 | EI: 300 |
| 62 | P12 | 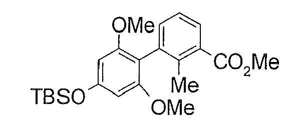 | ESI+: 417 |

**[Table 10]**

| PEx | Syn | Structure | Data |
|---|---|---|---|
| 63 | P14 | 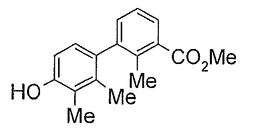 | EI: 270 |
| 64 | P14 | 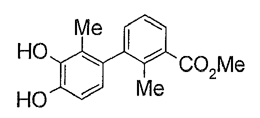 | EI: 272 |
| 65 | P15 | 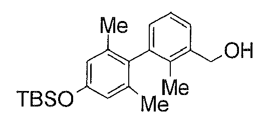 | EI: 356 |
| 66 | P15 | 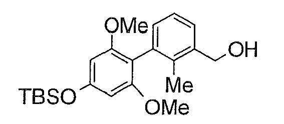 | ESI+: 389 |
| 67 | P16 | 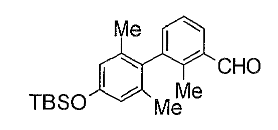 | ESI+: 355 |
| 68 | P16 | 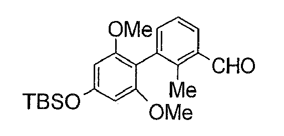 | ESI+: 387 |
| 69 | P25 | 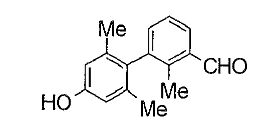 | ESI+: 241 |
| 70 | P25 | 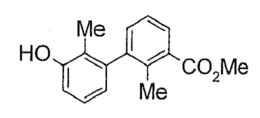 | EI: 256 |
| 71 | P25 | 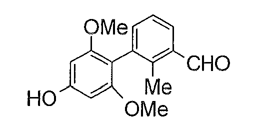 | ESI+: 273 |
| 72 | P30 | 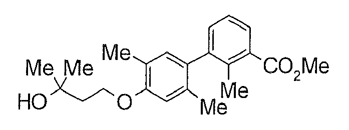 | EI: 356 |

**[Table 11]**

| PEx | Syn | Structure | Data |
|---|---|---|---|
| 73 | P30 | 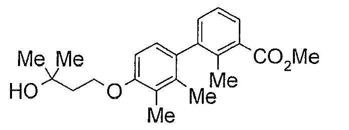 | EI: 356 |
| 74 | P30 | 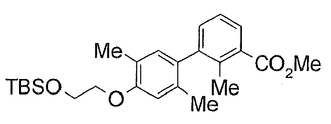 | CI: 429 |
| 75 | P30 | 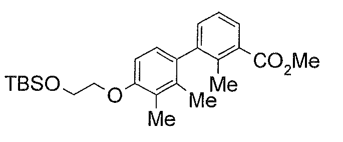 | CI: 429 |
| 76 | P30 | 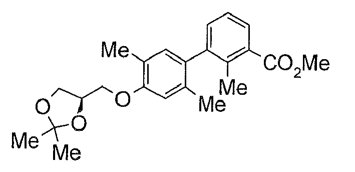 | EI: 385 |
| 77 | P30 | 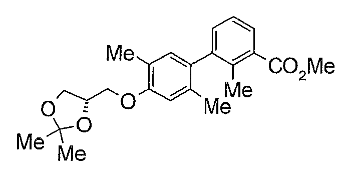 | EI: 385 |
| 78 | P30 | 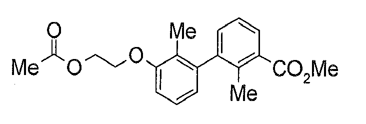 | EI: 342 |
| 79 | P30 | 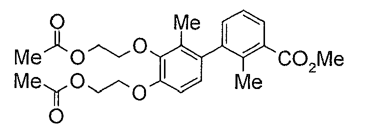 | FAB+: 445 |
| 80 | P12 | 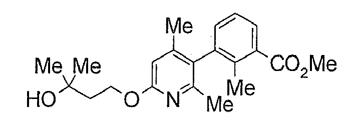 | ESI+: 358 |
| 81 | P12 | 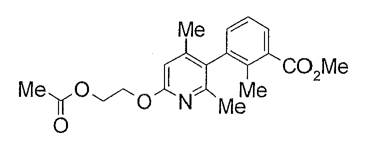 | ESI+: 358 |

**[Table 12]**

| PEx | Syn | Structure | Data |
|---|---|---|---|
| 82 | P12 | 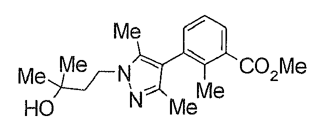 | ESI+: 331 |
| 83 | P12 | 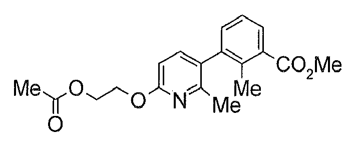 | ESI+: 344 |
| 84 | P12 | 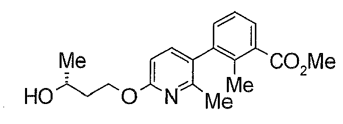 | ESI+: 330 |
| 85 | P12 | 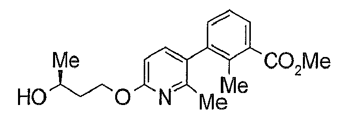 | ESI+: 330 |
| 86 | P12 | 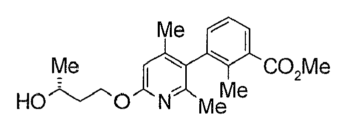 | ESI+: 344 |
| 87 | P12 | 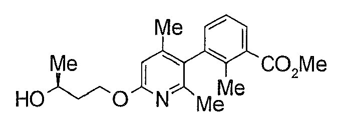 | ESI+: 344 |
| 88 | P12 | 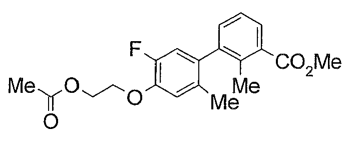 | CI: 361 |
| 89 | P12 | 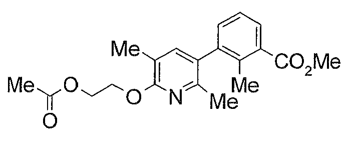 | EI: 357 |
| 90 | P12 | 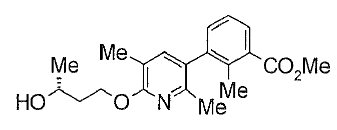 | EI: 343 |
| 91 | P12 | 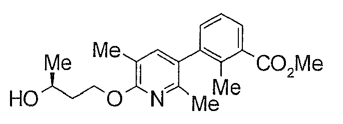 | EI: 343 |

**[Table 13]**

| PEx | Syn | Structure | Data |
|---|---|---|---|
| 92 | P12 | 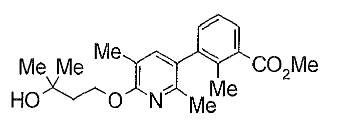 | EI: 357 |
| 93 | P12 | 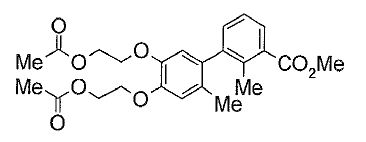 | EI: 444 |
| 94 | P11 | 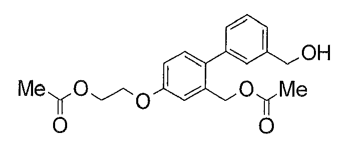 | EI: 358 |
| 95 | P30 | 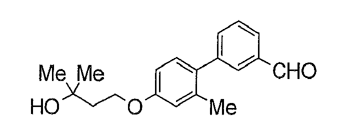 | ESI-MS [M-OH]⁺: 281 |
| 96 | P30 | 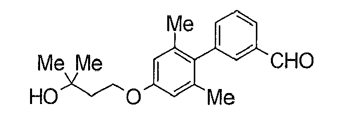 | ESI-MS [M-OH]⁺: 295 |
| 97 | P30 | 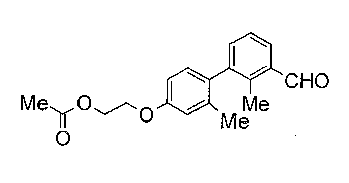 | NMR2: 2.01(3H,s),2.12(3H,s),2.37 (3H,s),4.18-4.25(2H,m),4.42-4.50(2H,m),6.81 (1H,dd,J=2.6,8. 3Hz),6.86(1H,d,J=2.6Hz),7.00( 1H,d,J=8.3Hz),7.34(1H,dd,J=1. 7,7.5Hz),7.39(1H,t,J=7.5Hz),7.8 2(1H,dd,J=1.7,7.5Hz),10.37(1H ,s) |
| 98 | P30 | 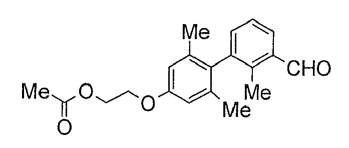 | ESI+: 327 |

**[Table 14]**

| PEx | Syn | Structure | Data |
|---|---|---|---|
| 99 | P30 | 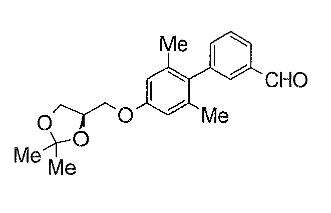 | NMR2: 1.42(3H,s),1.49(3H,s),1.99 (6H,s),3.86-4.02(2H,m),4.04-4.24(2H,m),4.44-4.56(1H,m),6.70(2H,s),7.38-7.45(1H,m),7.59(1H,t,J=7.6 Hz),7.63-7.68(1H,m),7.83-7.89(1H,m),10.05(1H,s) |
| 100 | P30 | 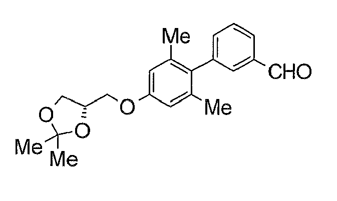 | NMR2: 1.42(3H,s),1.49(3H,s),1.99 (6H,s),3.86-4.02(2H,m),4.04-4.24(2H,m),4.44-4.56(1H,m),6.70(2H,s),7.38-7.45(1H,m),7.59(1H,t,J=7.6 Hz),7.63-7.68(1H,m),7.83-7.89(1H,m),10.05(1H,s) |
| 101 | P30 | 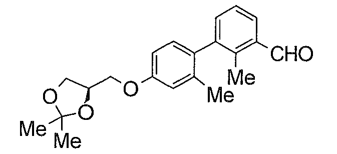 | ESI+: 341 |
| 102 | P30 | 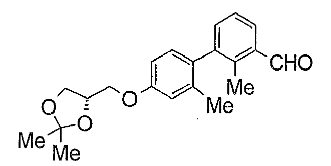 | ESI+: 341 |
| 103 | P30 | 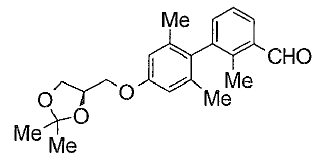 | ESI+: 355 |
| 104 | P30 | 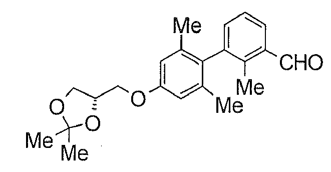 | ESI+: 355 |

**[Table 15]**

| PEx | Syn | Structure | Data |
|---|---|---|---|
| 105 | P30 | 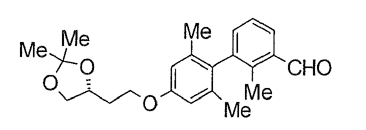 | EI: 368 |
| 106 | P30 | 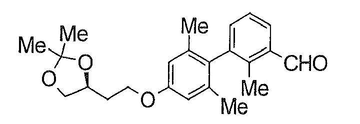 | EI: 368 |
| 107 | P30 | 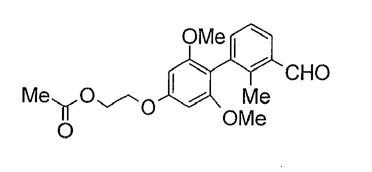 | NMR2: 2.07(3H,s),2.26(3H,s), 3.64(6H,s),4.23-4.32(2H,m),4.32-4.42(2H,m),6.37(2H,s),7. 25-7.33(1H,m),7.38(1H,t,J= 7.6Hz),7.72-7.80(1H,m),10.28(1H,s) |
| 108 | P30 | 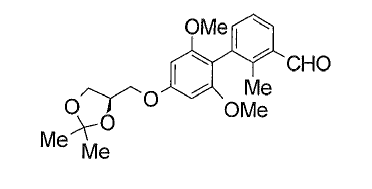 | NMR1: 1.33(3H,s),1.39(3H,s), 2.26(3H,s),3.64(6H,s),3.7 4-3.83(1H,m),4.02-4.19(3H,m),4.37-4.52(1H,m),6.36(2H,s),7. 29(1H,dd,J=1.4,7.5Hz),7. 38(1H,t,J=7.6Hz),7.75(1 H,dd,J=1.4,7.7Hz),10.28( 1H,s) |
| 109 | P30 | 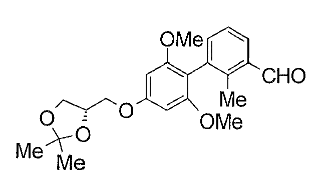 | NMR1: 1.33(3H,s),1.39(3H, s),2.2G(3H,s),3.64(6H,s), 3.74-3.83(1H,m),4.02-4.19(3H,m),4.37-4.52(1H,m),6.36(2H,s),7. 29(1H,dd,J=1.4,7.5Hz),7. 38(1H,t,J=7.6Hz),7.75(1 H,dd,J=1.4,7.7Hz),10.28( 1H,s) |
| 110 | P20 | 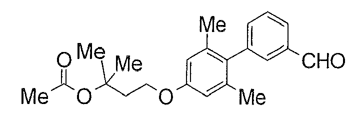 | ESI-MS [M-OAc]⁺: 295 |

**[Table 16]**

| PEx | Syn | Structure | Data |
|---|---|---|---|
| 111 | P15 | 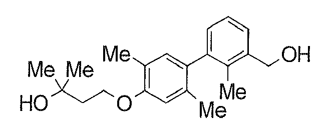 | EI: 328 |
| 112 | P15 | 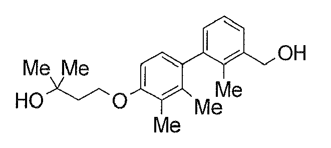 | EI: 328 |
| 113 | P15 | 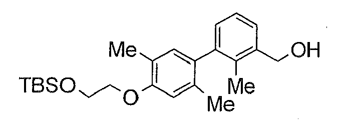 | CI: 401 |
| 114 | P15 | 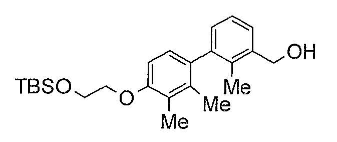 | CI: 401 |
| 115 | P15 | 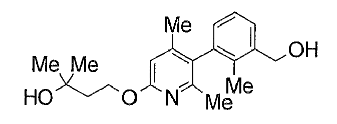 | ESI+: 330 |
| 116 | P15 | 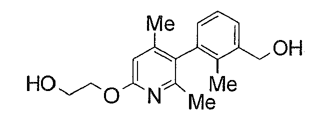 | ESI+: 288 |
| 117 | P15 | 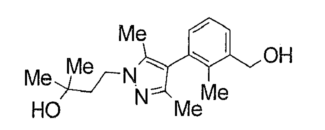 | ESI+: 303 |
| 118 | P15 | 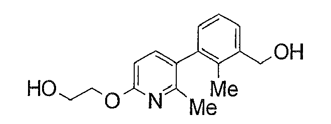 | EI: 273 |
| 119 | P15 | 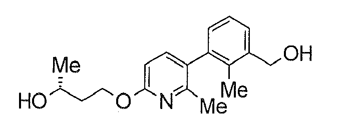 | EI: 301 |
| 120 | P15 | 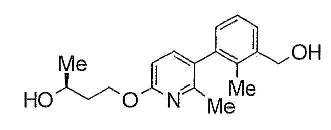 | EI: 301 |

**[Table 17]**

| PEx | Syn | Structure | Data |
|---|---|---|---|
| 121 | P15 | 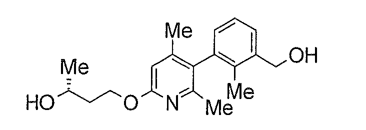 | ESI+: 316 |
| 122 | P15 | 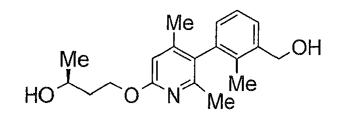 | ESI+: 316 |
| 123 | P15 | 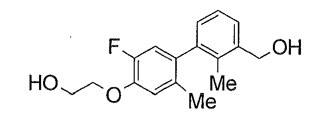 | EI: 290 |
| 124 | P15 | 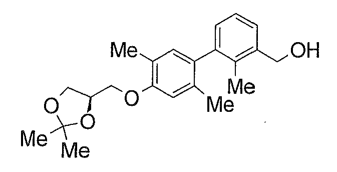 | ESI+: 357 |
| 125 | P15 | 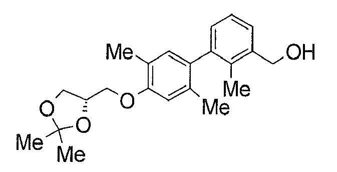 | ESI+: 357 |
| 126 | P15 | 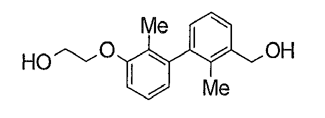 | EI: 272 |
| 127 | P15 | 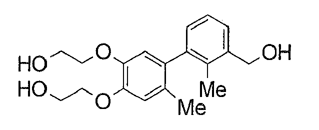 | EI: 332 |
| 128 | P15 | 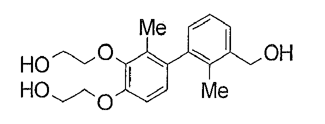 | ESI+: 333 |
| 129 | P11 | 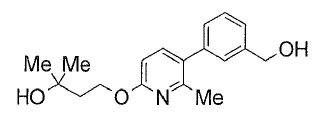 | ESI+: 302 |
| 130 | P12 | 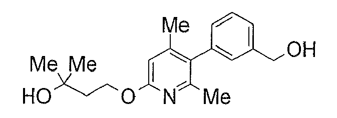 | ESI+: 316 |

**[Table 18]**

| PEx | Syn | Structure | Data |
|---|---|---|---|
| 131 | P12 | 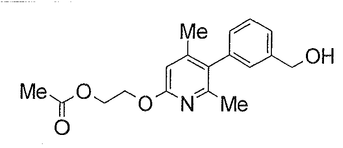 | EI: 315 |
| 132 | P11 | 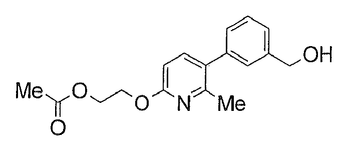 | ESI+: 302 |
| 133 | P16 | 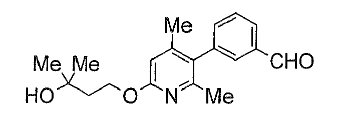 | ESI+: 314 |
| 134 | P16 | 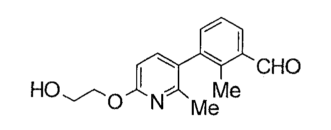 | EI: 271 |
| 135 | P16 | 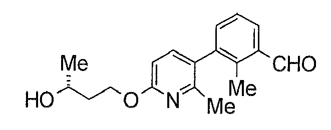 | CI: 300 |
| 136 | P16 | 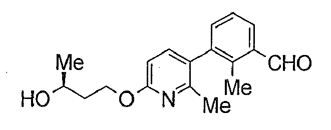 | CI: 300 |
| 137 | P16 | 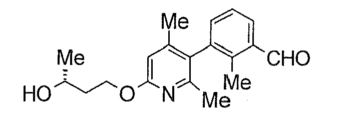 | ESI+: 314 |
| 138 | P16 | 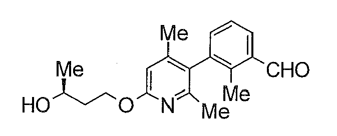 | ESI+: 314 |
| 139 | P16 | 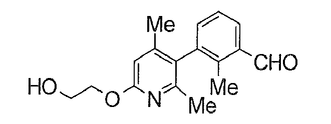 | ESI+: 286 |
| 140 | P16 | 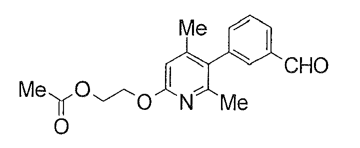 | EI: 313 |

**[Table 19]**

| PEx | Syn | Structure | Data |
|---|---|---|---|
| 141 | P16 | 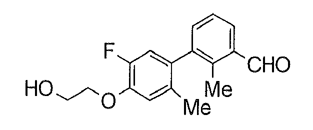 | EI: 288 |
| 142 | P16 | 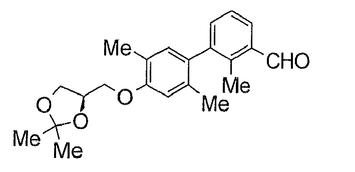 | EI: 355 |
| 143 | P16 | 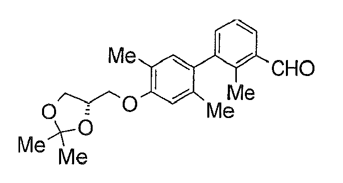 | EI: 355 |
| 144 | P16 | 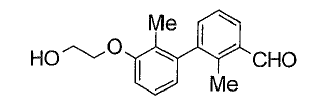 | EI: 270 |
| 145 | P22 | 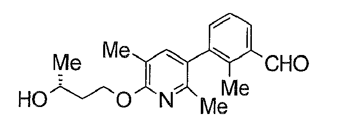 | EI: 313 |
| 146 | P22 | 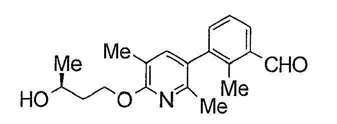 | EI: 313 |
| 147 | P22 | 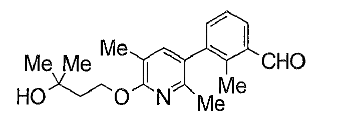 | EI: 327 |
| 148 | P20 | 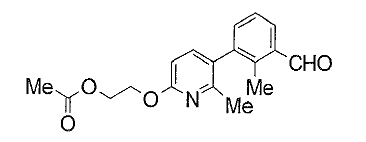 | EI: 313 |
| 149 | P20 | 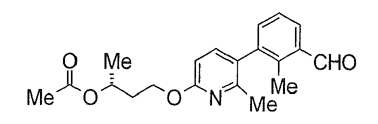 | EI: 341 |

**[Table 20]**

| PEx | Syn | Structure | Data |
|---|---|---|---|
| 150 | P20 | 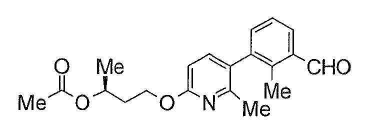 | EI: 341 |
| 151 | P17 | 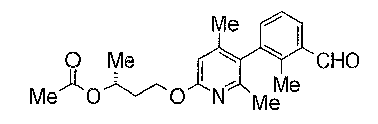 | EI: 356 |
| 152 | P17 | 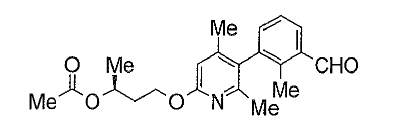 | ESI+: 356 |
| 153 | P17 | 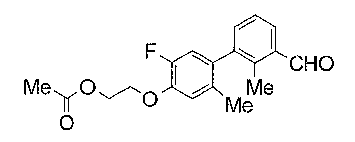 | CI: 331 |
| 154 | P17 | 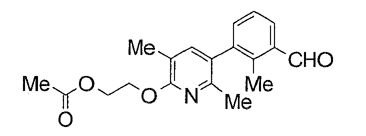 | FAB+: 328 |
| 155 | P17 | 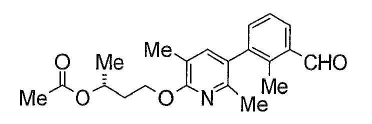 | FAB+: 356 |
| 156 | P17 | 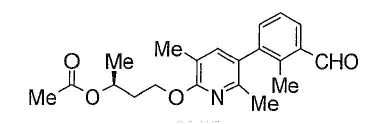 | FAB+: 356 |
| 157 | P20 | 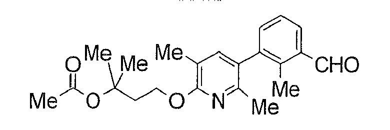 | FAB+: 370 |
| 158 | P17 | 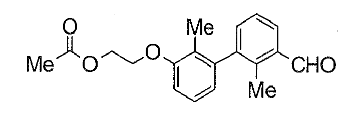 | EI: 312 |

**[Table 21]**

| PEx | Syn | Structure | Data |
|---|---|---|---|
| 159 | P18 | 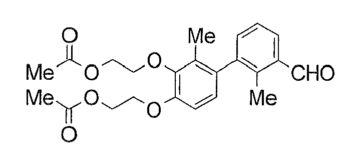 | NMR2: 1.96(3H,s),2.09(3H, s),2.12(3H,s),2.36(3H,s), 4.15-4.58(8H,m),6.73-6.88(2H,m),7.29-7.46(2H,m),7.74-7.92(1H,m),10.36(1H,s) |
| 160 | P30 | 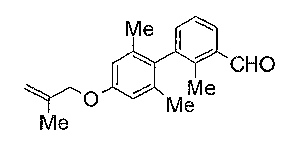 | EI: 294 |
| 161 | P20 | 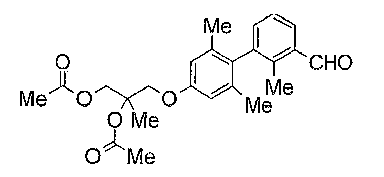 | EI: 412 |
| 162 | P24 | 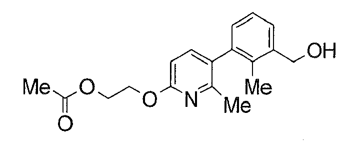 | EI: 315 |
| 163 | P24 | 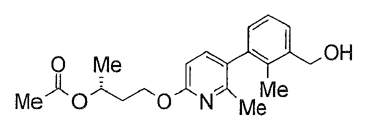 | EI: 343 |
| 164 | P24 | 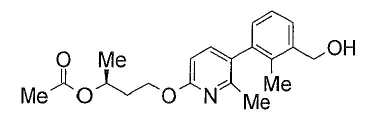 | EI: 343 |
| 165 | P24 | 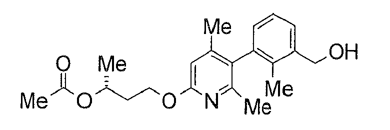 | EI: 358 |
| 166 | P24 | 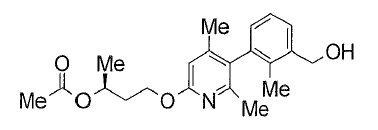 | EI: 358 |

**[Table 22]**

| PEx | Syn | Structure | Data |
|---|---|---|---|
| 167 | P24 | 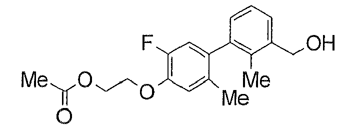 | EI: 332 |
| 168 | P24 | 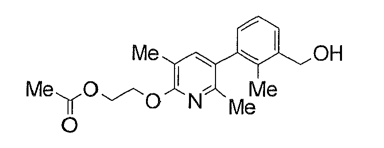 | FAB+: 330 |
| 169 | P24 | 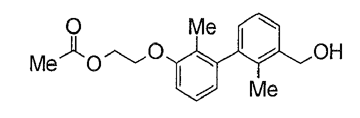 | EI: 314 |
| 170 | P7 | 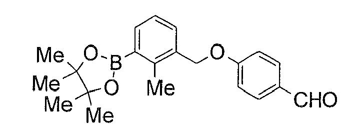 | CI: 352 |
| 171 | P29 | 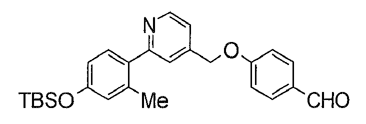 | ESI+: 434 |
| 172 | P29 | 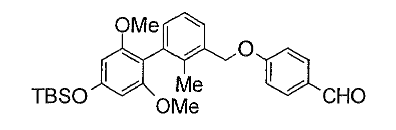 | ESI+: 493 |

**[Table 23]**

| PEx | Syn | Structure | Data |
|---|---|---|---|
| 173 | P12 | 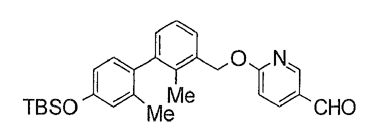 | EI: 447 |
| 174 | P12 | 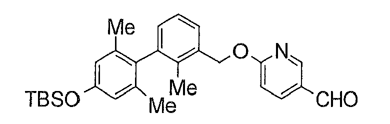 | ESI-: 460 |
| 175 | P11 | 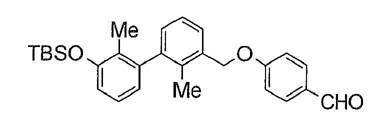 | ESI+: 447 |
| 176 | P12 | 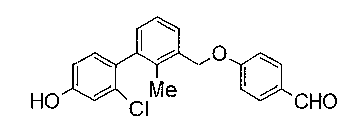 | ESI-: 351 |
| 177 | P25 | 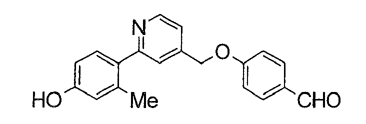 | ESI+: 320 |
| 178 | P25 | 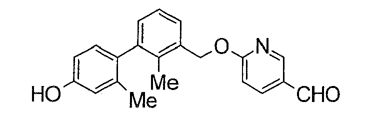 | ESI-: 332 |
| 179 | P25 | 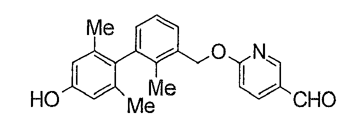 | ESI-: 346 |
| 180 | P25 | 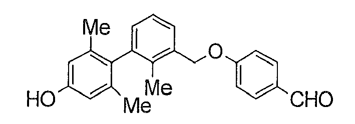 | ESI-: 345 |
| 181 | P25 | 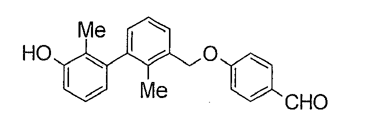 | ESI+: 333 |
| 182 | P25 | 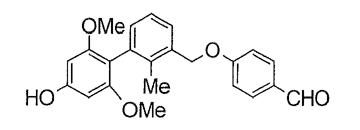 | ESI-: 377 |

**[Table 24]**

| PEx | Syn | Structure | Data |
|---|---|---|---|
| 183 | P17 | 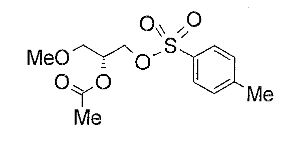 | FAB+: 303 |
| 184 | P30 | 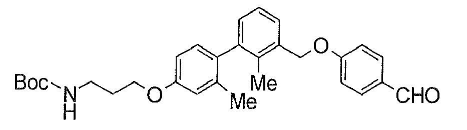 | ESI-: 488 |
| 185 | P30 | 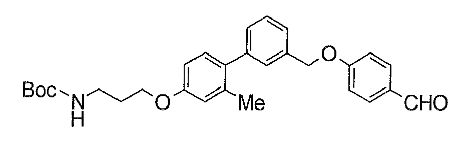 | ESI+: 476 |
| 186 | P30 | 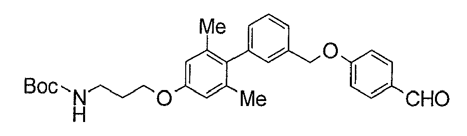 | ESI-: 488 |
| 187 | P30 | 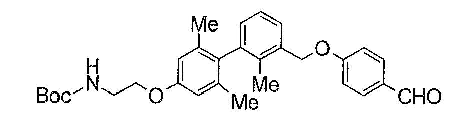 | FAB-: 488 |
| 188 | P30 | 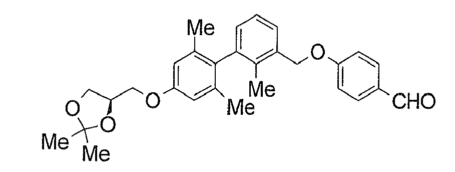 | ESI-: 459 |
| 189 | P30 | 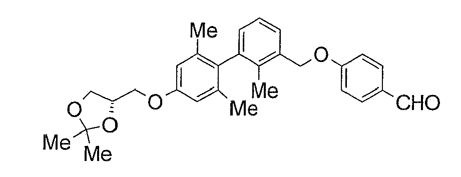 | ESI-: 459 |

**[Table 25]**

| PEx | Syn | Structure | Data |
|---|---|---|---|
| 190 | P30 | 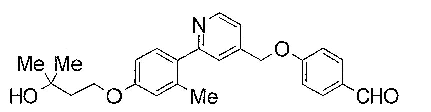 | ESI+: 406 |
| 191 | P30 | 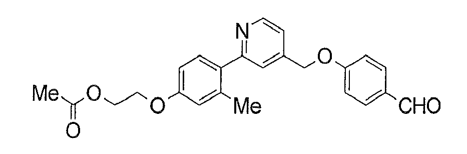 | ESI+: 406 |
| 192 | P30 | 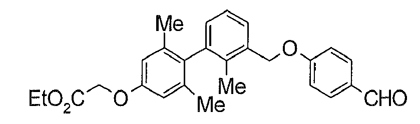 | ESI+: 433 |
| 193 | P30 | 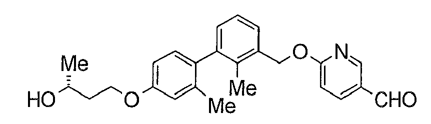 | CI: 405 |
| 194 | P30 | 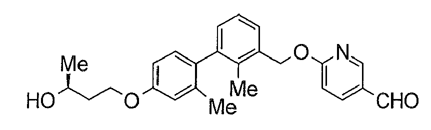 | CI: 405 |
| 195 | P30 | 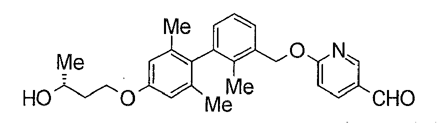 | CI: 419 |
| 196 | P30 | 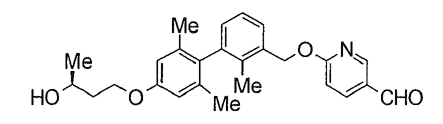 | CI: 419 |
| 197 | P30 | 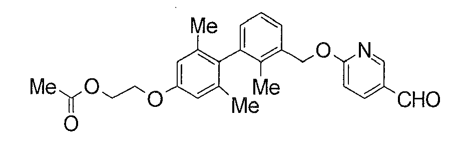 | EI-: 433 |
| 198 | P30 | 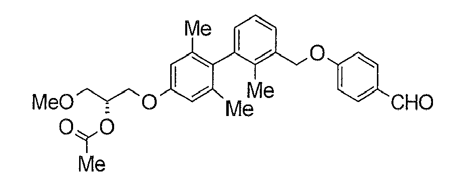 | ESI-: 475 |

**[Table 26]**

| PEx | Syn | Structure | Data |
|---|---|---|---|
| 199 | P30 | 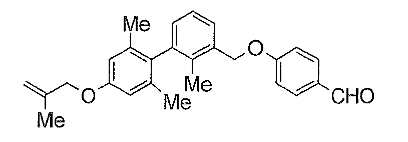 | ESI-: 399 |
| 200 | P30 | 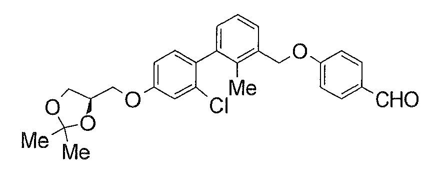 | ESI-: 465 |
| 201 | P30 | 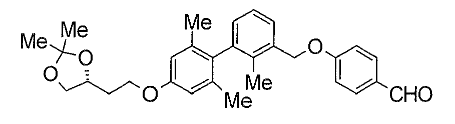 | ESI-: 473 |
| 202 | P30 | 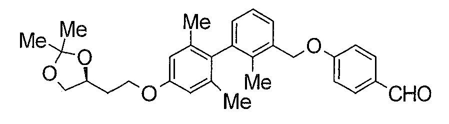 | ESI-: 473 |
| 203 | P30 | 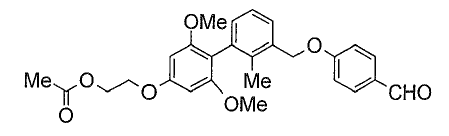 | ESI-: 463 |

**[Table 27]**

| PEx | Syn | Structure | Data |
|---|---|---|---|
| 204 | P25 | 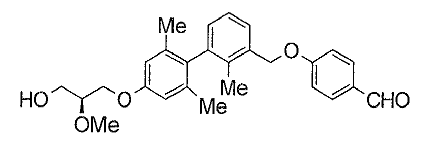 | ESI-: 433 |
| 205 | P19 | 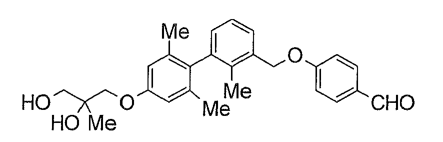 | ESI-: 433 |
| 206 | P17 | 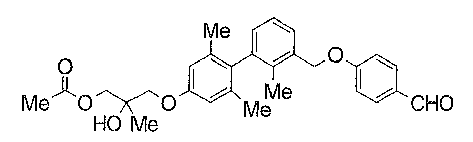 | FAB+: 477 |
| 207 | P29 | 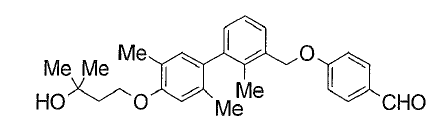 | FAB+: 433 |
| 208 | P29 | 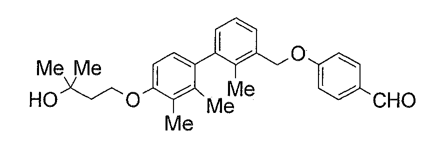 | FAB+: 433 |
| 209 | P29 | 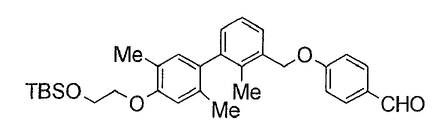 | FAB+: 505 |
| 210 | P29 | 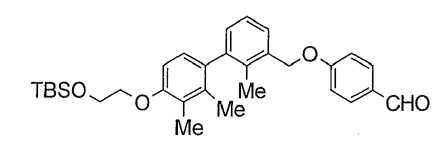 | FAB+: 505 |
| 211 | P29 | 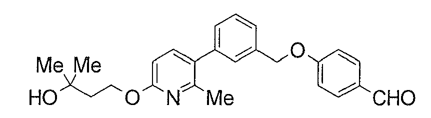 | ESI+: 406 |
| 212 | P29 | 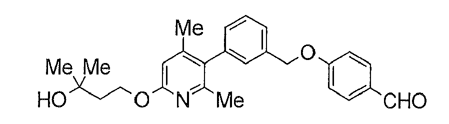 | ESI+: 420 |

**[Table 28]**

| PEx | Syn | Structure | Data |
|---|---|---|---|
| 213 | P29 | 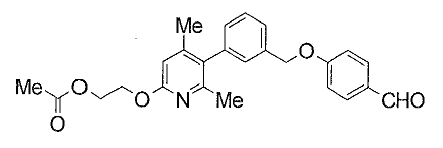 | ESI+: 420 |
| 214 | P29 | 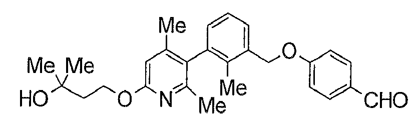 | ESI+: 434 |
| 215 | P29 | 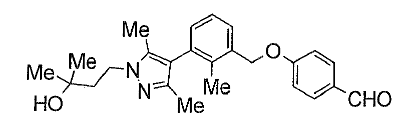 | ESI+: 407 |
| 216 | P35 | 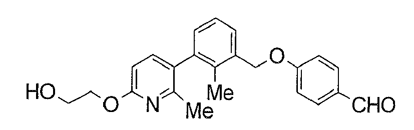 | FAB+: 378 |
| 217 | P29 | 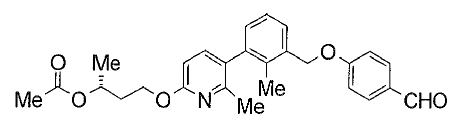 | FAB+: 448 |
| 218 | P29 | 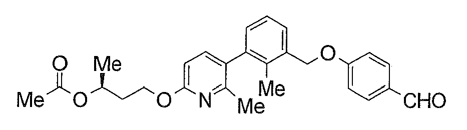 | FAB+: 448 |

**[Table 29]**

| PEx | Syn | Structure | Data |
|---|---|---|---|
| 219 | P29 | 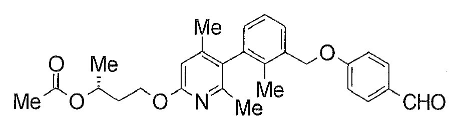 | ESI+: 462 |
| 220 | P29 | 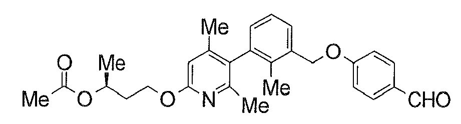 | ESI+: 462 |
| 221 | P29 | 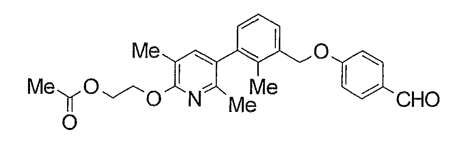 | ESI+: 434 |
| 222 | P29 | 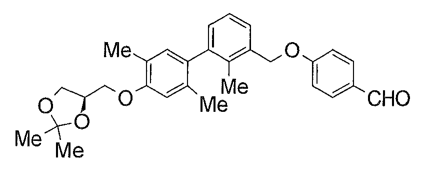 | ESI-: 459 |
| 223 | P29 | 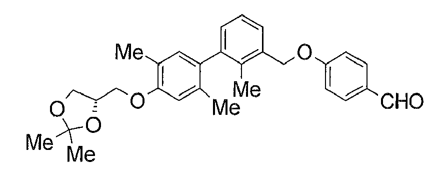 | ESI-: 459 |
| 224 | P29 | 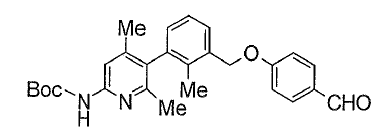 | ESI-: 445 |
| 225 | P29 | 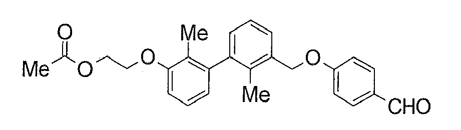 | ESI+: 419 |
| 226 | P29 | 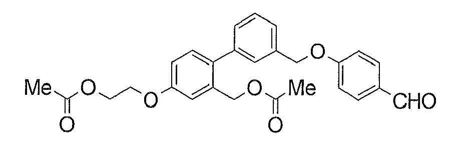 | FAB+: 463 |

**[Table 30]**

| PEx | Syn | Structure | Data |
|---|---|---|---|
| 227 | P30 | 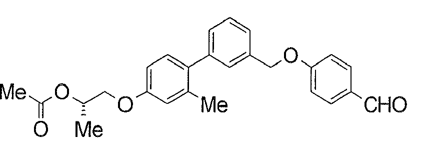 | ESI-: 417 |
| 228 | P30 | 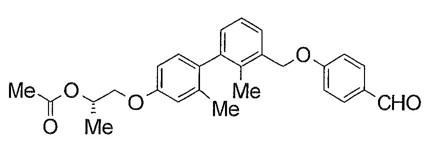 | ESI-: 431 |
| 229 | P30 | 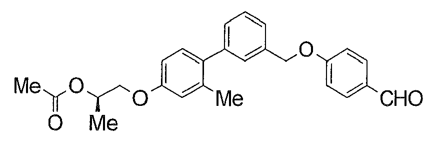 | ESI+: 419 |
| 230 | P30 | 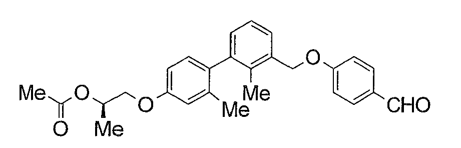 | ESI-: 431 |
| 231 | P30 | 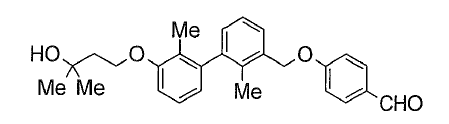 | FAB-MS [M]⁺: 418 |
| 232 | P30 | 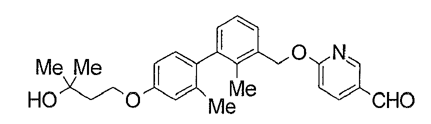 | ESI-: 418 |
| 233 | P25 | 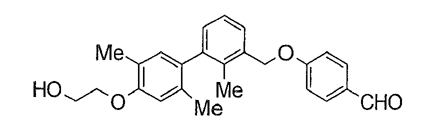 | EI: 390 |
| 234 | P25 | 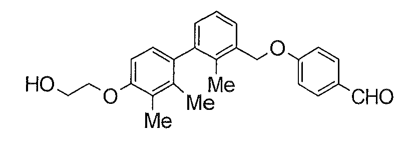 | CI: 391 |
| 235 | P12 | 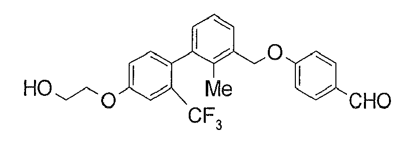 | ESI-: 429 |

**[Table 31]**

| Ex | Structure |
|---|---|
| 1 | 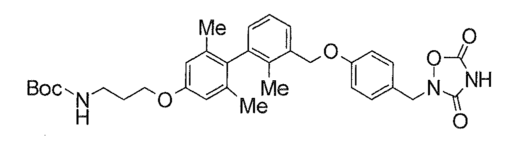 |
| 2 | 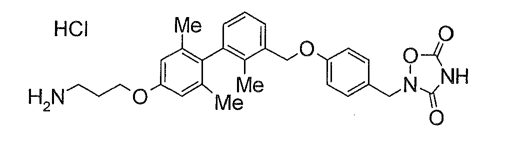 |
| 3 | 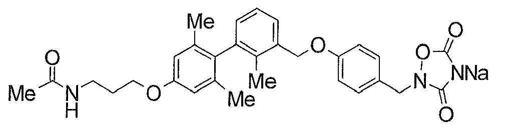 |
| 4 | 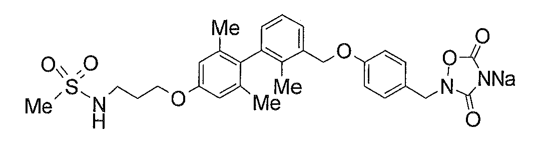 |
| 5 | 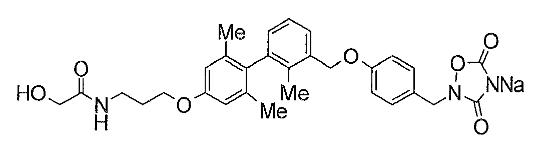 |
| 6 | 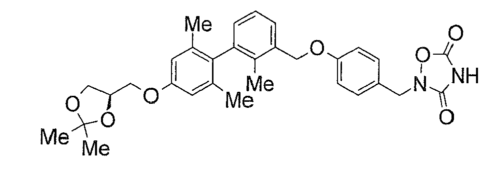 |
| 7 | 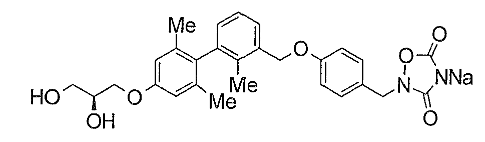 |
| 8 | 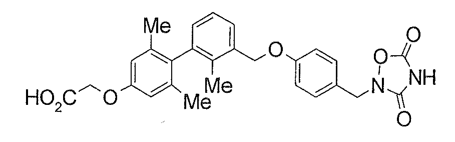 |

**[Table 32]**

| Ex | Structure |
|---|---|
| 9 | 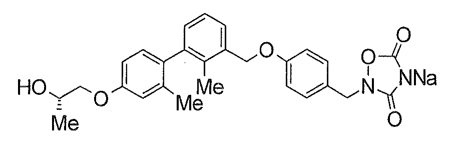 |
| 10 | 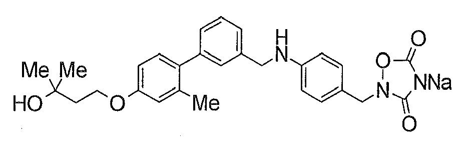 |
| 11 | 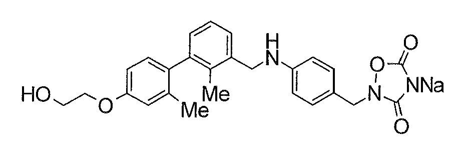 |
| 12 | 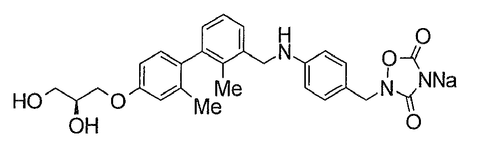 |
| 13 | 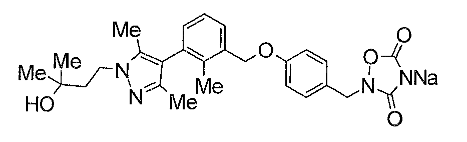 |
| 14 | 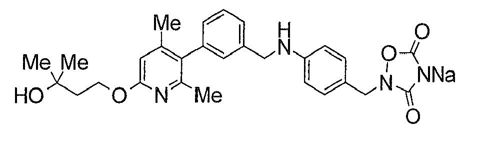 |
| 15 | 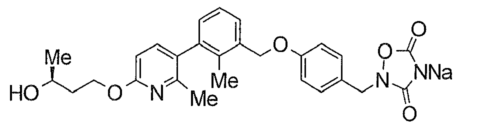 |
| 16 | 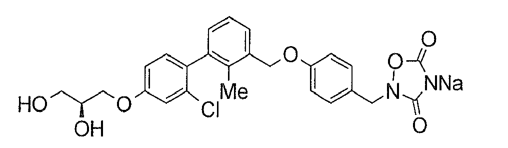 |
| 17 | 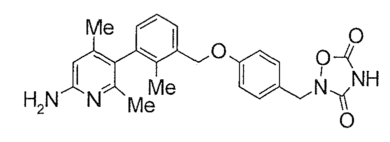 |

**[Table 33]**

| Ex | Structure |
|---|---|
| 18 | 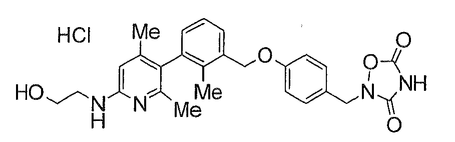 |
| 19 | 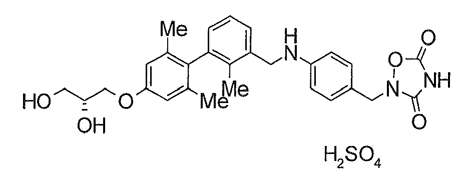 |
| 20 | 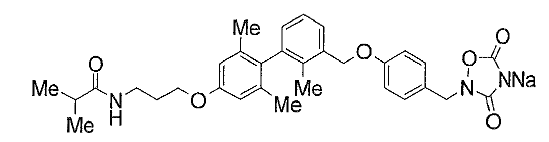 |
| 21 | 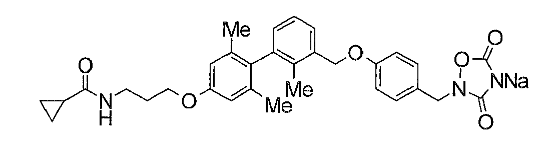 |
| 22 | 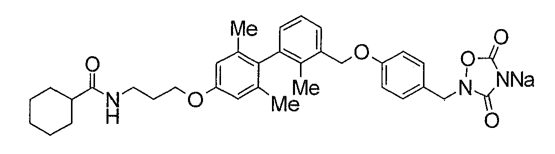 |
| 23 | 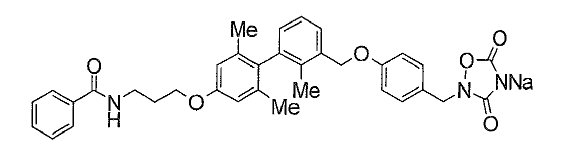 |
| 24 | 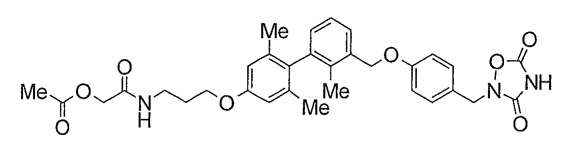 |
| 25 | 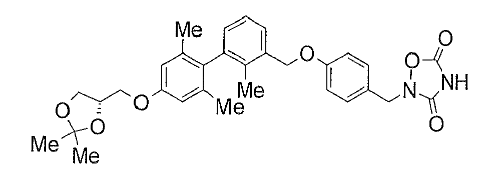 |

**[Table 34]**

| Ex | Structure |
|---|---|
| 26 | 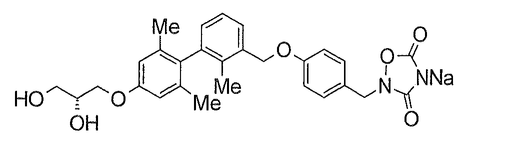 |
| 27 | 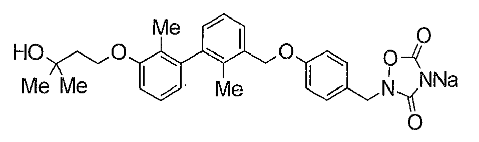 |
| 28 | 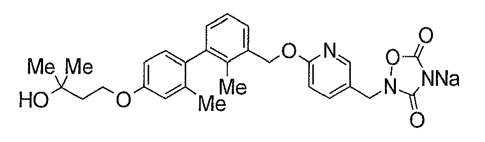 |
| 29 | 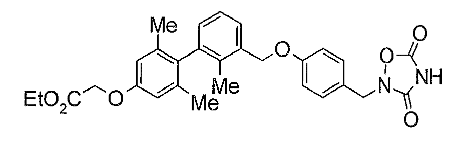 |
| 30 | 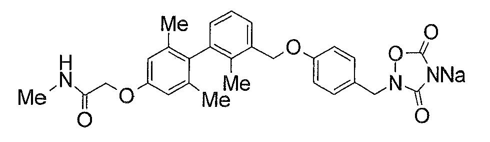 |
| 31 | 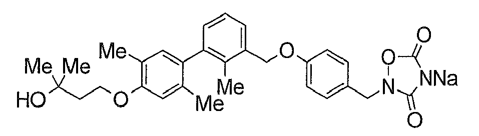 |
| 32 | 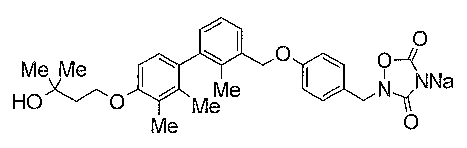 |
| 33 | 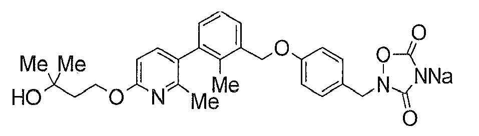 |
| 34 | 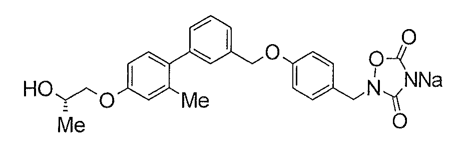 |

**[Table 35]**

| Ex | Structure |
|---|---|
| 35 | 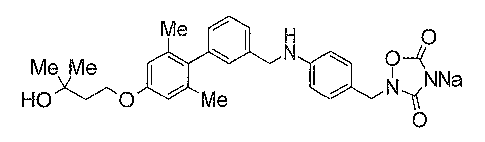 |
| 36 | 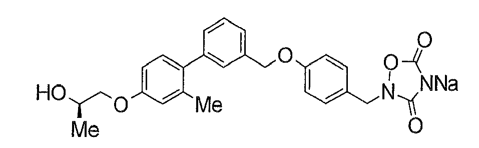 |
| 37 | 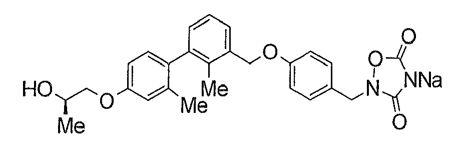 |
| 38 | 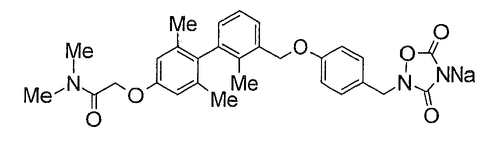 |
| 39 | 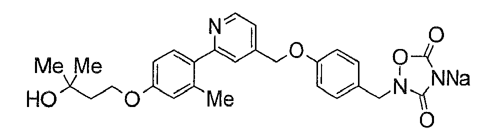 |
| 40 | 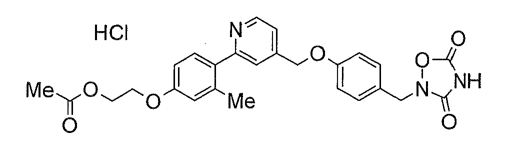 |
| 41 | 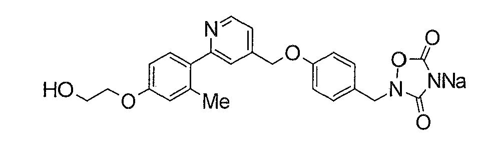 |
| 42 | 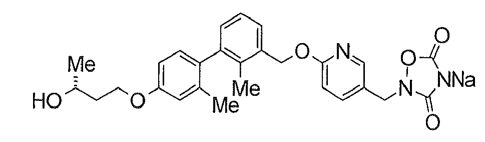 |
| 43 | 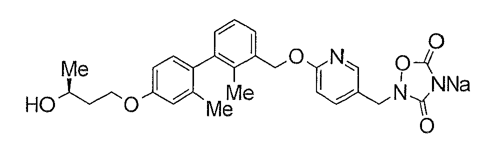 |

**[Table 36]**

| Ex | Structure |
|---|---|
| 44 | 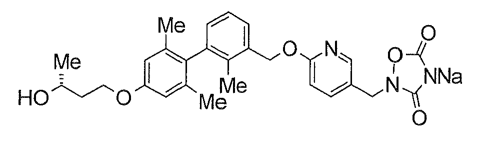 |
| 45 | 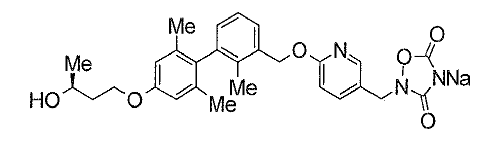 |
| 46 | 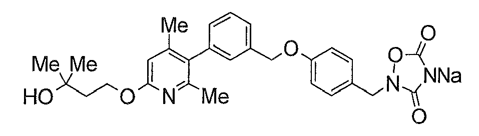 |
| 47 | 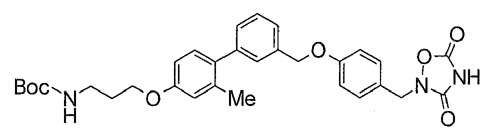 |
| 48 | 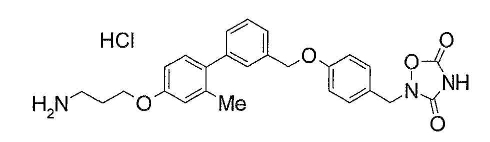 |
| 49 | 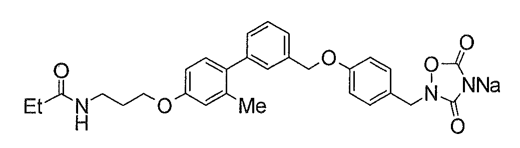 |
| 50 | 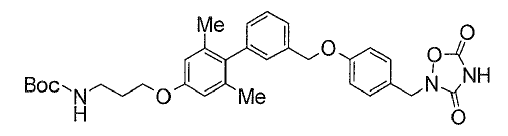 |
| 51 | 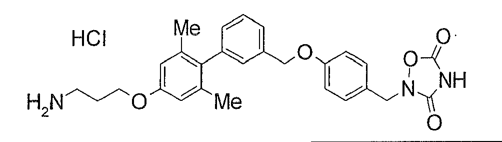 |
| 52 | 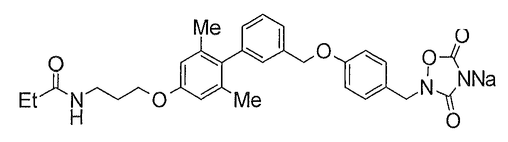 |

**[Table 37]**

| Ex | Structure |
|---|---|
| 53 | 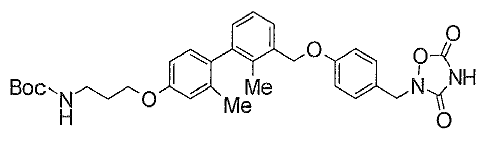 |
| 54 | 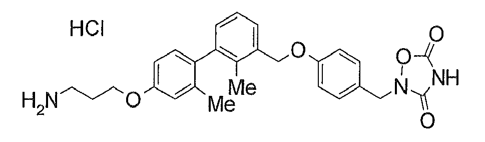 |
| 55 | 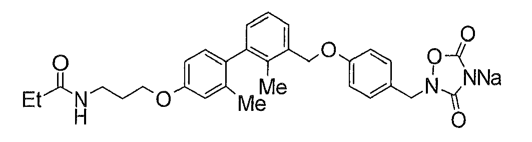 |
| 56 | 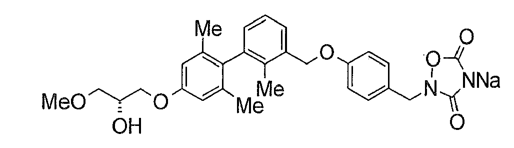 |
| 57 | 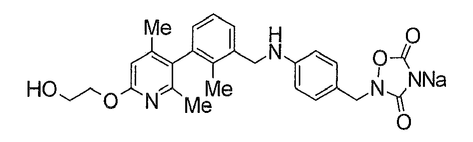 |
| 58 | 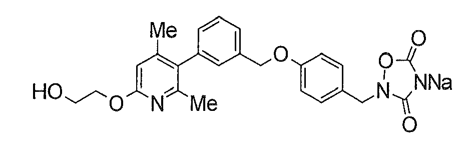 |
| 59 | 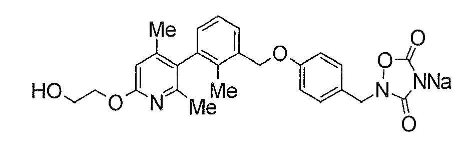 |
| 60 | 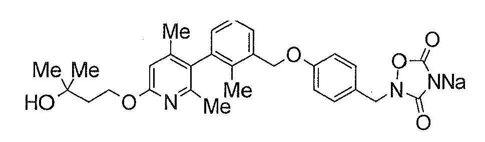 |
| 61 | 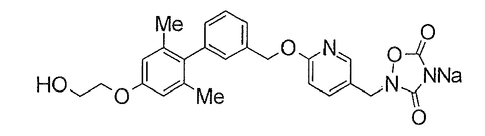 |

**[Table 38]**

| Ex | Structure |
|---|---|
| 62 | 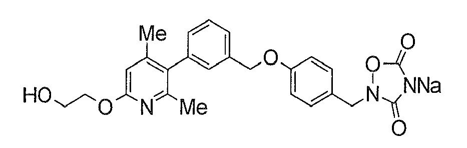 |
| 63 | 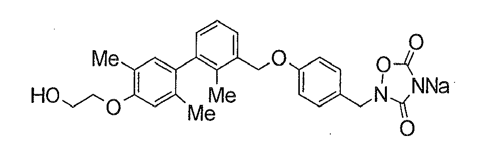 |
| 64 | 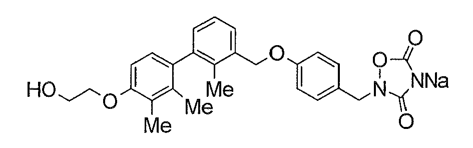 |
| 65 | 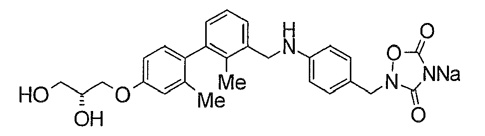 |
| 66 | 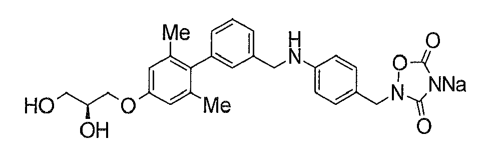 |
| 67 | 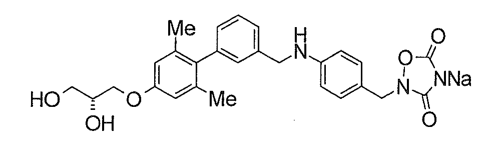 |
| 68 | 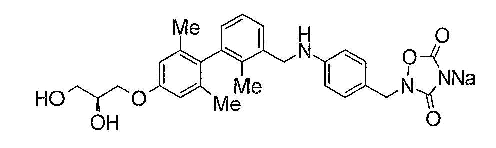 |

**[Table 39]**

| Ex | Structure |
|---|---|
| 69 | 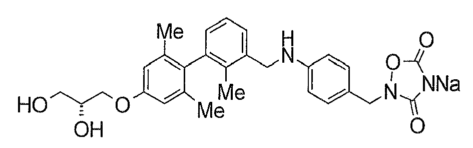 |
| 70 | 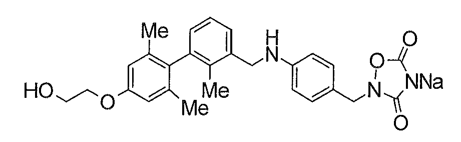 |
| 71 | 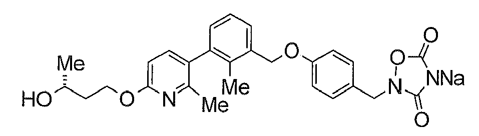 |
| 72 | 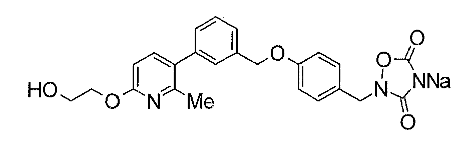 |
| 73 | 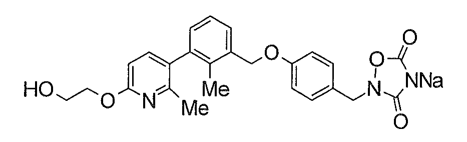 |
| 74 | 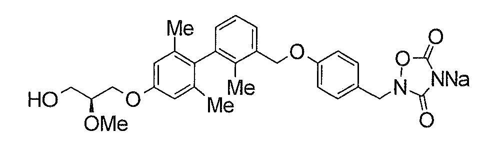 |
| 75 | 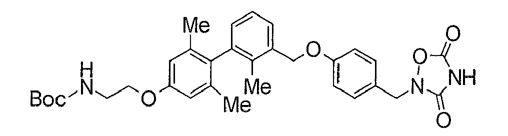 |

**[Table 40]**

| Ex | Structure |
|---|---|
| 76 | 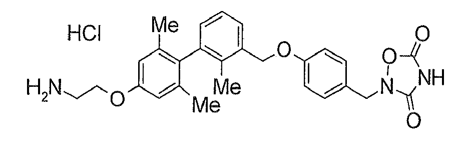 |
| 77 | 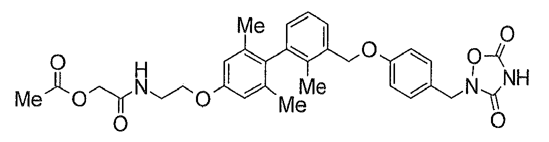 |
| 78 | 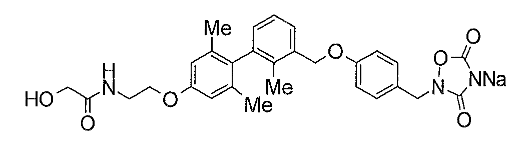 |
| 79 | 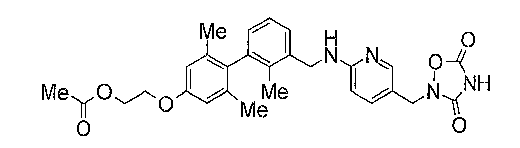 |
| 80 | 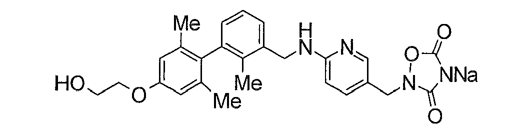 |
| 81 | 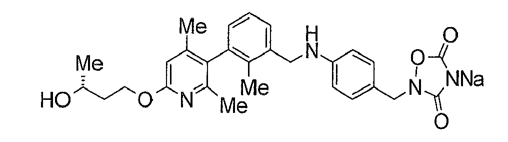 |
| 82 | 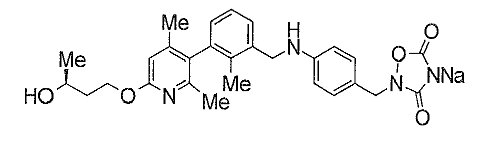 |
| 83 | 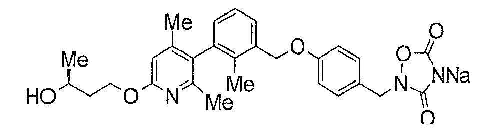 |

**[Table 41]**

| Ex | Structure |
|---|---|
| 84 | 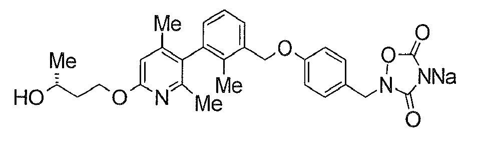 |
| 85 | 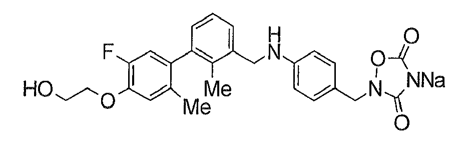 |
| 86 | 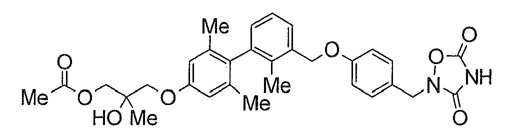 |
| 87 | 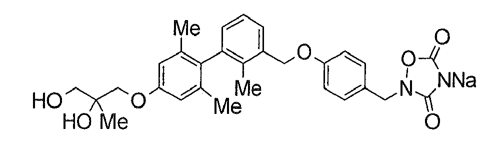 |
| 88 | 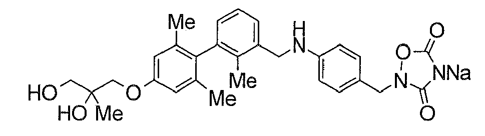 |
| 89 | 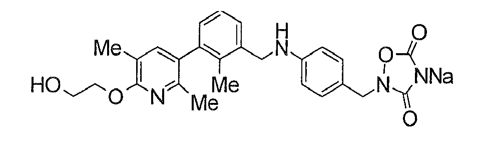 |
| 90 | 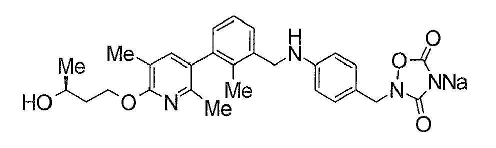 |
| 91 | 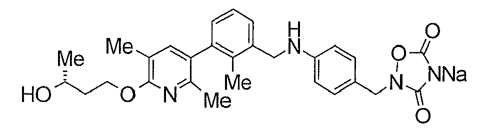 |

**[Table 42]**

| Ex | Structure |
|---|---|
| 92 | 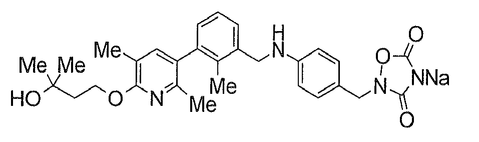 |
| 93 | 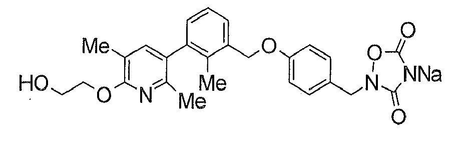 |
| 94 | 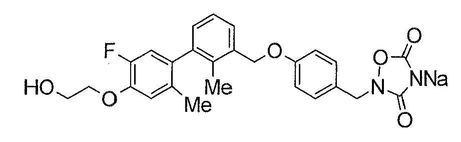 |
| 95 | 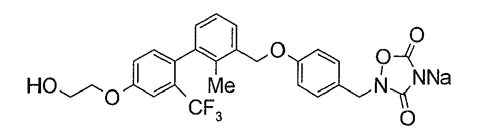 |
| 96 | 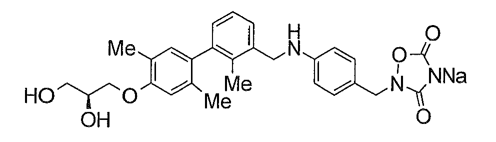 |
| 97 | 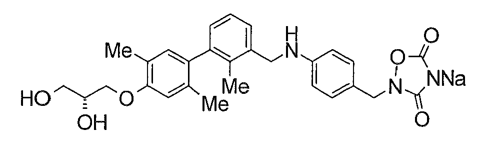 |
| 98 | 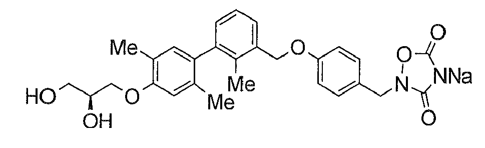 |

**[Table 43]**

| Ex | Structure |
|---|---|
| 99 | 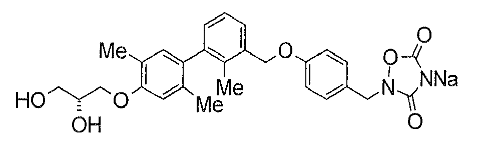 |
| 100 | 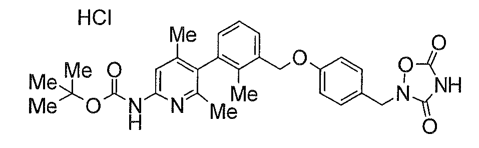 |
| 101 | 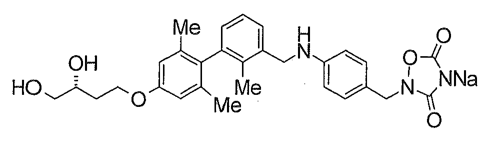 |
| 102 | 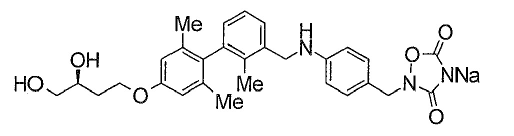 |
| 103 | 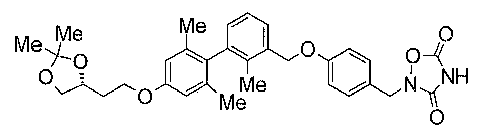 |
| 104 | 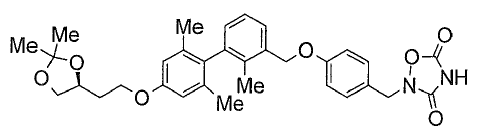 |
| 105 | 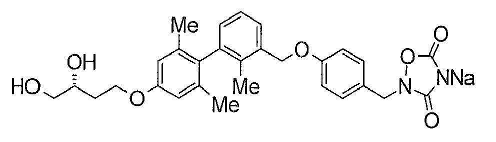 |

**[Table 44]**

| Ex | Structure |
|---|---|
| 106 | 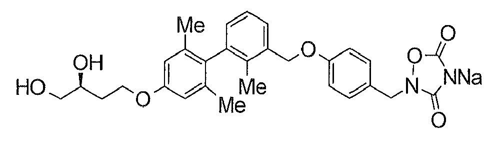 |
| 107 | 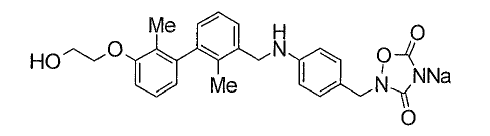 |
| 108 | 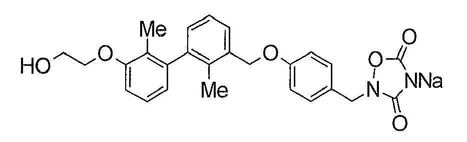 |
| 109 | 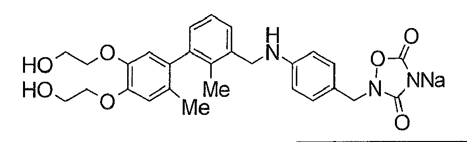 |
| 110 | 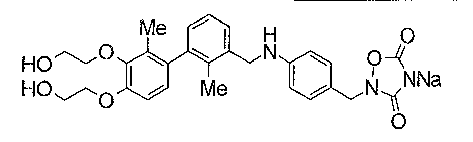 |
| 111 | 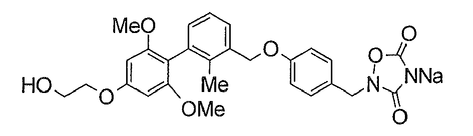 |
| 112 | 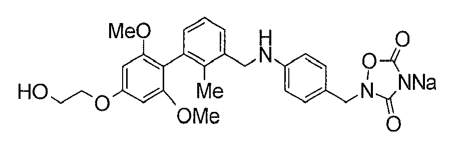 |

**[Table 45]**

| Ex | Structure |
|---|---|
| 113 | 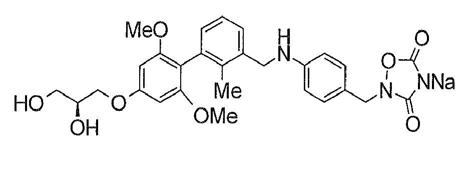 |
| 114 | 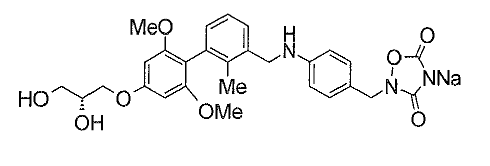 |
| 115 | 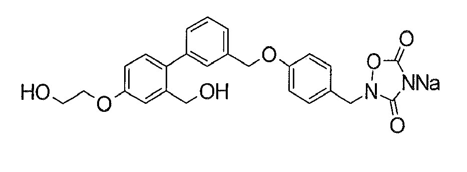 |
| 116 | 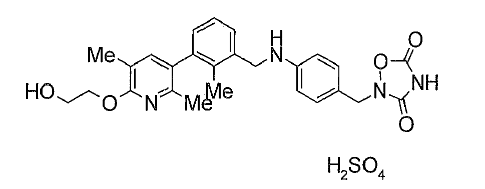 |
| 117 | 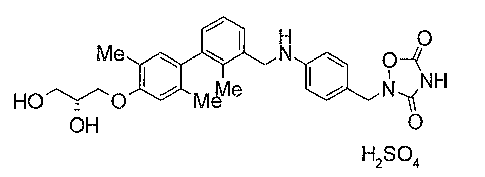 |
| 118 | 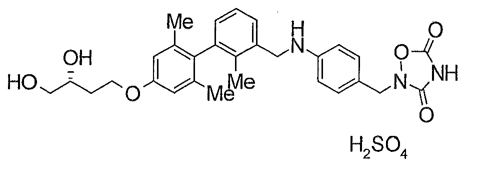 |
| 119 | 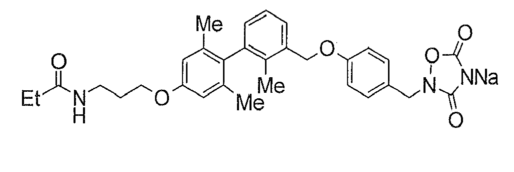 |

**[Table 46]**

| Ex | Syn | Data |
|---|---|---|
| 1 | 1 | ESI-:588 |
| 2 | 2 | ESI+:490 |
| 3 | 3 | NMR1:1.74-1.89(11H,m),1.92(3H,s),3.12-3.25(2H,m),3.95-4.06(2H,m),4.33(2H,s),5.11(2H,s),6.71(2H,s),6.95(1H,d,J=7.2Hz ),6.99(2H,d,J=8.4Hz),7.21(2H,d,J=8.4Hz),7.26(1H,dd,J=7.6,7.6 Hz),7.43(1H,d,J=7.2Hz),7.90-7.98(1H,m) ESI+:532 |
| 4 | 4 | ESI-:566 |
| 5 | 5 | ESI-:546 |
| 6 | 6 | ESI-:545 |
| 7 | 7 | NMR1:1.85(6H,s),1.92(3H,s),3.29-3.48(2H,m),3.76-3:90(2H,m),3,96- 4.03(1H,m),4.33(2H,s),4.67(1H,brs),4.95(1H,brs),5.11(2H,s),6.7 1(2H,s),6.95(1H,d,J=7.6Hz),6.99(2H,d,J=8.4Hz),7.21(2H,d,J=8. 4Hz),7.26(1H,dd,J=7.6,7.6Hz),7.42(1H,d,J=7.2Hz ESI-:505 |
| 8 | 8 | ESI-:489 |
| 9 | 9 | FAB-:475 |
| 10 | 10 | ESI+:490 |
| 11 | 11 | NMR1:1.97(3H,s),1.99(3H,s),3.73(2H,m),4.01(2H,t,J=5.0Hz),4. 19- 4.27(4H,m),4.88(1H,bs),6.02(1H,t,J=5.6Hz),6.54(2H,d,J=8.4Hz), 6.78-6.83(1H,m),6.86-6.89(1H,m),6.92-7.00(4H,m),7.13- 7.19(1H,m),7.28(1H,d,J=7.3Hz) ESI-:460 |
| 12 | 12 | NMR1:1.97(3H,s),1.99(3H,s),3.42-3.51(2H,m),3.76- 3.85(1H,m),3.85-3.92(1H,m),3.99-4.05(1H,m),4.18- 4.26(4H,m),4.66-4.74(1H,m),4.94- 5.02(1H,m),6.02(1H,t,J=5.5Hz),6.54(2H,d,J=8.5Hz),6.77- 6.83(1H,m),6.86-6.89(1H,m),6.92-7.01(4H,m),7.13- 7.19(1H,m),7.28(1H,d,J=7.5Hz) ESI-:490 |
| 13 | 13 | ESI+:493 |
| 14 | 14 | ESI-:503 |
| 15 | 15 | ESI-:490 |

**[Table 47]**

| Ex | Syn | Data |
|---|---|---|
| 16 | 16 | NMR1:2.04(3H,s),3.45-3.48(2H,m),3.79-3.83(1H,m),3.92-3.96(1H,m),4.06-4.10(1H,m),4.34(2H,s),4.69-4.72(1H,m),5.00-5.01(1H,m),5.08-5.14(2H,m),6.98-7.01(3H,m),7.09-7.10(1H,m),7.13-7.14(1H,m),7.19-7.28(4H,m),7.46-7.47(1H,m) FAB-:511 |
| 17 | 17 | ESI-:431 |
| 18 | 18 | ESI-:475 |
| 19 | 19 | NMR1:1.84(6H,s),1.88(3H,s),3.41-3.50(2H,m),3.76-3.83(1H,m),3.83-3.89(1H,m),3.97-4.02(1H,m),4.20-5.00(9H,m),6.71(2H,s),6.72-6.78(2H,m),6.86-6.91(1H,m),7.11(2H,d,J=8.4Hz),7.21(1H,t,J=7.5Hz),7.27-7.32(1H,m), 12.26-12.50(1H,br) ESI-:504 |
| 20 | 3 | ESI+:560 |
| 21 | 3 | NMR1:0.60-0.69(4H,m),1.50-1.57(1H,m),1.80-1.90(8H,m),1.92(3H,s),3.19-3.26(2H,m),3.97-4.03(2H,m),4.33(2H,s),5.11 (2H,s),6.72(2H,s),6.95(1H,d,J=6.8Hz ),6.98(2H,d,J=8.8Hz),7.21(2H,d,J=8.4Hz),7.26(1H,dd,J=7.6,7.6 Hz),7.42(1H,d,J=7.2Hz),8.13-819(1H,m) ESI+:558 |
| 22 | 3 | ESI+:600 |
| 23 | 3 | ESI+:594 |
| 24 | 3 | ESI-:588 |
| 26 | 7 | NMR1:1.85(6H,s),1.92(3H,s),3.26-3.49(2H,m),3.76-3.89(2H,m),3.94-4.03(1H,m),4.33(2H,s),4.67(1H,brs),4.95(1H,brs),5.11(2H,s),6.7 2(2H,s),6.95(1H,d,J=6.8Hz),6.98(2H,d,J=8.8Hz),7.21(2H,d,J=8. 8Hz),7.26(1H,dd,J=7.6,7.6Hz),7.42(1H,d,J=7.6Hz) ESI-:505 |
| 27 | 6 | FAB-:503 |

**[Table 48]**

| Ex | Syn | Data |
|---|---|---|
| 28 | 6 | NMR1:1.18(6H,s),1.86(2H,t,J=7.0,7.2Hz),1.97(3H,s),2.00(3H,s), 4.10(2H,t,J=7.0,7.2Hz),4.36(2H,s),4.38(1H,s),5.37(2H,s),6.78-6.81(1H,m),6.84-6.87(2H,m),6.96-6.98(1H,m),7.03-7.05(1H,m),7.20-7.24(1H,m),7.40-7.42(1H,m),7.62-7.64(1H,m),8.07-8.08(1H,m) ESI+:506 |
| 29 | 1 | ESI-:517 |
| 30 | 3 | ESI+:504 |
| 31 | 6 | NMR1:1.20(6H,s),1.89(2H,t,J=6.9Hz),1.96(3H,s),2.00(3H,s),2.1 2(3H,s),4.11(2H,t,J=6.9Hz),4.34(2H,s),4.39(1H,s),5.10(2H,s),6.8 3(1H,s),6.86(1H,s),6.99(2H,d,J=8.5Hz),7.02(1H,m),7.16-7.26(3H,m),7.35-7.44(1H,m) FAB-:517 |
| 32 | 6 | NMR1:1.19(6H,s),1.84-1.93(5H,m),1.98(3H,s),2.14(3H,s),4.10(2H,t,J=6.9Hz),4.33(2H,s ),4.38(1H,s),5.10(2H,s),6.87(2H,s),6.99(2H,d,J=8.5Hz),7.01-7.06(1H,m),7.17-7.26(3H,m),7.36-7.45(1H,m) FAB-:517 |
| 33 | 6 | ESI+:492 |
| 34 | 9 | FAB-:461 |
| 35 | 10 | NMR1:1.17(6H,s),1.83(2H,t,J=7.1Hz),1.88(6H,s),4.06(2H,t,J=7. 1Hz),4.19(2H,s),4.29(2H,d,J=5.9Hz),4.38(1H,s),6.21(1H,t,J=6. Hz),6.49(2H,d,J=8.5Hz),6.65(2H,s),6.91-6.97(3H,m),7.06(1H,s),7.30(1H,d,J=7.7Hz),7.33-7.39(1H,m) ESI+:504 |
| 36 | 9 | FAB-:461 |
| 37 | 9 | FAB-:475 |
| 38 | 3 | FAB+:518 |
| 39 | 6 | FAB+:492 |
| 40 | 1 | FAB+:492 |
| 41 | 5 | ESI+:450 |
| 42 | 6 | ESI-:490 |

**[Table 49]**

| Ex | Syn | Data |
|---|---|---|
| 43 | 6 | FAB-:490 |
| 44 | 6 | NMR1:1.12(3H,d,J=6.2Hz),1.69-1.80(2H,m),1.83(6H,s),1.90(3H,s),3.77-3.87(1H,m),3.97-4.08(2H,m),4.35(2H,s),4.54- 4.59(1H,m),5.35(2H,s),6.61(2H,s),6.83-6.86(1H,m),6.91-6.95(1H,m),7.21-7.27(1H,m),7.37-7.42(1H,m),7.59-7.64(1H,m),8.04-8.08(1H,m) ESI-:504 |
| 45 | 6 | NMR1:1.12(3H,d,J=6.2Hz),1.69-1.80(2H,m),1.83(6H,s),1.90(3H,s),3.77-3.87(1H,m),3.97-4.08(2H,m),4.35(2H,s),4.54-4.59(1H,m),5.35(2H,s),6.61(2H,s),6.83-6.86(1H,m),6.91-6.95(1H,m),7.21-7.27(1H,m),7.37-7.42(1H,m),7.59-7.64(1H,m),8.04-8.08(1H,m) ESI-:504 |
| 46 | 6 | ESI-:504 |
| 47 | 1 | ESI-:560 |
| 48 | 2 | ESI-:460 |
| 49 | 3 | ESI-:516 |
| 50 | 1 | ESI-:574 |
| 51 | 2 | ESI-:474 |
| 52 | 3 | ESI-:530 |
| 53 | 1 | ESI-:574 |
| 54 | 2 | ESI-:474 |
| 55 | 3 | ESI-:530 |
| 56 | 9 | NMR1:1.85(6H,s),1.92(3H,s),3.27-3.47(5H,m),3.84-3.99(3H,m),4.33(2H,s),5.08- 5.15(3H,m),6.72(2H,s),6.95(1H,d,J=7.2Hz),6.98(2H,d,J=8.8Hz), 7.21(2H,d,J=8.4Hz),7.26(1H,dd,J=7.4,7.4Hz),7.42(1H,d,J=7.2Hz ESI- : 519 |
| 57 | 11 | ESI+:477 |
| 58 | 9 | ESI-:462 |
| 59 | 9 | ESI-:476 |

**[Table 50]**

| Ex | Syn | Data |
|---|---|---|
| 60 | 6 | NMR1:1.18(6H,s),1.82-1.87(5H,m),1.95(3H,s),2.01(3H,s),4.32-4.38(5H,m),5.13(2H,s),6.58(1H,s),6.96-7.05(3H,m),7.17-7.24(2H,m),7.26-7.32(1H,m),7.44-7.49(1H,m) FAB+:520 |
| 61 | 9 | NMR1:1.84(6H,s),1.91(3H,s),3.67-3.73(2H,m),3.95-4.00(2H,m),4.35(2H,s),4.74-4.90(1H,m),5.36(2H,s),6.70(2H,s),6.83-6.86(1H,m),6.91- 6.95(1H,m),7.21-7.27(1H,m),7.38-7.42(1H,m),7.59-7.64(1H,m),8.05-8.08(1H,m) ESI+:478 |
| 62 | 10 | ESI-:461 |
| 63 | 6 | NMR1:1.95(3H,s),2.00(3H,s),2.15(3H,s),3.69- 3.80(2H,m),4.02(2H,t,J=5.1Hz),4.33(2H,s),4.84(1H,bs),5.10(2H, s),6.84(1H,s),6.85(1H,s),6.94-7.07(3H,m),7.17-7.27(3H,m),7.37-7.45(1H,m) ESI-:475 |
| 64 | 6 | NMR1:1.90(3H,s),1.98(3H,s),2.17(3H,s),3.71- 3.80(2H,m),4.01(2H,t,J=5.0Hz),4.33(2H,s),4.85(1H,bs),5.11 (2H, s),6.81-6.91(2H,m),6.95-7.08(3H,m),7.18-7.27(3H,m),7.38-7.46(1H,m) ESI-:475 |
| 65 | 12 | NMR1:1.97(3H,s),1.99(3H,s),3.42-3.51(2H,m),3.76-3.85(1H,m),3.85-3.92(1H,m),3.99-4.05(1H,m),4.18-4.26(4H,m),4.68(1H,t,J=5.7Hz),4.95(1H,d,J=5.1Hz),6.02(1H,t,J= 5.5Hz),6.54(2H,d,J=8.5Hz),6.77-6.83(1H,m),6.86-6.89(1H,m),6.92-7.01(4H,m),7.13-7.19(1H,m),7.28(1H,d,J=7.5Hz) ESI-:490 |
| 66 | 12 | NMR1:1.89(6H,s),3.41-3.48(2H,m),3.73-3.87(2H,m),3.94-4.01(1H,m),4.19(2H,s),4.29(2H,d,J=5.9Hz),4.65(1H,t,J=5.5Hz),4 .92(1H,d,J=5.0Hz),6.20(1H,t,J=6.1Hz),6.49(2H,d,J=8.5Hz),6.66( 2H,s),6.91-6.97(3H,m),7.05(1H,s),7.30(1H,d,J=7.7Hz),7.33-7.39(1H,m) ESI-:490 |

**[Table 51]**

| Ex | Syn | Data |
|---|---|---|
| 67 | 12 | NMR1:1.89(6H,s),3.41-3.48(2H,m),3.73-3.87(2H,m),3.94-4.01(1H,m),4.19(2H,s),4.29(2H,d,J=5.9Hz),4.65(1H,t,J=5.5Hz),4 .92(1H,d,J=5.0Hz),6.20(1H,t,J=6.1Hz),6.49(2H,d,J=8.5Hz),6.66( 2H,s),6.91-6.97(3H,m),7.05(1H,s),7.30(1H,d,J=7.7Hz),7.33-7.39(1H,m) ESI-:490 |
| 68 | 12 | NMR1:1.86(6H,s),1.91(3H,s),3.43-3.50(2H,m),3.76-3.90(2H,m),3.96-4.03(1H,m),4.18-4.27(4H,m),4.65(1H,t,J=5.6Hz),4.92(1H,d,J=5.3Hz),5.99(1H,t,J= 5.6Hz),6.54(2H,d,J=8.5Hz),6.71(2H,s),6.85(1H,d,J=6.5Hz),6.98( 2H,d,J=8.5Hz),7.15-7.22(1H,m),7.28(1H,d,J=6.8Hz) ESI-:504 |
| 69 | 12 | NMR1:1.86(6H,s),1.91(3H,s),3.43-3.50(2H,m),3.76-3.90(2H,m),3.96-4.03(1H,m),4.18-4.27(4H,m),4.65(1H,t,J=5.6Hz),4.92(1H,d,J=5.3Hz),5.99(1H,t,J= 5.6Hz),6.54(2H,d,J=8.5Hz),6.71(2H,s),6.85(1H,d,J=6.5Hz),6.98( 2H,d,J=8.5Hz),7.15-7.22(1H,m),7.28(1H,d,J=6.8Hz) ESI-:504 |
| 70 | 11 | NMR1:1.86(6H,s),1.91(3H,s),3.68-3.76(2H,m),3.99(2H,t,J=5.1Hz),4.18- 4.28(4H,m),4.85(1H,t,J=5.5Hz),6.01(1H,t,J=5.6Hz),6.54(2H,d,J= 8.4Hz),6.71(2H,s),6.85(1H,d,J=7.2Hz),6.98(2H,d,J=8.4Hz),7.14-7.22(1H,m),7.28(1H,d,J=7.5Hz) ESI-:474 |
| 71 | 15 | ESI-:490 |
| 72 | 6 | ESI-:448 |
| 73 | 6 | ESI-:462 |
| 74 | 13 | NMR3:1.89(6H,s),1.97(3H,s),3.52(3H,s),3.59-3.81(3H,m),4.01-4.06(1H,m),4.09-4.14(1H,m),4.59(2H,s),5.10(2H,s),6.71(2H,s),6.94-7.01(3H,m),7.24(1H,dd,J=7.6,7.6Hz),7.29(2H,d,J=8.8Hz),7.40(1 H,d,J=7.2Hz) ESI- : 519 |

**[Table 52]**

| Ex | Syn | Data |
|---|---|---|
| 75 | 1 | ESI-:574 |
| 76 | 2 | ESI-:474 |
| 77 | 3 | ESI-:574 |
| 78 | 5 | ESI-:532 |
| 79 | 14 | ESI+:519 |
| 80 | 5 | NMR1:1.85(6H,s),1.90(3H,s),3.67-3.76(2H,m),3.95-4.03(2H,m),4.20(2H,s),4.43-4.51(2H,m),4.80-4.89(1H,m),6.46-6.53(1H,m),6.65(2H,s),6.80-6.91(2H,m),7.14-7.33(3H,m),7.81-7.86(1H,m) ESI-:475 |
| 81 | 11 | NMR1:1.13(3H,d,J=6.2Hz),1.68-1.82(2H,m),1.86(3H,s),1.94(3H,s),2.01(3H,s),3.76-3.87(1H,m),4.23(2H,s),4.25(2H,d,J=5.5Hz),4.30(2H,t,J=6.5Hz),4 .54-4.62(1H,m),6.04(1H,t,J=5.5Hz),6.54(2H,d,J=8.4Hz),6.59(1H,s),6 .91(1H,d,J=7.5Hz),6.98(2H,d,J=8.4Hz),7.18- 7.25(1H,m),7.31 (1H,d,J=7.4Hz) ESI+:505 |
| 82 | 11 | NMR1:1.13(3H,d,J=6.2Hz),1.68-1.82(2H,m),1.86(3H,s),1.94(3H,s),2.01(3H,s),3.76-3.87(1H,m),4.23(2H,s),4.25(2H,d,J=5.5Hz),4.30(2H,t,J=6.5Hz),4 .54-4.62(1H,m),6.04(1H,t,J=5.5Hz),6.54(2H,d,J=8.4Hz),6.59(1H,s),6 .91(1H,d,J=7.5Hz),6.98(2H,d,J=8.4Hz),7.18-7.25(1H,m),7.31(1H,d,J=7.4Hz) ESI+:505 |
| 83 | 15 | NMR1:1.13(3H,d,J=6.2Hz),1.69-1.82(2H,m),1.85(3H,s),1.95(3H,s),2.00(3H,s),3.76-3.85(1H,m),4.27-4.36(4H,m),4.55-4.60(1H,m),5.12(2H,s),6.59(1H,s),6.97-7.04(3H,m),7.22(2H,d,J=8.6Hz),7.26-7.32(1H,m),7.46(1H,d,J=7.3Hz) ESI+:506 |
| 84 | 15 | NMR1:1.13(3H,d,J=6.2Hz), 1.69-1.82(2H,m), 1.85(3H,s), 1.95(3H,s),2.00(3H,s),3.76-3.85(1H,m),4.27-4.36(4H,m),4.55-4.60(1H,m),5.12(2H,s),6.59(1H,s),6.97-7.04(3H,m),7.22(2H,d,J=8.6Hz),7.26-7.32(1H,m),7.46(1H,d,J=7.3Hz) ESI+:506 |

**[Table 53]**

| Ex | Syn | Data |
|---|---|---|
| 85 | 11 | NMR1:1.96(3H,s),2.01(3H,s),3.71- 3.80(2H,m),4.10(2H,t,J=5.0Hz),4.19- 4.27(4H,m),4.95(1H,bs),6.04(1H,t,J=5.7Hz),6.53(2H,d,J=8.5Hz), 6.91(1H,d,J=11.9Hz),6.94- 7.01(3H,m),7.11(1H,d,J=8.9Hz),7.17(1H,t,J=7.6Hz),7.26-7.33(1H,m) ESI-:478 |
| 86 | 6 | ESI-:561 |
| 87 | 5 | NMR3:1.28(3H,s),1.89(6H,s),1.97(3H,s),3.52-3.63(2H,m),3.85- 3.93(2H,m),4.58(2H,s),5.11(2H,s),6.73(2H,s),6.94-7.00(3H,m),7.24(1H,dd,J=7.6,7.6Hz),7.29(2H,d,J=8.4Hz),7.40(1 H,d,J=7.2Hz) ESI- : 519 |
| 88 | 10 | NMR3:1.28(3H,s),1.89(6H,s),1.96(3H,s),3.52-3.64(2H,m),3.83-3.94(2H,m),4.29(2H,s),4.50(2H,s),6.58(2H,d,J=8.8Hz),6.72(2H,s ),6.87(1H,d,J=7.2Hz),7.10(2H,d,J=8.4Hz),7.17(1H,dd,J=7.6,7.6 Hz),7.32(1H,d,J=7.6Hz) ESI- : 518 |
| 89 | 11 | NMR1:2.02(3H,s),2.08(3H,s),2.15(3H,s),3.67-3.81(2H,m),4.15-4.28(4H,m),4.29-4.40(2H,m),4.81(1H,t,J=5.6Hz),6.03(1H,t,J=5.6Hz),6.54(2H,d,J= 8.5Hz),6.90-7.04(3H,m),7.18(1H,t,J=7.6Hz),7.24(1H,s),7.31(1H,d,J=7.5Hz) ESI+:477 |
| 90 | 11 | NMR1:1.14(3H,d,J=6.2Hz),1.70-1.90(1H,m),2.02(3H,s),2.08(3H,s),2.12(3H,s),3.78-3.94(1H,m),4.16-4.30(4H,m),4.37(2H,t,J=6.5Hz),4.56(1H,dd,J=2.4,4.9Hz),6.03(1 H,t,J=5.6Hz),6.53(2H,d,J=8.4Hz),6.88-7.05(3H,m),7.18(1H,t,J=7.6Hz),7.23(1H,s),7.30(1H,d,J=7.3Hz) ESI+:505 |
| 91 | 11 | NMR1:1.14(3H,d,J=6.4Hz),1.70-1.90(1H,m),2.02(3H,s),2.08(3H,s),2.12(3H,s),3.78-3.94(1H,m),4.16-4.30(4H,m),4.36(2H,t,J=6.5Hz),4.56(1H,dd,J=2.3,4.8Hz),6.03(1 H,t,J=5.5Hz),6.53(2H,d,J=8.4Hz),6.88-7.05(3H,m),7.18(1H,t,J=7.6Hz),7.23(1H,s),7.30(1H,d,J=7.4Hz) ESI+:505 |

**[Table 54]**

| Ex | Syn | Data |
|---|---|---|
| 92 | 11 | NMR1:1.20(6H,s),1.87(2H,t,J=7.OHz),2.02(3H,s),2.09(3H,s),2.1 2(3H,s),4.16-4.30(4H,m),4.36(1H,s),4.41(2H,t,J=7.0Hz),6.04(1H,t,J=5.5Hz),6 .54(2H,d,J=8.4Hz),6.90-7.05(3H,m),7.18(1H,t,J=7.6Hz),7.23(1H,s),7.31(1H,d,J=7.3Hz) ESI+:519 |
| 93 | 9 | NMR1:2.03(3H,s),2.07(3H,s),2.14(3H,s),3.65-3.85(2H,m),4.30-4.39(4H,m),4.65-4.90(1H,m),5.12(2H,s),6.99(2H,d,J=8.6Hz),7.05- 7.13(1H,m),7.17-7.32(4H,m),7.45(1H,d,J=7.1Hz) ESI+:478 |
| 94 | 9 | NMR1:1.95(3H,s),2.01(3H,s),3.70- 3.81(2H,m),4.10(2H,t,J=4.9Hz),4.33(2H,s),4.87- 4.96(1H,m),5.11(2H,s),6.93(1H,d,J=11.9Hz),6.99(2H,d,J=8.7Hz) ,7.03-7.08(1H,m),7.11(1H,d,J=8.9Hz),7.18-7.29(3H,m),7.41- 7.48(1H,m) FAB-:479 |
| 95 | 6 | NMR1:1.96(3H,s),3.74-3.78(2H,m),4.12- 4.14(2H,m),4.34(2H,s),4.92-4.94(1H,m),5.07- 5.13(2H,m),6.99(2H,d,J=8.7Hz),7.08- 7.10(1H,m),7.22(2H,d,J=8.7Hz),7.22-7.32(4H,m),7.46- 7.48(1H,m) ESI-:515 |
| 96 | 12 | NMR1:1.96(3H,s),1.99(3H,s),2.14(3H,s),3.45-3.55(2H,m),3.78- 3.87(1H,m),3.87-3.94(1H,m),3.97-4.03(1H,m),4.17- 4.25(4H,m),4.63-4.70(1H,m),4.89- 4.96(1H,m),6.02(1H,t,J=5.6Hz),6.53(2H,d,J=8.5Hz),6.82(1H,s),6 .84(1H,s),6.93(1H,d,J=7.2Hz),6.98(2H,d,J=8.4Hz),7.11-7.19(1H,m),7.27(1H,d,J=7.5Hz) ESI-:504 |

**[Table 55]**

| Ex | Syn | Data |
|---|---|---|
| 97 | 12 | NMRI:1.96(3H,s),1.99(3H,s),2.14(3H,s),3.45-3.55(2H,m),3.78- 3.87(1H,m),3.87-3.94(1H,m),3.97-4.03(1H,m),4.17- 4.25(4H,m),4.63-4.70(1H,m),4.89- 4.96(1H,m),6.02(1H,t,J=5.6Hz),6.53(2H,d,J=8.5Hz),6.82(1H,s),6 .84(1H,s),6.93(1H,d,J=7.2Hz),6.98(2H,d,J=8.5Hz),7.11- 7.19(1H,m),7.27(1H,d,J=7.5Hz) ESI-:504 |
| 98 | 16 | NMR1:1.95(3H,s),2.00(3H,s),2.14(3H,s),3.43-3.56(2H,m),3.79- 3.87(1H,m),3.87-3.95(1H,m),3.97-4.04(1H,m),4.33(2H,s),4.63- 4.70(1H,m),4.89-4.96(1H,m),5.10(2H,s),6.83(1H,s),6.84(1H,s),6.99(2H,d,J=8.4Hz ),7.03(1H,d,J=7.3Hz),7.18-7.26(3H,m),7.41(1H,d,J=7.5Hz) ESI-:505 |
| 99 | 16 | NMR1:1.95(3H,s),2.00(3H,s),2.14(3H,s),3.43-3.56(2H,m),3.79- 3.87(1H,m),3.87-3.95(1H,m),3.97-4.04(1H,m),4.33(2H,s),4.63- 4.70(1H,m),4.89- 4.96(1H,m),5.10(2H,s),6.83(1H,s),6.84(1H,s),6.99(2H,d,J=8.4Hz ),7.03(1H,d,J=7.3Hz),7.18-7.26(3H,m),7.41(1H,d,J=7.5Hz) ESI-:505 |
| 100 | 6 | ESI-:531 |
| 101 | 12 | NMR1:1.58-1.70(1H,m),1.80-1.99(10H,m),3.27-3.42(2H,m),3.61-3.70(1H,m),4.01-4.12(2H,m),4.17-4.27(4H,m),4.52-4.63(2H,m),5.96- 6.02(1H,m),6.54(2H,d,J=8.4Hz),6.70(2H,s),6.85(1H,d,J=6.8Hz), 6.98(2H,d,J=8.4Hz),7.18(1H,dd,J=7.6,7.6Hz),7.28(1H,d,J=7.6Hz ) ESI+ : 520 |
| 102 | 12 | NMR1:1.59-1.71(1H,m),1.78-1.99(10H,m),3.29- 3.39(2H,m),3.61-3.70(1H,m),4.02-4.11(2H,m),4.18- 4.28(4H,m),4.51-4.66(2H,m),5.96-6.04(1H,m),6.54(2H,d,J=8.8Hz),6.70(2H,s),6.85(1H,d,J=7.2Hz), 6.98(2H,d,J=8.4Hz),7.18(1H,dd,J=7.4,7.4Hz),7.28(1H,d,J=7.6Hz ) ESI+ : 520 |

**[Table 56]**

| Ex | Syn | Data |
|---|---|---|
| 103 | 6 | ESI-:559 |
| 104 | 6 | ESI-:559 |
| 105 | 7 | NMR1:1.58-1.70(1H,m),1.88-2.00(10H,m),3.24- 3.47(2H,m),3.60-3.71(1H,m),4.02-4.13(2H,m),4.34(2H,s),4.53- 4.68(2H,m),5.11(2H,s),6.71(2H,s),6.96(1H,d,J =7.2Hz),6.99(2H,d,J=8.4Hz),7.21(2H,d,J=8.8Hz),7.26(1H,dd,J= 7.6,7.6Hz),7.42(1H,d,J=7.2Hz) ESI- : 519 |
| 106 | 7 | NMR1:1.58-1.71(1H,m),1.79-1.99(10H,m),3.25- 3.46(2H,m),3.60-3.71(1H,m),4.02-4.13(2H,m),4.33(2H,s),4.49-4.71(2H,m),5.11(2H,s),6.71(2H,s),6.96(1H,d,J=7.6Hz),6.99(2H,d ,J=8.8Hz),7.21(2H,d,J=8.4Hz),7.26(1H,dd,J=7.6Hz),7.42(1H,d,J =7.6Hz) ESI- : 519 |
| 107 | 11 | ESI+:462 |
| 108 | 9 | ESI-:461 |
| 109 | 11 | ESI-:520 |
| 110 | 11 | ESI-:520 |
| 111 | 9 | FAB-:507 |
| 112 | 10 | ESI+:508 |
| 113 | 12 | ESI+:538 |
| 114 | 12 | ESI+:538 |
| 115 | 9 | NMR1:3.69- 3.79(2H,m),4.03(2H,t,J=5.1Hz),4.32(2H,s),4.37(2H,d,J=5.4Hz),4 .88(1H,t,J=5.6Hz),5.12(2H,s),5.17(1H,t,J=5.4Hz),6.85- 6.92(1H,m),6.96(2H,d,J=8.7Hz),7.11- 7.16(2H,m),7.20(2H,d,J=8.5Hz),7.25-7.31(1H,m),7.35- 7.46(3H,m) FAB-:463 |

**[Table 57]**

| Ex | Syn | Data |
|---|---|---|
| 116 | 19 | NMR1:1.98(3H,s),2.05(3H,s),2.13(3H,s),3.69-3.78(2H,m),4.19- 4.98(10H,m),6.74-6.84(2H,m),6.98-7.05(1H,m),7.09- 7.25(4H,m),7.29-7.36(1H,m),12.30-12.49(1H,br) ESI-:475 |
| 117 | 19 | NMR1:1.95(3H,s),1.98(3H,s),2.14(3H,s),3.45-3.55(2H,m),3.60- 4.32(10H,m),4.63(2H,s),6.71(2H,d,J=8.2Hz),6.80(1H,s),6.84(1H ,s),6.94-7.00(1H,m),7.10(2H,d,J=8.5Hz),7.17(1H,t,J=7.6Hz),7.28(1H,d,J =7.6Hz),12.30-12.48(1H,br) ESI-:504 |
| 118 | 19 | NMR1:1.57-1.72(1H,m),1.84(6H,s),1.86-2.01(4H,m),3.25- 3.42(2H,m),3.50-4.22(8H,m),4.25-4.37(2H,m),4.62(2H,s),6.62- 6.76(4H,m),6.88(1H,d,J=7.0Hz),7.08(2H,d,J=8.4Hz),7.20(1H,t,J =7.6Hz),7.28(1H,d,J=7.3Hz),12.20-12.80(1H,br) ESI-:518 |
| 119 | 3 | NMR1:0.99(3H,t,J=7.6Hz),1.79- 1.89(8H,m),1.92(3H,s),2.07(2H,q,J=7.6Hz),3.16- 3.26(2H,m),3.95- 4.02(2H,m),4.33(2H,s),5.11(2H,s),6.71(2H,s),6.95(1H,d,J=7.2Hz ),6.98(2H,d,J=8.4Hz),7.21(2H,d,J=8.4Hz),7.26(1H,dd,J=7.6,7.6 Hz),7.42(1H,d,J=7.2Hz),7.82-7.90(1H,m) ESI+ : 546 |

### INDUSTRIAL APPLICABILITY

The compound of the formula (I) or a pharmaceutically acceptable salt thereof has GPR40 receptor agonistic action, and can be used as insulin secretagogues and an agent for preventing and/or treating diseases associated with GPR40 such as diabetes mellitus (insulin-dependent diabetes mellitus (type 1 diabetes), non-insulin-dependent diabetes mellitus (type 2 diabetes) and borderline thereof (impaired glucose tolerance/fasting blood glucose)), obesity, and the like.

### Sequence Listing Free Text

At the numeric caption <223> of the following sequence listing, explanation of "Artificial Sequence" will be described. Concretely, the base sequence represented by SEQ ID NO. 1 of the sequence listing is an artificially-synthesized primer base sequence. In addition, the base sequence represented by SEQ ID NO. 2 of the sequence listing is an artificially-synthesized primer base sequence.

## Claims

1. A compound of the formula (I): 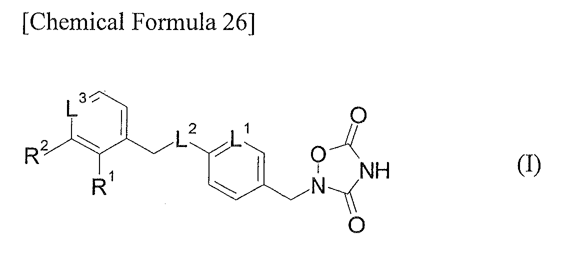 wherein the symbols have the following meanings:
L¹ and L³ are the same with or different from each other and each represents CH or N;
L² represents O or NH;
R¹ represents -H or C₁₋₆ alkyl;
R² represents a group of the formula (II) or (III): 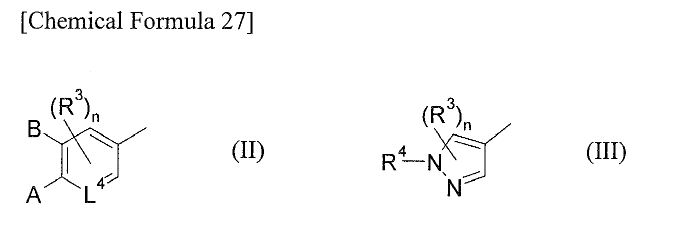 L⁴ represents CH or N;
A and B are the same with or different from each other and represent -O-(C₁₋₆ alkyl substituted with one or more group(s) selected from G¹ group), amino which may be substituted with one or more group(s) selected from G² group, -H or -R³ (provided that at least one of A and B represents a group other than -H and -R³);
R³ is the same with or different from each other and represents C₁₋₆ alkyl which may be substituted with one or more group (s) selected from the group consisting of -OH and halogen, halogen or -O-(C₁₋₆ alkyl);
R⁴ represents C₁₋₆ alkyl which is substituted with one or more group(s) selected from _{G}¹ group;
n represents 1 or 2;
G¹ group represents the group consisting of -NHCO₂R^{Z}, -NH₂, -NHCOR^{Z}, -NHCO-(cycloalkyl), -NHCO-(aryl), -NHSO₂R^{Z}, 1,3-dioxolan-4-yl which may be substituted with 1 to 5 C₁₋₆ alkyl, -OH, -OCOR^{Z}, -OR^{Z}, -CO₂R^{Z}, -CO₂H, -CONHR^{Z} and -CON(R^{Z})₂;
G² group represents the group consisting of -CO₂R^{Z} and -R^{Z}; and
R^{Z} is the same with or different from each other and represents C₁₋₆ alkyl which may be substituted with one or more group(s) selected from the group consisting of -OH and - OCO-(C₁₋₆ alkyl);
or a pharmaceutically acceptable salt thereof.

2. The compound according to Claim 1, wherein L³ is CH; R¹ is -H or methyl; R² is a group of the formula (II); either one of A and B is -O- (C₁₋₆ alkyl substituted with one or more group(s) selected from G¹ group), and the other of A or B is -H or -R³; and R³ is the same with or different from each other, and methyl which may be substituted with one or more halogen, halogen or -O-methyl; or a pharmaceutically acceptable salt thereof.

3. The compound according to Claim 2, wherein either one of A and B is -O-(C₁₋₆ alkyl which is substituted with one or more group(s) selected from the group consisting of-NHCOR^{Z}, -NHCO-(cycloalkyl), -OH and -OR^{Z}); R^{Z} is C₁₋₆ alkyl which may be substituted with one or more -OH, and the other of A or B is -H, methyl or halogen; or a pharmaceutically acceptable salt thereof.

4. The compound according to Claim 3, wherein R³ is methyl, or a pharmaceutically acceptable salt thereof.

5. The compound according to Claim 4, wherein R¹ is methyl, or a pharmaceutically acceptable salt thereof.

6. The compound according to Claim 5, wherein either one of A and B is -H; and n is 2; or a pharmaceutically acceptable salt thereof.

7. The compound according to Claim 5, wherein either one of A and B is methyl or halogen; and n is 1; or a pharmaceutically acceptable salt thereof.

8. The compound according to Claim 6 or 7, wherein A is -O-(C₁₋₆ alkyl which is substituted with one or more group(s) selected from the group consisting of -NHCOR^{Z},-NHCO-(cycloalkyl), -OH and -OR^{Z}), or a pharmaceutically acceptable salt thereof.

9. The compound according to Claim 8, wherein A is -O-(C₁₋₆ alkyl substituted with one or more -OH), or a pharmaceutically acceptable salt thereof.

10. The compound according to Claim 9, wherein L¹ is CH, or a pharmaceutically acceptable salt thereof.

11. The compound according to Claim 10, wherein L² is O, or a pharmaceutically acceptable salt thereof.

12. The compound according to Claim 10, wherein L² is NH, or a pharmaceutically acceptable salt thereof.

13. The compound according to Claim 1 that is
2-(4-{[(4'-{[(2S)-2,3-dihydroxypropyl]oxy}-2,2',6'-trimethylbiphenyl-3-yl)methyl]amino}benzyl)-1,2,4-oxadiazolidine-3,5-dione, 2-(4-{[(4'-{[(2R)-2,3-dihydroxypropyl]oxy}-2,2',6'-trimethylbiphenyl-3-yl)methyl]amino}benzyl)-1,2,4-oxadiazolidine-3,5-dione, 2-[4-({[5'-fluoro-4'-(2-hydroxyethoxy)-2,2'-dimethylbiphenyl-3-yl]methyl}amino)benzyl]-1,2,4-oxadiazolidine-3,5-dione, 2-[4-({3-[6-(2-hydroxyethoxy)-2,5-dimethylpyridin-3-yl]-2-methylbenzyl}amino)benzyl]-1,2,4-oxadiazolidie-3,5-dione,
2-(4-{[5'-fluoro-4'-(2-hydroxyethoxy)-2,2'-dimethylbiphenyl-3-yl]methoxy}benzyl)-1,2,4-oxadiazolidine-3,5-dione,
2-(4-{[(4'-{[(2S)-2,3-dihydroxypropyl]oxy}-2,2', 5'-trimethylbiphenyl-3-yl)methyl]amino}benzyl)-1,2,4-oxadiazolidine-3,5-dione,
2-(4-{[4'-{[(2R)-2,3-dihydroxypropyl]oxy}-2,2',5'-trimethylbiphenyl-3-yl)methyl]amino}benzyl)-1,2,4-oxadiazolidine-3, 5-dione,
2-(4-{[(4'-{[(3R)-3,4-dihydroxybutyl]oxy}-2,2',6'-trimethylbiphenyl-3-yl)methyl]amino}benzyl)-1,2,4-oxadiazolidine-3,5-dione,
2-(4-{[(4'-{[(3S)-3,4-dihydroxybutyl]oxy}-2,2',6' -trimethylbiphenyl-3-yl)methyl]amino}benzyl)-1,2,4-oxadiazolidine-3,5-dione,
2-[4-({2,2',6'-trimethyl-4'-[3-(propionylamino)propoxy]biphenyl-3-yl}methoxy)benzyl]-1,2,4-oxadiazolidine-3,5-dione,
2-{4-[(4'-{3-[(cyclopropylcarbonyl)amino]propoxy}-2,2',6'-trimethylbiphenyl-3-yl)methoxy]benzyl}-1,2,4-oxadiazolidine-3,5-dione,
2-{4-[(4'-{[(2S)-2,3-dihydroxypropyl]oxy}-2,2',6'-trimethylbiphenyl-3-yl)methoxy]benzyl}-1,2,4-oxadiazolidine-3,5-dione,
2-{4-[(4'-{[(2R)-2,3-dihydroxypropyl]oxy}-2,2',6'-trimethylbiphenyl-3-yl)methoxy]benzyl}-1,2,4-oxadiazolidine-3,5-dione,
2-(4-{[3'-(3-hydroxy-3-methylbutoxy)-2,2'-dimethylbiphenyl-3-yl]methoxy}benzyl)-1,2,4-oxadiazolidine-3,5-dione,
2-(4-{[4'-(3-hydroxy-3-methylbutoxy)-2,2',5'-trimethylbiphenyl-3-yl]methoxy}benzyl)-1,2,4-oxadiazolidine-3,5-dione,
2-(4-{[4'-(3-hydroxy-3-methylbutoxy)-2,2',3'-trimethylbiphenyl-3-yl]methoxy}benzyl)-1,2,4-oxadiazolidine-3,5-dione,
2-({6-[(4'-{[(3R)-3-hydroxybutyl]oxy}-2,2',6'-trimethylbiphenyl-3-yl)methoxy]pyridin-3-yl}methyl)-1,2,4-oxadiazolidine-3,5-dione,
2-({6-[(4'-{[(3S)-3-hydroxybutyl]oxy}-2,2',6'-trimethylbiphenyl-3-yl)methoxy]pyridin-3-yl}methyl)-1,2,4-oxadiazolidine-3,5-dione,
2-{4-[(4'-{[(2R)-2-hydroxy-3-methoxypropyl]oxy}-2,2',6'-trimethylbiphenyl-3-yl)methoxy]benzyl}-1,2,4-oxadiazolidine-3,5-dione,
2-[4-({3-[6-(3-hydroxy-3-methylbutoxy)-2,4-dimethylpyridin-3-yl]-2-methylbenzyl}oxy)benzyl]-1,2,4-oxadiazolidine-3,5-dione,
2-[(6-{[4'-(2-hydroxyethoxy)-2,2',6'-trimethylbiphenyl-3-yl]methoxy}pyridin-3-yl)methyl]-1,2,4-oxadiazolidine-3,5-dione,
2-(4-{[4'-(2-hydroxyethoxy)-2,2',5'-trimethylbiphenyl-3-yllmethoxy}benzyl)-1,2,4-oxadiazolidine-3,5-dione,
2-[4-({[4'-(2-hydroxyethoxy)-2,2',6'-trimethylbiphenyl-3-yl]methyl}amino)benzyl]-1,2,4-oxadiazolidine-3,5-dione,
2-{4-[(4'-{[(2S)-3-hydroxy-2-methoxypropyl]oxy}-2,2',6'-trimethylbiphenyl-3-yl)methoxy]benzyl}-1,2,4-oxadiazolidine-3,5-dione,
2-{[6-({[4'-(2-hydroxyethoxy)-2,2',6'-trimethylbiphenyl-3-yl]methyl}amino)pyridin-3-yl]methyl}-1,2,4-oxadiazolidine-3,5-dione,
2-(4-{[3-(6-{[(3R)-3-hydroxybutyl]oxy}-2,4-dimethylpyridin-3-yl)-2-methylbenzyl]amino}benzyl)-1,2,4-oxadiazolidine-3,5-dione or
2-(4-{[3-(6-{[(3R)-3-hydroxybutyl]oxy}-2,4-dimethylpyridin-3-yl)-2-methylbenzyl]oxy}benzyl)-1,2,4-oxadiazolidine-3,5-dione,
or a pharmaceutically acceptable salt thereof.

14. The compound according to Claim 1 which is
2-(4-{[(4'-{[(2S)-2,3-dihydroxypropyl]oxy}-2,2',6'-trimethylbiphenyl-3-yl)methyl]amino}benzyl)-1,2,4-oxadiazolidine-3,5-dione,
2-(4-{[(4'-{[(2R)-2,3-dihydroxypropyl]oxy}-2,2',6'-trimethylbiphenyl-3-yl)methyl]amino}benzyl)-1,2,4-oxadiazolidine-3,5-dione,
2-[4-({[5'-fluoro-4'-(2-hydroxyethoxy)-2,2'-dimethylbiphenyl-3-yl]methyl}amino)benzyl]-1,2,4-oxadiazolidine-3,5-dione,
2-[4-({3-[6-(2-hydroxyethoxy)-2,5-dimethylpyridin-3-yl]-2-methylbenzyl}amino)benzyl]-1,2,4-oxadiazolidine-3,5-dione,
2-(4-{[5'-fluoro-4'-(2-hydroxyethoxy)-2,2'-dimethylbiphenyl-3-yl]methoxy}benzyl)-1,2,4-oxadiazolidine-3,5-dione,
2-(4-{[(4'-{[(2S)-2,3-dihydroxypropyl]oxy}-2,2',5'-trimethylbiphenyl-3-yl)methyl]amino}benzyl)-1,2,4-oxadiazolidine-3,5-dione,
2-(4-{[(4'-{[(2R)-2,3-dihydroxypropyl]oxy}-2,2',5'-trimethylbiphenyl-3-yl)methyl] amino }benzyl)-1,2,4-oxadiazolidine-3,5-dione,
2-(4-{[(4'-{[(3R)-3,4-dihydroxybutyl]oxy}-2,2',6'-trimethylbiphenyl-3-yl)methyl]amino}benzyl)-1,2,4-oxadiazolidine-3,5-dione or
2-(4-{[(4'-{[(3S)-3,4-dihydroxybutyl]oxy}-2,2',6'-trimethylbiphenyl-3-yl)methyl]amino}benzyl)-1,2,4-oxadiazolidine-3,5-dione,
or a pharmaceutically acceptable salt thereof.

15. A pharmaceutical composition which comprises the compound according to Claim 1 or a pharmaceutically acceptable salt thereof, and a pharmaceutical acceptable excipient.

16. A GPR40 agonist which comprises the compound according to Claim 1 or a pharmaceutically acceptable salt thereof.

17. An insulin secretagogue which comprises the compound according to Claim 1 or a pharmaceutical acceptable salt thereof.

18. A pharmaceutical composition for preventing and/or treating diabetes mellitus, which comprises the compound according to Claim 1 or a pharmaceutically acceptable salt thereof.

19. Use of the compound according to Claim 1 or a pharmaceutically acceptable salt thereof for the manufacture of an insulin secretagogue or an agent for preventing and/or treating diabetes mellitus.

20. A method of promoting insulin secretion or a method of preventing and/or treating diabetes mellitus, which comprises administering an effective amount of the compound according to Claim 1 or a salt thereof to a patient.


SEQUENCE LISTING

<110>  Astellas Pharma Inc.

<120>  oxadiazolidinedione compounds

<130>  A08031

<160>  4

<170>  PatentIn version 3.1

<210>  1
<211>  25
<212>  DNA
<213>  Artificial

<220>
<223>  Description of Artifical Sequence:an artificailly synthesized pri
       mer seqence
<220>
<223>  Inventor: Negoro, Kenji; Iwasaki, Fumiyoshi; Ohnuki, Kei;
         Inventor: Kurosaki, Toshio; Tsuchiya, Kazuyuki; Kuramoto, Kazuyuki;
         Inventor: Yoshida, Shigeru; Soga, Takatoshi

<400>  1
ggtctagaat ggacctgccc ccgca                                          25


<210>  2
<211>  27
<212>  DNA
<213>  Artificial

<220>
<223>  Description of Artifical Sequence:an artificailly synthesized pri
       mer seqence

<400>  2
ggtctagatt acttctggga cttgccc                                        27


<210>  3
<211>  903
<212>  DNA
<213>  Homo sapiens


<400>  3
atggacctgc ccccgcagct ctccttcggc ctctatgtgg ccgcctttgc gctgggcttc      60

ccgctcaacg tcctggccat ccgaggcgcg acggcccacg cccggctccg tctcacccct     120

agcctggtct acgccctgaa cctgggctgc tccgacctgc tgctgacagt ctctctgccc     180

ctgaaggcgg tggaggcgct agcctccggg gcctggcctc tgccggcctc gctgtgcccc     240

gtcttcgcgg tggcccactt cttcccactc tatgccggcg ggggcttcct ggccgccctg     300

agtgcaggcc gctacctggg agcagccttc cccttgggct accaagcctt ccggaggccg     360

tgctattcct ggggggtgtg cgcggccatc tgggccctcg tcctgtgtca cctgggtctg     420

gtctttgggt tggaggctcc aggaggctgg ctggaccaca gcaacacctc cctgggcatc     480

aacacaccgg tcaacggctc tccggtctgc ctggaggcct gggacccggc ctctgccggc     540

ccggcccgct tcagcctctc tctcctgctc ttttttctgc ccttggccat cacagccttc     600

tgctacgtgg gctgcctccg ggcactggcc cactccggcc tgacgcacag gcggaagctg 660

cgggccgcct gggtggccgg cggggccctc ctcacgctgc tgctctgcgt aggaccctac 720

aacgcctcca acgtggccag cttcctgtac cccaatctag gaggctcctg gcggaagctg 780

gggctcatca cgggtgcctg gagtgtggtg cttaatccgc tggtgaccgg ttacttggga 840

aggggtcctg gcctgaagac agtgtgtgcg gcaagaacgc aagggggcaa gtcccagaag 900

taa 903

<210> 4
<211> 300
<212> PRT
<213> Homo sapiens

<400> 4

Met Asp Leu Pro Pro Gln Leu Ser Phe Gly Leu Tyr Val Ala Ala Phe
1               5                   10                  15

Ala Leu Gly Phe Pro Leu Asn Val Leu Ala Ile Arg Gly Ala Thr Ala
            20                  25                  30

His Ala Arg Leu Arg Leu Thr Pro Ser Leu Val Tyr Ala Leu Asn Leu
        35                  40                  45

Gly Cys Ser Asp Leu Leu Leu Thr Val Ser Leu Pro Leu Lys Ala Val
    50                  55                  60

Glu Ala Leu Ala Ser Gly Ala Trp Pro Leu Pro Ala Ser Leu Cys Pro
65                  70                  75                  80

Val Phe Ala Val Ala His Phe Phe Pro Leu Tyr Ala Gly Gly Gly Phe
                85                  90                  95

Leu Ala Ala Leu Ser Ala Gly Arg Tyr Leu Gly Ala Ala Phe Pro Leu
            100                 105                 110

Gly Tyr Gln Ala Phe Arg Arg Pro Cys Tyr Ser Trp Gly Val Cys Ala
        115                 120                 125

Ala Ile Trp Ala Leu Val Leu Cys His Leu Gly Leu Val Phe Gly Leu
    130                 135                 140

Glu Ala Pro Gly Gly Trp Leu Asp His Ser Asn Thr Ser Leu Gly Ile
145                 150                 155                 160

Asn Thr Pro Val Asn Gly Ser Pro Val Cys Leu Glu Ala Trp Asp Pro
                165                 170                 175

Ala Ser Ala Gly Pro Ala Arg Phe Ser Leu Ser Leu Leu Leu Phe Phe
            180                 185                 190

Leu Pro Leu Ala Ile Thr Ala Phe Cys Tyr Val Gly Cys Leu Arg Ala
        195                 200                 205

Leu Ala His Ser Gly Leu Thr His Arg Arg Lys Leu Arg Ala Ala Trp
    210                 215                 220

Val Ala Gly Gly Ala Leu Leu Thr Leu Leu Leu Cys Val Gly Pro Tyr
225                 230                 235                 240

Asn Ala Ser Asn Val Ala Ser Phe Leu Tyr Pro Asn Leu Gly Gly Ser
                245                 250                 255

Trp Arg Lys Leu Gly Leu Ile Thr Gly Ala Trp Ser Val Val Leu Asn
            260                 265                 270

Pro Leu Val Thr Gly Tyr Leu Gly Arg Gly Pro Gly Leu Lys Thr Val
        275                 280                 285

Cys Ala Ala Arg Thr Gln Gly Gly Lys Ser Gln Lys
    290                 295                 300